# FASCICULE DE BREVET EUROPEEN

(11) **EP 3 362 559 B1**
(45) Date de publication et mention de la délivrance du brevet: **06.05.2020**
(21) Numéro de dépôt: 16795118.5
(22) Date de dépôt: 14.10.2016
(51) Int. Cl.: C12N 15/10, C12N 9/22, C12N 15/63, C12N 15/90, C12N 15/74, C12R 1/145, C12P 7/04, C12P 7/26

(54) **OUTIL GENETIQUE DE TRANSFORMATION DE BACTERIES CLOSTRIDIUM**
GENETISCHES WERKZEUG ZUR TRANSFORMATION VON CLOSTRIDIUM
GENETIC TOOL FOR THE TRANSFORMATION OF CLOSTRIDIUM

(30) Priorité: 16.10.2015 FR 1559846
(43) Date de publication de la demande: 22.08.2018
(73) Titulaire: IFP Energies nouvelles, 92500 Rueil-Malmaison (FR); Stichting Wageningen Research, 6708 PB Wageningen (NL)
(72) Inventeur: WASELS, François, 57050 Metz (FR); LOPES FERREIRA, Nicolas, 28210 Croisilles (FR); COLLAS, Florent, 6702 A Wageningen (NL); LOPEZ CONTRERAS, Ana, 3522 Gl Utrecht (NL)
(74) Mandataire: Cabinet Becker et Associés
(86) Numéro de dépôt international: PCT/FR2016/052663
(87) Numéro de publication internationale: WO 2017/064439

(56) Documents cités:
- TAO XU ET AL: "Efficient Genome Editing in Clostridium cellulolyticum via CRISPR-Cas9 Nickase", APPLIED AND ENVIRONMENTAL MICROBIOLOGY, AMERICAN SOCIETY FOR MICROBIOLOGY, US, vol. 81, no. 13, 1 juillet 2015 (2015-07-01), pages 4423-4431, XP002741442, ISSN: 1098-5336, DOI: 10.1128/AEM.00873-15 [extrait le 2015-04-24]
- TAO XU ET AL: "Supplementary Data for Efficient Genome Editing in Clostridium cellulolyticum via CRISPR-Cas9 Nickase", APPLIED AND ENVIRONMENTAL MICROBIOLOGY, AMERICAN SOCIETY FOR MICROBIOLOGY, US, 24 avril 2015 (2015-04-24), pages 1-13, XP002741729, ISSN: 0099-2240, DOI: 10.1128/AEM.00873-15
- WANG Y ET AL: "Markerless chromosomal gene deletion in Clostridium beijerinckii using CRISPR/Cas9 system", JOURNAL OF BIOTECHNOLOGY, ELSEVIER SCIENCE PUBLISHERS, AMSTERDAM, NL, vol. 200, 20 avril 2015 (2015-04-20), pages 1-5, XP002741440, ISSN: 0168-1656, DOI: 10.1016/J.JBIOTEC.2015.02.005 [extrait le 2015-02-11]
- CORNILLOT E ET AL: "The genes for butanol and acetone formation in Clostridium acetobutylicum ATCC 824 reside on a large plasmid whose loss leads to degeneration of the strain", JOURNAL OF BACTERIOLOGY, AMERICAN SOCIETY FOR MICROBIOLOGY, US, vol. 179, no. 17, 1 janvier 1997 (1997-01-01), pages 5442-5447, XP002412768, ISSN: 0021-9193
- FLORENT COLLAS ET AL: "Simultaneous production of isopropanol, butanol, ethanol and 2,3-butanediol by Clostridium acetobutylicum ATCC 824 engineered strains", AMB EXPRESS, vol. 2, no. 1, 1 janvier 2012 (2012-01-01) , page 45, XP055173412, ISSN: 2191-0855, DOI: 10.1080/10408410701364604

## Description

La présente description concerne un outil génétique comprenant au moins deux acides nucléiques distincts permettant la transformation par recombinaison homologue d'une bactérie du genre *Clostridium,* typiquement d'une bactérie solvantogène du genre *Clostridium.*

### ARRIERE-PLAN TECHNOLOGIQUE

Les bactéries appartenant au genre *Clostridium* sont des bacilles anaérobies strictes à coloration de gram positive, capables de former des endospores et appartenant au phylum des Firmicutes. Ce genre contient de nombreuses espèces étudiées en raison de leur caractère pathogène ou de leur intérêt industriel et médical. Ainsi, *Clostridium tetani, Clostridium botulinum, Clostridium perfringens* et *Clostridium difficile* sont les agents respectivement responsables du tétanos, du botulisme, de gangrènes gazeuses et de colites pseudomembraneuses. Parallèlement, d'autres espèces telles que *Clostridium acetobutylicum, Clostridium butyricum* et *Clostridium beijerinckii,* non pathogènes pour l'homme, sont utilisées en fermentation. Enfin, *Clostridium novyi* et *Clostridium sporogenes* ont récemment été utilisées dans des études ayant pour but la mise au point de thérapies anticancéreuses. Les espèces de *Clostridium* dites d'intérêt industriel sont capables de produire des solvants à partir d'une large variété de sucres et substrats allant du glucose à la cellulose. La croissance des bactéries *Clostridium* productrices de solvants est dite biphasique. Des acides (acétiques et butyriques) sont produits lors de la phase exponentielle de croissance. Puis, lorsque la croissance cellulaire cesse et que les bactéries entrent en phase stationnaire, elles produisent des solvants.

La majorité des souches solvantogènes de *Clostridium* produit de l'Acétone, du Butanol et de l'Éthanol comme produits finaux. Ces souches sont désignées « souches ABE ». C'est par exemple le cas des souches ATCC824 de *C*. *acetobutylicum* et NCIMB 8052 de *C*. *beijerinckii.* D'autres souches sont également capables de réduire l'Acétone en Isopropanol, et sont désignées « souches IBE ». C'est le cas par exemple de la souche DSM 6423 (NRRL B593) de *C*. *beijerinckii* qui possède dans son génome un gène *adh* codant une alcool-déshydrogénase primaire/secondaire qui permet la réduction de l'acétone en isopropanol.

La production d'isopropanol par *C*. *acetobutylicum* ATCC824 est possible après introduction d'un plasmide permettant l'expression du gène *adh* de *C. beijerinckii* DSM 6423. Une telle souche génétiquement modifiée possède des performances équivalentes à la souche DSM 6423. Ces performances peuvent être améliorées après surexpression au sein d'une structure opéronique des gènes *ctfA, ctfB* et *adc* codant respectivement les CoA-transférases impliquées dans la ré-assimilation d'acides et l'acétoacétate decarboxylase (Collas *et a*l*,* 2012). L'introduction d'un plasmide contenant ces gènes modifie le profil fermentaire de la souche ATCC824 pour produire un mélange IBE. Cependant, le maintien de cette construction génétique nécessite la présence d'antibiotique dans le milieu de croissance, rendant impossible l'utilisation de cette souche pour des applications industrielles.

Malgré l'intérêt indéniable des bactéries du genre *Clostridium,* leur métabolisme est encore peu étudié et/ou modifié du fait des difficultés à obtenir des souches modifiées génétiquement. Les systèmes les plus performants sont basés sur des événements de recombinaison homologue permettant la modification précise du génome de manière stable. Les fréquences de recombinaison homologue observées chez *Clostridium* étant cependant très faibles, des marqueurs de sélection (*e. g.* un gène de résistance à un antibiotique) et de contre-sélection (*e.* g. un gène codant une toxine) se sont avérés nécessaires. Deux outils ont récemment été développés (Al Hinai *et al.,* 2012 ; Cartman *et al.,* 2012), chacun étant basé sur l'utilisation d'un plasmide unique. Bien que novateurs, ces systèmes présentent des inconvénients. Le premier système présente le défaut de laisser au niveau du site de modification une cassette FRT (utilisée lors de l'excision du marqueur de sélection) pouvant altérer le contexte génétique du mutant, et empêchant la réutilisation de l'outil pour réaliser de nombreuses modifications. Ces défauts sont bien connus de l'homme de métier. Le second système, nécessitant deux événements séquentiels de recombinaison homologue, ne peut pas être utilisé pour modifier des régions essentielles du génome. Un outil impliquant l'utilisation séquentielle de deux plasmides, l'un d'entre eux codant la méganucléase I-SceI capable de provoquer des coupures d'ADN double-brin au niveau de sites cibles spécifiques et de favoriser les événements de recombinaison homologues, a ensuite été développé (Zhang *et al.,* 2015). Là aussi, la modification de certains gènes essentiels est rendue impossible par la nécessité de réaliser deux événements de recombinaison homologue séquentiels. La dernière génération d'outils développés à ce jour (Wang *et al.,* 2015 ; Xu *et al.,* 2015) fait appel à la technologie CRISPR (Clustered Regularly Interspaced Short Palindromic Repeats) qui fonctionne d'une manière analogue à l'interférence ARN observée chez les eucaryotes (Barrangou *et al,* 2007). Les outils décrits par Wang *et al.* et Xu *et al.,* respectivement adaptés à *C*. *beijerinckii* et *C. cellulolyticum,* reposent sur l'utilisation d'un plasmide unique. Xu *et al.* utilisent une version modifiée de l'enzyme Cas9 créant des coupures simple brin au lieu de coupure double brin. Les deux outils disponibles fondés sur la technologie CRISPR présentent l'inconvénient majeur de limiter significativement la taille de l'acide nucléique d'intérêt (et donc le nombre de séquences codantes ou gènes) susceptible d'être inséré dans le génome bactérien (1,8 kb environ au mieux d'après Xu *et al.*)*.*

### RESUME DE L'INVENTION

L'objectif de la présente invention est de fournir un outil génétique adapté au genre *Clostridium* dans son ensemble, en particulier aux bactéries solvantogènes du genre *Clostridium,* permettant pour la première fois de modifier le génome bactérien afin de permettre l'exploitation des bactéries du genre *Clostridium* à l'échelle industrielle.

La description concerne ainsi un outil génétique permettant la transformation par recombinaison homologue d'une bactérie du genre *Clostridium,* de préférence une bactérie solvantogène du genre *Clostridium,* caractérisé en ce qu'il comprend au moins deux acides nucléiques distincts. Cet outil est par exemple capable de modifier une région du génome de la bactérie du genre *Clostridium* incluant une séquence essentielle à la survie bactérienne, ou de permettre l'insertion de grands fragments de séquences d'acide nucléique impossible à l'aide des outils existants.

Un premier aspect de la description concerne ainsi un outil génétique permettant la transformation par recombinaison homologue d'une bactérie solvantogène du genre *Clostridium* caractérisé en ce qu'il comprend :
- un premier acide nucléique codant au moins Cas9, dans lequel la séquence codant Cas9 est placée sous le contrôle d'un promoteur, et
- au moins un deuxième acide nucléique contenant une matrice de réparation permettant, par un mécanisme de recombinaison homologue, le remplacement d'une portion de l'ADN bactérien ciblée par Cas9 par une séquence d'intérêt,
et en ce que i) au moins l'un desdits acides nucléiques code en outre un ou plusieurs ARN guides (ARNg), ou ii) l'outil génétique comprend en outre un ou plusieurs ARN guides, chaque ARN guide comprenant une structure ARN de fixation à l'enzyme Cas9 et une séquence complémentaire de la portion ciblée de l'ADN bactérien.

L'invention concerne par ailleurs un procédé pour transformer et/ou modifier génétiquement par recombinaison homologue une bactérie du genre *Clostridium,* typiquement une bactérie solvantogène du genre *Clostridium,* caractérisé en ce qu'il comprend une étape d'introduction dans la bactérie d'un outil génétique selon l'invention. L'invention concerne également les bactéries du genre *Clostridium* ainsi transformée et/ou modifiée génétiquement.

Les inventeurs décrivent également un kit pour transformer et/ou modifier génétiquement une bactérie du genre *Clostridium,* ou pour produire au moins un solvant, par exemple un mélange de solvants, à l'aide d'une bactérie du genre *Clostridium* comprenant les éléments de l'outil génétique selon l'invention, et éventuellement, notamment, un ou plusieurs inducteurs adaptés au(x) promoteur(s) inductible(s) sélectionné(s) utilisé(s) au sein de l'outil.

Sont également décrites les utilisations de l'outil génétique selon l'invention, du procédé pour transformer et/ou modifier génétiquement par recombinaison homologue une bactérie du genre *Clostridium,* de la bactérie ainsi transformée et/ou modifiée génétiquement pour permettre la production d'un solvant ou d'un mélange de solvants à l'échelle industrielle, de préférence d'acétone, de butanol, d'éthanol, d'isopropanol ou d'un mélange de ceux-ci, typiquement d'un mélange isopropanol/butanol.

### DESCRIPTION DETAILLEE DE L'INVENTION

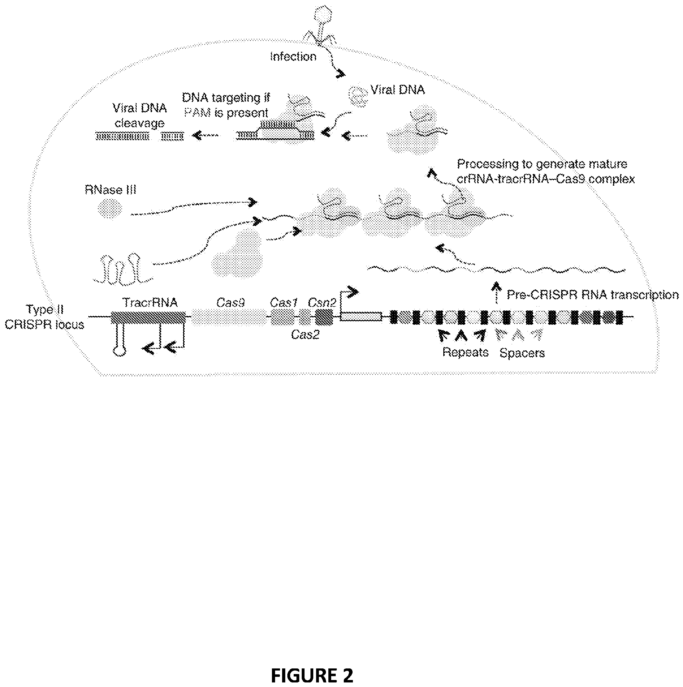

CRISPR (Clustered Regularly Interspaced Short Palindromic Repeats) désigne chez les bactéries et les archées des locus de gènes ayant un rôle de défense immunitaire contre les phages et plasmides. Le système CRISPR-Cas9 repose essentiellement sur l'association d'une protéine Cas9 réalisant des coupures double-brins dans le génome cible et d'un ARN guide (ARNg) responsable de la spécificité du site de clivage. Cette capacité à créer des coupures double-brins ciblées dans l'ADN permet de favoriser les événements de recombinaison homologue nécessaires pour introduire des mutations dans le génome des souches d'intérêt. La viabilité cellulaire dépend de l'intégrité du génome. La bactérie devra réparer toute coupure dans son ADN, que ce soit par un mécanisme de jonction d'extrémités non homologues, ou par un mécanisme de recombinaison homologue nécessitant une matrice de réparation. En fournissant à la cellule une telle matrice, il est alors possible de modifier la région correspondant au site de coupure (Figure 2). La capacité de CRISPR à effectuer des coupures double-brin dans des molécules d'ADN a permis son utilisation récente comme outil génétique chez différents organismes, en particulier *Streptococcus pyogenes,* chez qui il a d'abord été caractérisé, chez *E. coli,* et au sein de cellules eucaryotes (Jiang *et al,* 2013 ; Cong *et al,* 2013 ; Hwang *et al,* 2013 ; Hsu *et al,* 2013). Il a dernièrement été utilisé chez *Clostridium beijerinckii* et *Clostridium cellulolyticum* à l'aide d'outils génétiques qui n'ont permis qu'une modification limitée du génome bactérien, inexploitable à l'échelle industrielle (Wang *et al.,* 2015 ; Xu *et al.,* 2015).

Un outil génétique permettant la transformation par recombinaison homologue d'une bactérie du genre *Clostridium* et comprenant au moins deux acides nucléiques distincts est pour la première fois décrit dans le présent texte. Les inventeurs ont apporté la démonstration que cet outil permet de transformer et/ou modifier génétiquement les bactéries solvantogènes du genre *Clostridium,* de manière suffisamment efficace pour les rendre notamment exploitables sur le plan industriel, répondant ainsi à un besoin exprimé depuis longtemps.

Un outil génétique particulier décrit par les inventeurs, permettant la transformation par recombinaison homologue d'une bactérie du genre *Clostridium,* comprend :
- un premier acide nucléique codant au moins Cas9, dans lequel la séquence codant Cas9 est placée sous le contrôle d'un promoteur, et
- au moins un deuxième acide nucléique contenant une matrice de réparation permettant, par un mécanisme de recombinaison homologue, le remplacement d'une portion de l'ADN bactérien ciblée par Cas9 par une séquence d'intérêt,
étant entendu que i) au moins l'un desdits acides nucléiques code en outre un ou plusieurs ARN guides (ARNg) ou que ii) l'outil génétique comprend en outre un ou plusieurs ARN guides. Dans cet outil chaque ARN guide comprend une structure ARN de fixation à l'enzyme Cas9 et une séquence complémentaire de la portion ciblée de l'ADN bactérien.

Par « bactérie du genre *Clostridium* », on entend en particulier les espèces de *Clostridium* dites d'intérêt industriel, typiquement les bactéries solvantogènes du genre *Clostridium.* L'expression « bactérie du genre *Clostridium* » englobe les bactéries sauvages ainsi que les souches dérivées de celles-ci modifiées génétiquement dans le but d'améliorer leur performance (par exemple surexprimant les gènes *ctfA, ctfB* et *adc*) sans avoir été exposées au Système CRISPR.
Par « espèce de *Clostridium* d'intérêt industriel » on entend les espèces capables de produire, par fermentation, des solvants à partir d'oses tels que le glucose, le xylose, le fructose ou le mannose, de polyosides tels que la cellulose ou les hémicelluloses, d'acides tels que l'acide butyrique ou l'acide acétique, ou de toute autre source de carbone assimilable et utilisable par les bactéries du genre *Clostridium* (CO, CO₂, et méthanol par exemple). Des exemples de bactéries solvantogènes d'intérêt sont les bactéries du genre *Clostridium* productrices d'acétone, de butanol, d'éthanol et/ou d'isopropanol, telles que les souches identifiées dans la littérature en tant que « souche ABE » [souches réalisant des fermentations permettant la production d'acétone, de butanol et d'éthanol] et « souche IBE » [souches réalisant des fermentations permettant la production d'isopropanol (par réduction de l'acétone), de butanol et d'éthanol]. Des bactéries solvantogènes du genre *Clostridium* peuvent être sélectionnées parmi *C. acetobutylicum, C. cellulolyticum, C. phytofermentans, C. beijerinckii, C. saccharobutylicum, C. saccharoperbutylacetonicum, C. sporogenes, C. butyricum, C. aurantibutyricum et C. tyrobutyricum,* de manière préférée parmi *C*. *acetobutylicum, C. beijerinckii, C. butyricum et C. tyrobutyricum* et *C*. *cellulolyticum* et de manière encore plus préférée parmi *C*. *acetobutylicum* et *C*. *beijerinckii.*
Dans un mode de réalisation particulier, la bactérie du genre *Clostridium* concernée est une « souche ABE », de préférence la souche ATCC824 de *C*. *acetobutylicum* ou la souche NCIMB 8052 de *C*. *beijerinckii.*
Dans un autre mode de réalisation particulier, la bactérie du genre *Clostridium* concernée est une « souche IBE », de préférence la souche DSM 6423 (également identifiée en tant que souche NRRL B593) de C. *beijerinckii.*

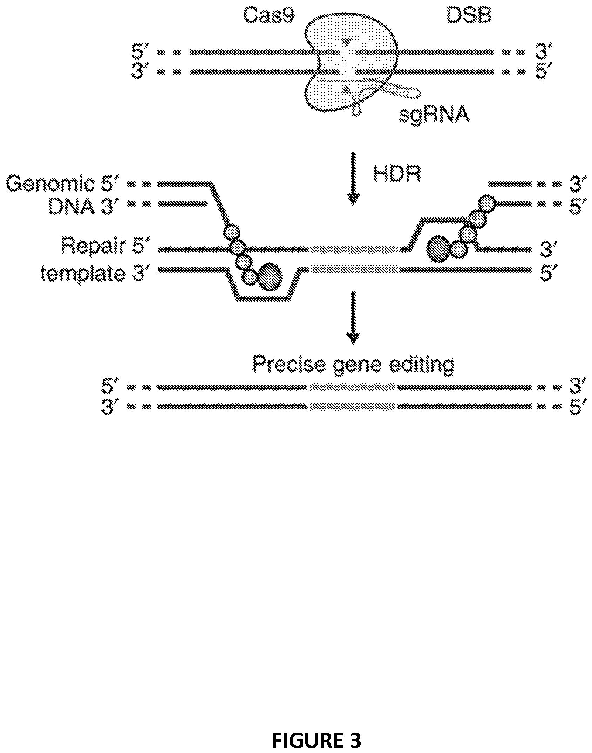

Le système CRISPR contient deux éléments distincts, i.e. i) une endonucléase, dans le cas présent la nucléase associée au système CRISPR (Cas ou « CRISPR associated protein »), Cas9, et ii) un ARN guide. L'ARN guide se présente sous la forme d'un ARN chimérique qui consiste en la combinaison d'un ARN bactérien CRISPR (ARNcr) et d'un ARNtracr (*trans*-activating ARN CRISPR) (Jinek et al., Science 2012 - cf. figure 3). L'ARNg combine la spécificité de ciblage de l'ARNcr correspondant aux "séquences espaçantes" qui servent de guides aux protéines Cas, et les propriétés conformationnelles de l'ARNtracr en un transcrit unique. Lorsque l'ARNg et la protéine Cas9 sont exprimés simultanément dans la cellule, la séquence génomique cible peut être modifiée ou interrompue de manière permanente. La modification est avantageusement guidée par une matrice de réparation.
Un outil génétique décrit par les inventeurs comprend un premier acide nucléique codant au moins Cas9. Par « Cas9 » on entend une protéine Cas9 (aussi appelée Csn1 ou Csx12) ou un fragment protéique, peptidique ou polypeptidique fonctionnel de celle-ci, i.e. capable d'interagir avec le/les ARN guides et d'exercer l'activité enzymatique (nucléasique) qui lui permet de réaliser la coupure double-brins de l'ADN du génome cible. « Cas9 » peut ainsi désigner une protéine modifiée, par exemple tronquée afin de supprimer les domaines de la protéine non essentiels aux fonctions prédéfinies de la protéine, en particulier les domaines non nécessaires à l'interaction avec le/les ARNg.
La séquence codant Cas9 (la protéine entière ou un fragment de celle-ci) telle qu'utilisée dans le cadre de l'invention peut être obtenue à partir de toute protéine Cas9 connue (Makarova *et al.,* 2011). Des exemples de protéines Cas9 utilisables dans la présente invention incluent, sans y être limités, les protéines Cas9 de *Streptococcus pyogenes, Streptococcus thermophilus, Streptococcus mutans, Campylobacter jejuni, Francisella novicida* et *Neisseria meningitidis.* D'autres protéines Cas9 utilisables dans la présente invention sont également décrites dans l'article de Fonfara *et al,* 2013. Dans un mode de réalisation particulier, la protéine Cas9, ou un fragment protéique, peptidique ou polypeptidique fonctionnel de celle-ci, codée par l'un des acides nucléiques de l'outil génétique selon l'invention comprend, ou consiste en, la séquence d'acides aminés SEQ ID NO : 1, ou toute autre séquence d'acides aminés ayant au moins 50%, de préférence au moins 60%, d'identité avec celle-ci, et contenant au minimum les deux acides aspartiques (« D ») occupant les positions 10 (« D10 ») et 840 (« D840 ») de la séquence d'acides aminés SEQ ID NO : 1.
Dans un mode de réalisation préféré, Cas9 comprend, ou consiste en, la protéine Cas9 (Numéro d'entrée NCBI: WP_010922251.1, SEQ ID NO: 1), codée par le gène *cas9* de la souche de *Streptococcus pyogenes* M1 GAS (Numéro d'entrée NCBI : NC_002737.2 SPy_1046, SEQ ID NO : 2) ou une version de celui-ci ayant subi une optimisation (« version optimisée ») à l'origine d'un transcrit contenant les codons utilisés préférentiellement par les bactéries du genre *Clostridium,* typiquement les codons riches en bases adénines (« A ») et thymines (« T »), permettant une expression facilitée de la protéine Cas9 au sein de ce genre bactérien. Ces codons optimisés respectent le biais d'usage de codons, bien connu de l'homme de l'art, spécifique à chaque souche bactérienne. Dans les séquences peptidiques décrites dans ce document, les acides aminés sont représentés par leur code à une lettre selon la nomenclature suivante : C : cystéine; D : acide aspartique; E : acide glutamique; F : phénylalanine; G : glycine; H : histidine; I : isoleucine; K : lysine; L : leucine ; M : méthionine ; N : asparagine ; P : proline ; Q : glutamine ; R : arginine ; S : sérine ; T : thréonine ; V : valine ; W : tryptophane et Y : tyrosine.

Selon un mode de réalisation particulier, le domaine Cas9 est constitué d'une protéine Cas9 entière, de préférence la protéine Cas9 de *Streptococcus pyogenes* ou d'une version optimisée de celle-ci.

La séquence codant Cas9 présente au sein de l'un des acides nucléiques de l'outil génétique selon l'invention est placée sous le contrôle d'un promoteur. Ce promoteur peut être un promoteur constitutif ou un promoteur inductible. Dans un mode de réalisation préféré, le promoteur contrôlant l'expression de Cas9 est un promoteur inductible.
Des exemples de promoteurs constitutifs utilisables dans le cadre de la présente invention peuvent être sélectionnés parmi le promoteur du gène *thl,* du gène *ptb,* du gène *adc,* de l'opéron *bcs,* ou un dérivé de ceux-ci, de préférence un dérivé fonctionnel mais plus court (tronqué) tel que le dérivé « miniPthl » du promoteur du gène *thl* de *C. acetobutylicum* (Dong *et al.,* 2012), ou tout autre promoteur, bien connu de l'homme de métier, permettant l'expression d'une protéine au sein de *Clostridium.*
Des exemples de promoteurs inductibles utilisables dans le cadre de la présente invention peuvent être sélectionnés par exemple parmi un promoteur dont l'expression est contrôlée par le répresseur transcriptionnel TetR, par exemple le promoteur du gène *tetA* (gène de résistance à la tétracycline originellement présent sur le transposon Tn10 d'*E. coli*) ; un promoteur dont l'expression est contrôlée par la L-arabinose, par exemple le promoteur du gène *ptk* (cf. Zhang J. *et al.,* 2015), de préférence en combinaison avec la cassette *araR* de régulation de l'expression de *C. acetobutylicum* de façon à construire un système ARAi (cf. Zhang J. *et al.,* 2015) ; un promoteur dont l'expression est contrôlée par la laminaribiose (dimère de glucose β-1,3), par exemple le promoteur du gène *celC,* de préférence immédiatement suivi du gène répresseur glyR3 et du gène d'intérêt (cf. Meals EB *et al.* (2015)) ou le promoteur du gène *celC* (cf. Newcomb M. *et al.,* 2011) ; un promoteur dont l'expression est contrôlée par le lactose, par exemple le promoteur du gène *bgaL,* de préférence immédiatement suivi du gène *AdhE1* (aldehyde/alcohol dehydrogenase) (cf. Banerjee *et al.,* 2014) ; un promoteur dont l'expression est contrôlée par le xylose, par exemple le promoteur du gène *xylB* (cf. Nariya H *et al.,* 2011) ; et promoteur dont l'expression est contrôlée par l'exposition aux UV, par exemple le promoteur *bcn* (cf. Dupuy *et al.,* 2005).
Un promoteur dérivé de l'un des promoteurs décrits ci-dessus, de préférence un dérivé fonctionnel mais plus court (tronqué) peut également être avantageusement utilisé dans le contexte de l'invention. D'autres promoteurs inductibles utilisables dans la présente invention sont également décrits par exemple dans les articles de Ransom EM et al. (2015), Currie DH *et al.* (2013), D'Urzo N *et al.* (2013) et Hartman AH *et al.* (2011).

Un promoteur inductible préféré est un promoteur dérivé de *tetA,* inductible à l'anhydrotétracycline (aTc) (moins toxique que la tétracycline et capable de lever l'inhibition du répresseur transcriptionnel TetR à plus faible concentration), choisi parmi Pcm-2tetO1 et Pcm-2tetO2/1 (Dong *et al.,* 2012).
Un autre promoteur inductible préféré est un promoteur dérivé de *xylB,* inductible par le xylose, par exemple le promoteur *xylB* de *Clostridium difficile* 630 (Nariya *et al.,* 2011).

Les promoteurs inductibles tels que décrits dans la présente invention permettent de maîtriser avantageusement l'action de l'enzyme et de faciliter la sélection de transformants ayant subis les modifications génétiques souhaitées.

Le terme « ARN guide » ou « ARNg » désigne au sens de l'invention une molécule d'ARN capable d'interagir avec « Cas9 » afin de la guider vers une région cible du chromosome bactérien. La spécificité de coupure est déterminée par l'ARNg. Comme expliqué précédemment, chaque ARNg comprend deux régions :
- une première région (communément appelée région « SDS »), à l'extrémité 5' de l'ARNg, qui est complémentaire de la région chromosomique cible et qui imite l'ARNcr du système CRISPR endogène, et
- une seconde région (communément appelé région « handle »), à l'extrémité 3' de l'ARNg, qui mime les interactions d'appariement de bases entre l'ARNtracr (« trans-activating crRNA ») et l'ARNcr du système CRISPR endogène et présente une structure double brin en tige et boucle s'achevant en 3' par une séquence essentiellement simple brin. Cette seconde région est essentielle à la fixation de l'ARNg à Cas9.
La première région de l'ARNg (région « SDS ») varie selon la séquence chromosomique ciblée.
La région « SDS » de l'ARNg qui est complémentaire de la région chromosomique cible, comprend au moins 1 nucléotide, de préférence au moins 1, 2, 3, 4, 5, 10, 15, 20, 25, 30, 35 ou 40 nucléotides, typiquement entre 1 et 40 nucléotides. De préférence, cette région a une longueur de 20, 21, 22, 23, 24, 25, 26, 27, 28, 29 ou 30 nucléotides.
La seconde région de l'ARNg (région « handle ») a une structure en tige et boucle (ou structure en épingle à cheveux). Les régions « handle » des différents ARNg ne dépendent pas de la cible chromosomique choisie.
Selon un mode de réalisation particulier, la région « handle » comprend, ou consiste en, une séquence d'au moins 1 nucléotide, de préférence au moins 1, 50, 100, 200, 500 et 1000 nucléotides, typiquement entre 1 et 1000 nucléotides. De préférence, cette région a une longueur de 40 à 120 nucléotides.
La longueur totale d'un ARNg est en général de 50 à 1000 nucléotides, de préférence de 80 à 200 nucléotides, et de manière plus particulièrement préférée de 90 à 120 nucléotides. Selon un mode de réalisation particulier, un ARNg tel qu'utilisé dans la présente invention a une longueur comprise entre 95 et 110 nucléotides, par exemple une longueur d'environ 100 ou d'environ 110 nucléotides.

L'homme du métier peut aisément définir la séquence et la structure des ARNg selon la région chromosomique à cibler en utilisant des techniques bien connues (voir par exemple l'article de Di Carlo *et al.,* 2013).
La région/portion/séquence d'ADN ciblée au sein du chromosome bactérien peut correspondre à une portion d'ADN non codante ou à une portion d'ADN codante. Dans un mode de réalisation particulier, la portion ciblée de l'ADN bactérien comprend un ou plusieurs gènes ou portion(s) de gène essentiel(le) à la survie bactérienne ou encore un ou plusieurs gènes ou séquences d'ADN dont l'inactivation permet la sélection des bactéries ayant intégré le/les acides nucléiques d'intérêt.
Des exemples particuliers de portion d'ADN ciblée au sein d'une bactérie du genre *Clostridium* sont les séquences utilisées dans la partie expérimentale. Il s'agit par exemple des séquences codant les gènes *upp* (SEQ ID NO : 3) et *adhE1* (SEQ ID NO : 4).
La région/portion/séquence d'ADN ciblée est suivie d'une séquence « PAM » (« protospacer adjacent motif ») qui intervient dans la liaison à Cas9.

La région « SDS » d'un ARNg donné est identique (à 100%) ou identique à 80% au moins, de préférence à 85%, 90%, 95%, 96%, 97%, 98% ou 99% au moins à la région/portion/séquence d'ADN ciblée au sein du chromosome bactérien et est capable de s'hybrider à tout ou partie de la séquence complémentaire de ladite région/portion/séquence, typiquement à une séquence comprenant au moins 1 nucléotide, de préférence au moins 1, 2, 3, 4, 5, 10, 15, 20, 25, 30, 35 ou 40 nucléotides, typiquement entre 1 et 40 nucléotides, de préférence à une séquence comprenant 20, 21, 22, 23, 24, 25, 26, 27, 28, 29 ou 30 nucléotides.

Dans le procédé décrit par les inventeurs, un ou plusieurs ARNg peuvent être utilisés simultanément. Ces différents ARNg peuvent cibler des régions chromosomiques identiques ou différentes, de préférence différentes.
Les ARNg peuvent être introduits dans la cellule bactérienne sous la forme de molécules d'ARNg (matures ou précurseurs), sous la forme de précurseurs ou sous la forme d'un ou plusieurs acides nucléiques codant lesdits ARNg. Les ARNg sont de préférence introduits dans la cellule bactérienne sous la forme d'un ou plusieurs acides nucléiques codant lesdits ARNg.
Lorsque le ou les ARNg sont introduits dans la cellule directement sous la forme de molécules d'ARN, ces ARNg (matures ou précurseurs) peuvent contenir des nucléotides modifiés ou des modifications chimiques leur permettant, par exemple, d'augmenter leur résistance aux nucléases et ainsi d'augmenter leur durée de vie dans la cellule. Ils peuvent notamment comprendre au moins un nucléotide modifié ou non naturel tel que, par exemple, un nucléotide comportant une base modifiée, telle que l'inosine, la méthyl-5-désoxycytidine, la diméthylarnino-5-désoxyuridine, la désoxyuridine, la diamino-2,6-purine, la bromo-5-désoxyuridine ou toute autre base modifiée permettant l'hybridation. Les ARNg utilisés dans le cadre de l'invention peuvent également être modifiés au niveau de la liaison internucléotidique comme par exemple les phosphorothioates, les H-phosphonates ou les alkyl-phosphonates, ou au niveau du squelette comme par exemple les alpha-oligonucléotides, les 2'-O-alkyl ribose ou les PNA (Peptid Nucleic Acid) (Egholm *et al.,* 1992).
Les ARNg peuvent être des ARN naturels, synthétiques ou produits par des techniques de recombinaison. Ces ARNg peuvent être préparés par toutes méthodes connues de l'homme du métier telles que, par exemple, la synthèse chimique, la transcription *in vivo* ou des techniques d'amplification.
Lorsque les ARNg sont introduits dans la cellule bactérienne sous la forme d'un ou plusieurs acides nucléiques, la ou les séquences codant le ou les ARNg sont placées sous le contrôle d'un promoteur d'expression. Ce promoteur peut être constitutif ou inductible.
Lorsque plusieurs ARNg sont utilisés, l'expression de chaque ARNg peut être contrôlée par un promoteur différent. De préférence, le promoteur utilisé est le même pour tous les ARNg. Un même promoteur peut dans un mode de réalisation particulier être utilisé pour permettre l'expression de plusieurs, par exemple de quelques-uns seulement, des ARNg destinés à être exprimés.
Dans un mode de réalisation préféré, le/les promoteurs contrôlant l'expression du/des ARNg est/sont des promoteurs constitutifs.
Des exemples de promoteurs constitutifs utilisables dans le cadre de la présente invention peuvent être sélectionnés parmi le promoteur du gène *thl,* du gène *ptb* ou de l'opéron *bcs,* ou un dérivé de ceux-ci, de préférence miniPthl, ou tout autre promoteur, bien connu de l'homme de métier, permettant la synthèse d'un ARN (codant ou non codant) au sein de *Clostridium.*
Des exemples de promoteurs inductibles utilisables dans le cadre de la présente invention peuvent être sélectionnés parmi le promoteur du gène *tetA,* du gène *xylA,* du gène *lacI,* ou du gène *bgaL,* ou un dérivé de ceux-ci, de préférence 2tetO1 ou tetO2/1. Un promoteur inductible préféré est tetO2/1.

Les promoteurs contrôlant l'expression de Cas9 et du/des ARNg peuvent être identiques ou différents et constitutifs ou inductibles. Dans un mode de réalisation particulier et préféré de l'invention, l'un seulement des promoteurs contrôlant respectivement l'expression de Cas9 ou du/des ARNg est un promoteur inductible.

Par « acide nucléique », on entend au sens de l'invention, toute molécule d'ADN ou d'ARN naturelle, synthétique, semi-synthétique ou recombinante, éventuellement modifiée chimiquement (i.e. comprenant des bases non naturelles, des nucléotides modifiés comprenant par exemple une liaison modifiée, des bases modifiées et/ou des sucres modifiés), ou optimisée de manière à ce que les codons des transcrits synthétisés à partir des séquences codantes soient les codons les plus fréquemment trouvés chez une bactérie du genre *Clostridium* en vue de son utilisation chez celle-ci. Comme expliqué précédemment, dans le cas du genre *Clostridium,* les codons optimisés sont typiquement des codons riches en bases adénines (« A ») et thymines (« T »).
Chacun des acides nucléiques présents au sein de l'outil génétique décrit, typiquement le « premier » acide nucléique et le « deuxième » acide nucléique, consiste en une entité distincte et correspond par exemple i) à une cassette d'expression (ou « construction ») telle qu'un acide nucléique comprenant au moins un promoteur transcriptionnel lié de manière opérationnelle (au sens où l'entend l'homme du métier) à une ou plusieurs séquences (codantes) d'intérêt, typiquement à un opéron comprenant plusieurs séquence codantes d'intérêt dont les produits d'expression concourent à la réalisation d'une fonction d'intérêt au sein de la bactérie, ou tel qu'un acide nucléique comprenant en outre une séquence activatrice et/ou un terminateur de transcription ; ou ii) à un vecteur, circulaire ou linéaire, simple ou double-brins, par exemple un plasmide, un phage, un cosmide, un chromosome artificiel ou synthétique, comprenant une ou plusieurs cassettes d'expression telles que définies ci-dessus. De manière préférée, le vecteur est un plasmide.

Les cassettes et vecteurs d'expression peuvent être construits par des techniques classiques bien connues de l'homme du métier et peuvent comporter un(e) ou plusieurs promoteurs, origines de réplication bactérienne (séquences ORI), séquences de terminaison, gènes de sélection, par exemple gènes de résistance à un antibiotique, et séquences (« régions flanquées ») permettant l'insertion ciblée de la cassette ou du vecteur. Par ailleurs, ces cassettes et vecteurs d'expression peuvent être intégrées au sein du génome par des techniques bien connues de l'homme du métier.
Des séquences ORI d'intérêt peuvent être choisies parmi pIP404, pAMβ1, repH (origine de réplication chez *C. acetobutylicum*)*,* ColE1 ou rep (origine de réplication chez *E. coli*)*,* ou tout autre origine de réplication permettant le maintien du vecteur, typiquement du plasmide, au sein d'une cellule de *Clostridium.*
Des séquences de terminaison d'intérêt peuvent être choisies parmi celles des gènes *adc, thl,* de l'opéron *bcs,* ou de tout autre terminateur, bien connu de l'homme de l'art, permettant l'arrêt de la transcription au sein de *Clostridium.*
Des gènes de sélection d'intérêt peuvent être choisis parmi *ermB, catP, bla, tetA, tetM,* et tout autre gène de résistance à l'ampicilline, à l'érythromycine, au chloramphenicol, au thiamphénicol, à la tétracycline ou à tout autre antibiotique pouvant être utilisé pour sélectionner des bactéries du genre *Clostridium* bien connu de l'homme de l'art.
Un vecteur particulier comprend une ou plusieurs cassettes d'expression, chaque cassette codant un ARNg.
Dans un mode de réalisation particulier, la description concerne un outil génétique comprenant en tant que « premier » acide nucléique tel qu'identifié dans les revendications un vecteur plasmidique dont la séquence est sélectionnée parmi l'une des séquences SEQ ID NO : 5, SEQ ID NO : 6 et SEQ ID NO : 7.

Dans un mode de réalisation particulier, la description concerne un outil génétique comprenant en tant que « deuxième » ou « énième » acide nucléique un vecteur plasmidique dont la séquence est sélectionnée parmi l'une des séquences SEQ ID NO : 8, SEQ ID NO : 9, SEQ ID NO : 10 et SEQ ID NO : 11.

La séquence d'intérêt est introduite dans le génome bactérien via un mécanisme de recombinaison homologue guidé par une matrice de réparation sélectionnée (selon la technologie CRISPR). La séquence d'intérêt vient remplacer la portion ciblée au sein du génome bactérien. Le processus de recombinaison permet ainsi la modification ou la délétion total(le) ou partiel(le) de la portion ciblée au sein du génome de la bactérie ou permet l'insertion de fragments (dans un mode de réalisation particulier de grands fragments) d'acide nucléique dans le génome de la bactérie. La matrice de réparation sélectionnée peut en effet comprendre tout ou partie de la séquence ciblée du génome bactérien ou une version plus ou moins modifiée de celle-ci selon la nature de la transformation souhaitée. Tout comme la portion d'ADN ciblée, la matrice peut ainsi elle-même comprendre une ou plusieurs séquences d'acide nucléique ou portions de séquence d'acide nucléique correspondant à des séquences naturelles et/ou synthétiques, codantes et/ou non codantes. La matrice peut par exemple comprendre une ou plusieurs séquences ou portion(s) de séquences correspondant à un gène essentiel à la survie bactérienne, en particulier à la survie des bactéries du genre *Clostridium,* ou encore à un ou plusieurs gènes ou séquences d'ADN dont l'inactivation permet la sélection des bactéries du genre *Clostridium* ayant intégré le/les acides nucléiques d'intérêt. La matrice peut également comprendre une ou plusieurs séquences « étrangères », i.e. naturellement absentes du génome des bactéries appartenant au genre *Clostridium* ou du génome d'espèces particulières dudit genre. La matrice peut aussi comprendre une combinaison de séquences telles que décrites ci-dessus.
Des exemples particuliers de séquences d'intérêt sont les séquences utilisées dans la partie expérimentale. Il s'agit par exemple des séquences upp_del (SEQ ID NO : 12) et upp_stop (SEQ ID NO : 13).
L'outil génétique selon l'invention permet à la matrice de réparation de guider l'incorporation au sein du génome bactérien des bactéries du genre *Clostridium* d'un acide nucléique d'intérêt, typiquement d'une séquence ou portion de séquence d'ADN comprenant au moins 1 paire de base (pb), de préférence au moins 1, 2, 3, 4, 5, 10, 15, 20, 50, 100, 1 000, 10 000, 100 000 ou 1 000 000 pb, typiquement entre 1 pb et 20 kb ou entre 1 pb et 10 kb, de préférence entre 10 pb et 20 kb ou entre 10 pb et 10 kb, par exemple entre 1pb et 2 kb.

Dans un mode de réalisation particulier, la séquence d'ADN d'intérêt code au moins un produit d'intérêt, de préférence un produit favorisant la production de solvant, typiquement au moins une protéine d'intérêt, par exemple une enzyme ; une protéine membranaire tel qu'un transporteur ; un facteur de transcription ; ou une combinaison de ceux-ci.

Dans un mode de réalisation préféré, la séquence d'ADN d'intérêt favorise la production de solvant et est typiquement sélectionnée parmi une séquence codant pour i) une enzyme, de préférence une enzyme impliquée dans la conversion des aldéhydes en alcool, par exemple sélectionnée parmi une séquence codant pour une alcool déshydrogénase (par exemple une séquence sélectionnée parmi *adh, adhE, adhE1, adhE2, bdhA,* et *bdhB*), une séquence codant pour une transférase (par exemple une séquence sélectionnée parmi *ctfA, ctfB, atoA* et *atoB*)*,* une séquence codant pour une décarboxylase (par exemple *adc*)*,* une séquence codant pour une hydrogénase (par exemple une séquence sélectionnée parmi *etfA, etfB* et *hydA*), et une combinaison de celles-ci, ii) une protéine membranaire, par exemple une séquence codant pour une phosphotransférase (par exemple une séquence sélectionnée parmi *glcG*, *bglC*, *cbe4532, cbe4533, cbe4982, cbe4983, cbe0751*)*,* et iii) un facteur de transcription (par exemple une séquence sélectionnée parmi *sigE, sigF, sigG, sigH, sigK*)*.*

La présente description concerne par ailleurs un procédé pour transformer et/ou modifier génétiquement par recombinaison homologue une bactérie du genre *Clostridium,* de préférence une bactérie solvantogène du genre *Clostridium.* Ce procédé comprend une étape d'introduction dans la bactérie d'un outil génétique selon l'invention tel que décrit dans la présente demande. Le procédé peut comprendre en outre une étape d'obtention de la bactérie transformée, i.e. de la bactérie présentant la ou les recombinaisons/optimisations recherchées.

Un procédé particulier permettant de transformer et/ou modifier génétiquement par recombinaison homologue une bactérie solvantogène du genre *Clostridium,* comprend, dans l'ordre, les étapes suivantes :
a) l'introduction dans la bactérie d'un outil génétique selon l'invention tel que décrit dans la présente demande comprenant au moins un promoteur inductible, et
b) l'induction de l'expression du promoteur inductible permettant de modifier génétiquement la bactérie.

L'introduction dans la bactérie des éléments (acides nucléiques ou ARNg) de l'outil génétique décrit par les inventeurs est réalisée par toute méthode, directe ou indirecte, connue de l'homme du métier, par exemple par transformation, conjugaison, microinjection, transfection, electroporation, etc., de préférence par transformation (Lütke-Eversloh, 2014).

L'étape d'induction lorsqu'elle est nécessaire peut être mise en œuvre par toute méthode connue de l'homme du métier après introduction dans la bactérie cible de l'outil génétique décrit par les inventeurs. Elle est par exemple réalisée par mise en contact de la bactérie avec une substance adéquate, présente en quantité suffisante ou par exposition à la lumière UV. Cette substance permet de lever l'inhibition d'expression liée au promoteur inductible sélectionné. Lorsque le promoteur sélectionné est un promoteur inductible à l'anhydrotétracycline (aTc), choisi parmi Pcm-2tetO1 et Pcm-tetO2/1, l'aTc est de préférence utilisée à une concentration comprise entre environ 1 ng/ml et environ 5000 ng/ml, de préférence entre environ 100 ng par ml et environ 500 ng/ml ou entre environ 200 ng par ml et environ 300 ng/ml, par exemple environ 250 ng/ml.

Dans un mode de réalisation particulier, le procédé comprend une ou plusieurs étapes additionnelles, postérieures à l'étape b) lorsqu'elle est présente, d'introduction d'un énième, par exemple troisième, quatrième, cinquième, etc., acide nucléique codant i) une matrice de réparation distincte de celle(s) déjà introduite(s) et ii) un ou plusieurs ARN guides permettant leur intégration dans une zone ciblée du génome de la bactérie, chaque étape additionnelle étant de préférence avantageusement précédée d'une étape d'élimination de l'acide nucléique codant la matrice de réparation préalablement introduite, la cellule bactérienne étant alors considérée comme « curée » dudit acide nucléique, et de préférence d'une étape d'élimination du/des ARNs guides ou séquences codant le/les ARNs guides préalablement introduits.

De manière particulièrement avantageuse et contrairement aux outils disponibles dans l'art antérieur, l'outil génétique décrit par les inventeurs permet l'introduction de séquences d'intérêt de petites tailles aussi bien que de grandes tailles, en une étape, i.e. à l'aide d'un seul acide nucléique (typiquement le « deuxième » acide nucléique tel que décrit dans le présent texte) ou en plusieurs étapes, i.e. à l'aide de plusieurs acides nucléiques (typiquement le « deuxième » et le ou les « énième » acides nucléiques tels que décrits dans le présent texte), de préférence en une étape.
Dans mode de réalisation particulier, ce « énième » acide nucléique permet de supprimer la portion ciblée de l'ADN bactérien ou de la remplacer par une séquence plus courte (par exemple par une séquence délétée d'au moins une paire de bases) et/ou non fonctionnelle. Dans un mode de réalisation particulier préféré, le « deuxième » ou « énième » acide nucléique permet avantageusement d'introduire dans le génome bactérien un acide nucléique d'intérêt comprenant au moins une paire de base, et jusqu'à 2, 3, 4, 5, 6, 7, 8, 9, 10, 11, 12 ou 13 kb.

Les acides nucléiques d'intérêt peuvent être insérés dans le chromosome bactérien au niveau de régions identiques ou différentes selon les ARNg utilisés, et si cela s'avère utile dans une portion du génome bactérien comprenant un gène essentiel à la survie bactérienne, par exemple l'un des gènes *gyrA, pfkA, hydA, crt, thl, hbd,* ou tout autre gène connu de l'homme du métier comme essentiel à la survie d'une bactérie du genre *Clostridium,* et/ou dans un gène ou une séquence ADN dont l'inactivation permet la sélection des bactéries ayant intégré le/les acides nucléiques d'intérêt, par exemple le gène *upp.*
Grâce à l'outil génétique et au procédé décrits par les inventeurs, il est à présent possible de modifier efficacement (fréquence de recombinaison homologue performante), substantiellement (incorporation possible au sein du génome de la bactérie d'un acide nucléique d'intérêt de grande taille) et de manière stable (le maintien des bactéries transformées en contact avec des antibiotiques n'est pas nécessaire), les bactéries du genre *Clostridium* de manière à obtenir des bactéries transformées d'intérêt, par exemple des variants améliorés présentant une différence génotypique ou phénotypique par rapport à la bactérie dont elle est issue, typiquement des bactéries exploitables sur le plan industriel, par exemple des bactéries utilisables dans la production de solvants ou biocarburants.

Un autre objet décrit par les inventeurs concerne une bactérie du genre *Clostridium,* typiquement une bactérie solvantogène du genre *Clostridium,* transformée à l'aide du procédé et/ou de l'outil génétique selon l'invention. Une telle bactérie exprime le ou les acides nucléiques d'intérêt introduits dans son génome par recombinaison homologue à l'aide de la matrice de réparation. Une telle bactérie peut comprendre tout ou partie de l'outil génétique décrit par les inventeurs, typiquement Cas9 ou un acide nucléique codant Cas9.
Une bactérie du genre *Clostridium* particulière selon l'invention, par exemple une bactérie ATCC824, transformée à l'aide du procédé et de l'outil génétique selon l'invention, ne contient plus le mégaplasmide pSOL.
Dans un mode de réalisation particulier, la bactérie du genre *Clostridium* transformée à l'aide du procédé et de l'outil génétique décrits par les inventeurs, n'est en mesure de produire un ou plusieurs solvants que grâce à l'expression du ou des acides nucléiques d'intérêt introduits volontairement dans son génome.

La description concerne aussi un kit (trousse) pour transformer et/ou modifier génétiquement une bactérie du genre *Clostridium* comprenant tout ou partie des éléments de l'outil génétique tels que décrits dans le présent texte, typiquement i) un premier acide nucléique codant Cas9, dans lequel la séquence codant Cas9 est placée sous le contrôle d'un promoteur, et ii) au moins un deuxième acide nucléique codant une matrice de réparation permettant, par un mécanisme de recombinaison homologue, le remplacement d'une portion de l'ADN bactérien ciblée par Cas9 par une séquence d'intérêt, et éventuellement un ou plusieurs inducteurs adaptés au(x) promoteur(s) inductible(s) sélectionné(s) éventuellement utilisé(s) au sein de l'outil.
Un kit particulier permet l'expression d'une protéine Cas9 comprenant une étiquette (ou « tag »).
Les kits selon l'invention peuvent en outre comprendre un ou plusieurs consommables tels qu'un milieu de culture, au moins une bactérie compétente du genre *Clostridium* (i.e. conditionnée en vue de la transformation), au moins un ARNg, une protéine Cas9, une ou plusieurs molécules de sélection, ou encore une notice explicative.
La description concerne typiquement un kit pour la mise en œuvre du procédé de transformation décrit dans le présent texte ou pour la production de solvant(s) (au moins un solvant) à l'aide d'une bactérie du genre *Clostridium.*

La description concerne aussi les utilisations possibles de l'outil génétique, ou du procédé, ou du kit décrit par les inventeurs pour transformer et/ou modifier génétiquement une bactérie du genre *Clostridium,* typiquement une bactérie solvantogène du genre *Clostridium,* par exemple pour générer des variants améliorés d'une bactérie du genre *Clostridium.*
La description concerne enfin les utilisations possibles de l'outil génétique, du procédé, du kit ou d'une bactérie du genre *Clostridium* transformée comme décrit dans le présent texte, en particulier pour permettre la production de solvants ou biocarburants, ou de mélanges de ceux-ci, typiquement à l'échelle industrielle. Des solvants susceptibles d'être produits sont typiquement l'acétone, le butanol, l'éthanol, l'isopropanol ou un mélange de ceux-ci, typiquement un mélange éthanol/isopropanol, butanol/isopropanol, ou éthanol/butanol, de préférence un mélange isopropanol/butanol.
Dans un mode de réalisation particulier, le ratio du mélange éthanol/isopropanol est au moins égal à 1/4. Ce ratio est de préférence compris entre 1/3 et 1, et est de manière encore plus préférée égal à 1. Dans un mode de réalisation particulier, le ratio du mélange éthanol/butanol est au moins égal à 1/4. Ce ratio est de préférence compris entre 1/3 et 1, et est de manière encore plus préférée égal à 1.
Dans un mode de réalisation particulier, le ratio du mélange isopropanol/butanol est au moins égal à 1/4. Ce ratio est de préférence compris entre 1/3 et 1, et est de manière encore plus préférée égal à 1.

L'utilisation de bactéries transformées permet typiquement la production par an à l'échelle industrielle d'au moins 100 tonnes d'acétone, d'au moins 100 tonnes d'éthanol, d'au moins 1000 tonnes d'isopropanol, d'au moins 1800 tonnes de butanol, ou d'au moins 40 000 tonnes d'un mélange de ceux-ci.

Les exemples et figures ci-après ont pour but d'illustrer plus pleinement l'invention sans pour autant en limiter la portée.

### FIGURES

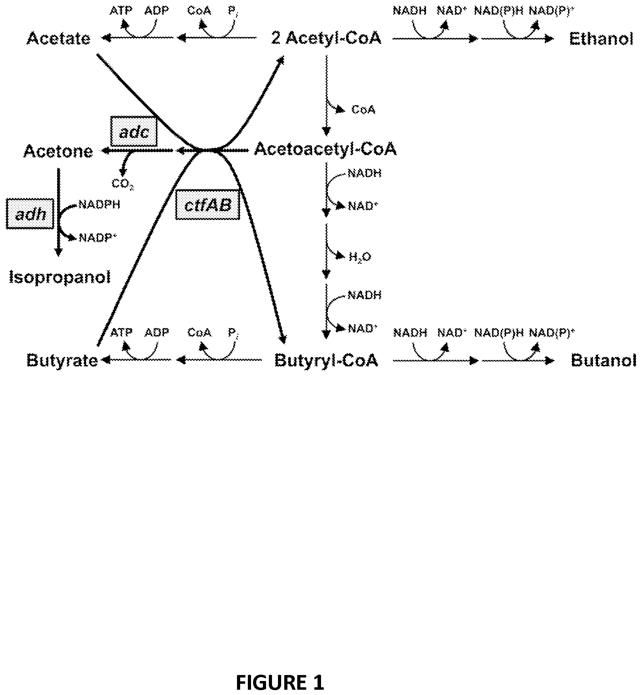

**Figure 1** : Métabolisme des souches de *Clostridium* solvantogènes. Les souches ABE produisent de l'acétone, de l'éthanol et du butanol alors que les souches IBE possèdent le gène *adh* convertissant l'acétone en isopropanol. Modifié de Lee *et al.,* 2012.
**Figure 2** **:** Mode d'action de CRISPR. Mali *et al.*
**Figure 3** **:** Utilisation de CRISPR-Cas9 pour l'édition de génome. La coupure double brins est réalisée par la nucléase Cas9, dirigée par le gRNA. La réparation de cette coupure par recombinaison homologue permet d'introduire dans le génome les modifications contenues dans la matrice de réparation. Figure modifiée depuis Ann Ran *et al.,* 2013.
**Figure 4** : Plasmides de ciblage de *upp.* pIP404, origine de réplication chez *C. acetobutylicum.* ColE1, origine de réplication chez *E. coli.* catP, gène de la chloramphénicol acétyltransférase (gène de résistance au chloramphénicol/thiamphénicol). CDS, coding sequence.
**Figure 5** **:** Plasmide de ciblage de pSOL. pIP404, origine de réplication chez *C. acetobutylicum.* ColE1, origine de réplication chez *E. coli. catP,* gène de la chloramphénicol acétyltransférase (gène de résistance au chloramphénicol/thiamphénicol). CDS, coding sequence.
**Figure 6** **:** carte du vecteur pEC500E-miniPthl-Cas9. pAMβ1, origine de réplication chez *C*. *acetobutylicum.* rep, origine de réplication chez *E. coli. bla,* gène de la β-lactamase (résistance à l'ampicilline). *ermB,* méthylase (résistance à l'érythromycine). CDS, coding sequence.
**Figure 7** **:** séquençage de la zone ciblée par *cas9* chez la souche sauvage et chez le transformant obtenu. NC_003030, séquence de Clostridium acetobutylicum ATCC824 (GenBank) ; crRNA, site reconnu par l'ARNg ; PAM, protospacer adjacent motif, intervenant dans la fixation de Cas9. CDS, coding sequence. SEQ ID NO : 22 correspond au fragment de NC_003030 apparaissant sur la Figure 7 and SEQ ID NO : 23 correspond aux fragments apparaissant sur la Figure 7 des séquences identifiées respectivement comme « matrice de réparation upp_stop », « ATCC824 » et « ATCC824 upp- ».
**Figure 8** **:** Résultats d'amplification.
   A : catP_fwd x catP_rev (taille attendue : 709pb)
   B : RH_ctfB_R x V-CTFA-CAC2707_R (taille attendue : 351pb)
   1 : Marqueur 2-Log (NEB). 2 : H 2 0, témoin négatif. 3 : ATCC824 non transformé. 4 : ATCC824 transformé avec pEC500E-miniPthl-*cas9*. 5 & 6 : ATCC824 transformé avec pEC500E-miniPthl-*cas*9 et pEC750C (2 transformants indépendants). 7 & 8 :ATCC824 transformé avec pEC500E-miniPthl-*cas9* et pEC750C-gRNA_adhE (2 transformants indépendants).

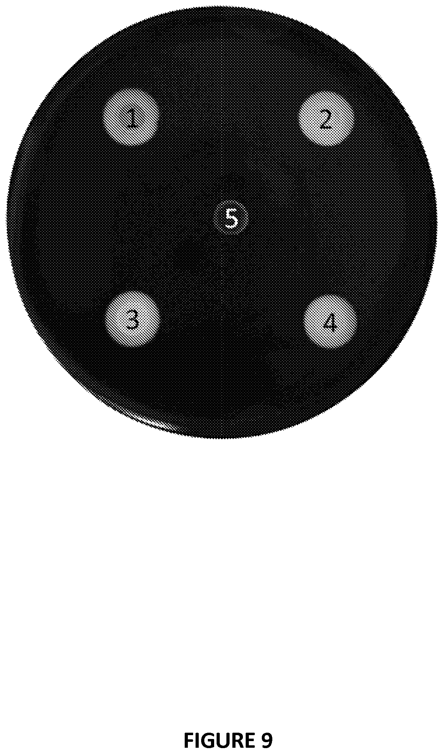

**Figure 9** **:** Détection de l'activité α-amylase des souches dérivées d'ATCC824. 1 : ATCC824 ;
   2 : ATCC824 transformé avec pEC500E et pEC750C ; 3 : ATCC824 transformé avec pEC500E-miniPthl-*cas9* et pEC750C ; 4 : ATCC824 transformé avec pEC500E et pEC750C-gRNA_*adhE* ; 5 : ATCC824 transformé avec pEC500E-miniPthl-*cas9* et pEC750C-gRNA_*adhE*

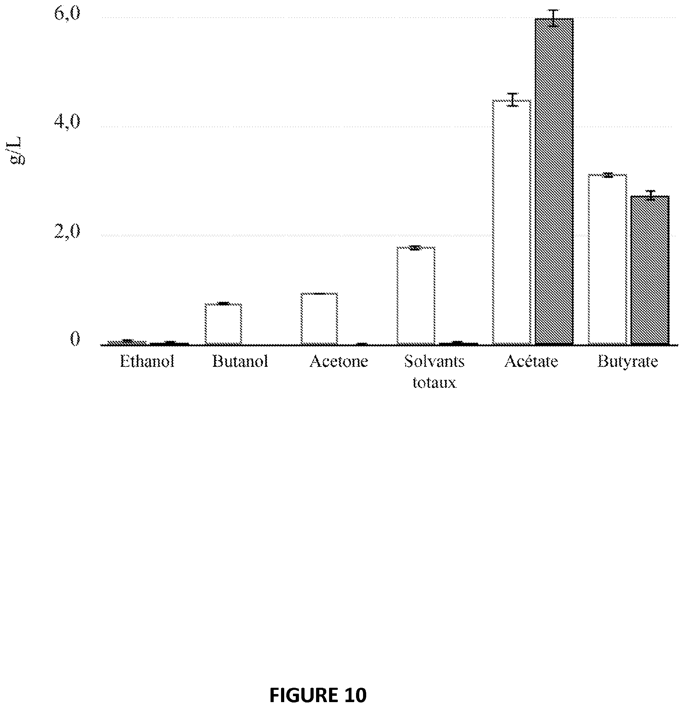

**Figure 10** : Bilans fermentaires de la souche sauvage et d'un transformant (deux réplicats techniques).

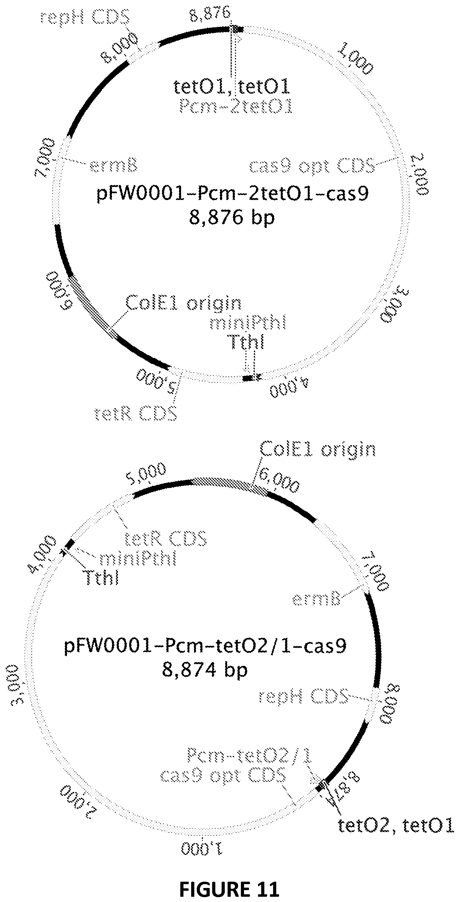

**Figure 11** : A/B. Plasmides d'expression inductible de *cas9. repH,* origine de réplication chez C. *acetobutylicum.* ColE1, origine de réplication chez *E. coli. ermB,* méthylase (résistance à l'érythromycine). *tetR,* gène codant le répresseur transcriptionnel TetR. CDS, coding sequence.

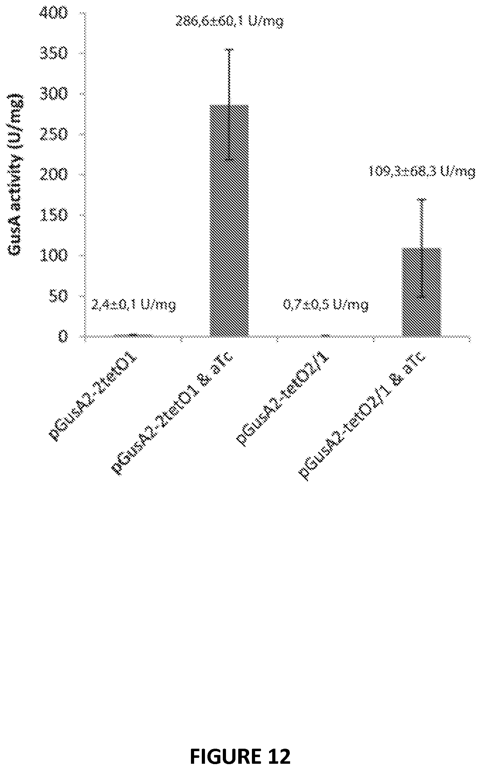

**Figure 12** : Effet de l'induction sur l'expression à partir des promoteurs Pcm-2tetO1 et Pcm-tetO2/1. Les promoteurs ont été placés en aval du gène *gusA,* et l'activité GusA a été mesurée dans des cellules de *C. acetobutylicum* ATCC824, en absence ou en présence de 100ng/mL d'aTc. Modifié de Dong *et al.,* 2012.

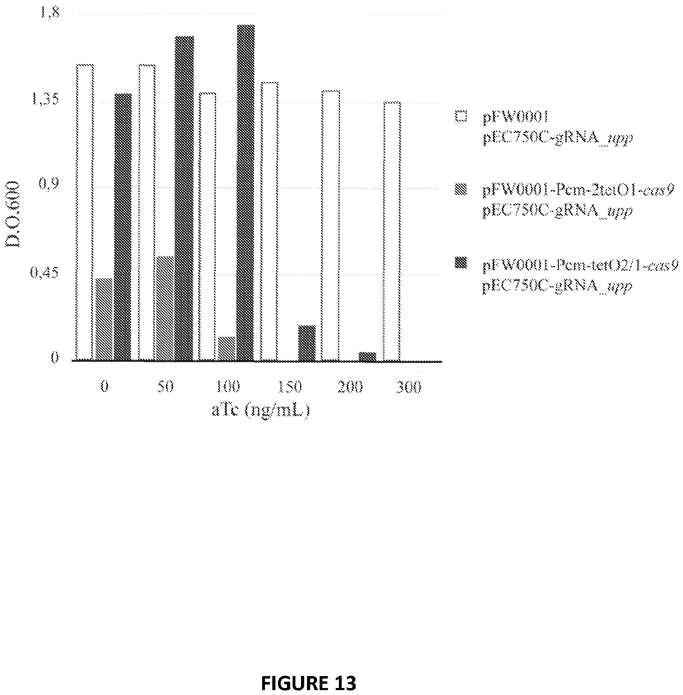

**Figure 13** **:** Effet de la concentration en aTc sur la viabilité de transformants contenant pEC750C-gRNA_*upp.*

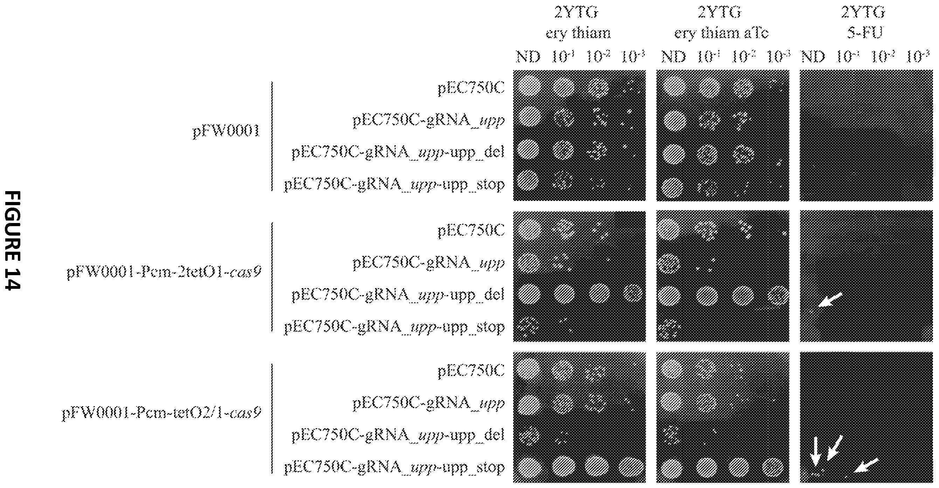

**Figure 14** : Génération de mutants résistants au 5-FU. Des dilutions en série de cultures liquides sont déposées sur différents milieux. Seuls les transformants dans lesquels des événements de recombinaison homologue ont permis l'insertion de la matrice de réparation sont capables de pousser sur 2YTG + 5-FU. Les flèches blanches désignent les colonies sélectionnées pour les expérimentations suivantes. ND, non dilué.

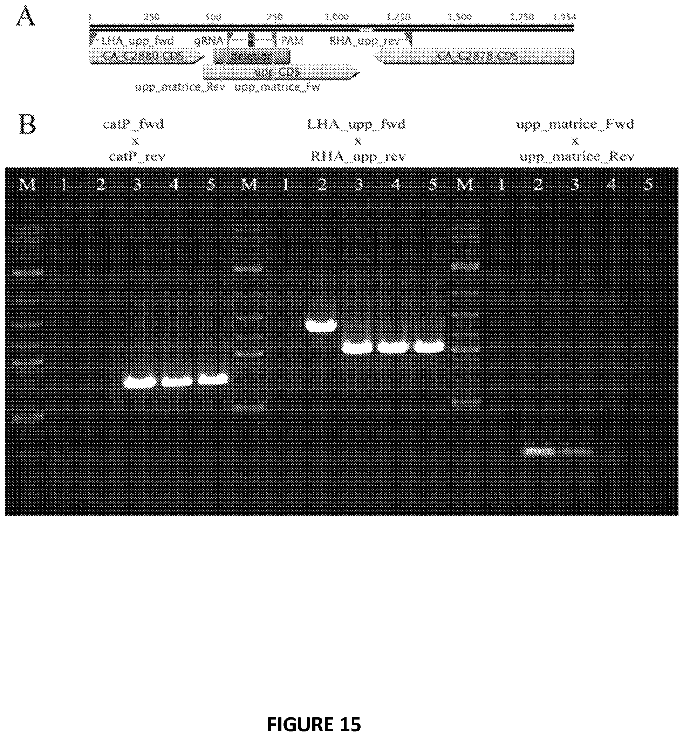

**Figure 15** : Analyse des transformants upp_del par PCR.
   A. Organisation génétique autour du gène *upp.* Les sequences codantes sont signalisées par des flèches. Le rectangle gris indique la région absente de la matrice upp_del. Les primers utilisés sont représentés par des triangles. CDS, séquence codante. PAM, protospacer adjacent motif, intervenant dans la fixation de Cas9.
   B. Résultats de l'amplification. M : Marqueur 2-Log (NEB). 1 : H 2 0, témoin négatif. 2 : ATCC824 non transformé.
   3 : ATCC824 transformé avec pFW0001-Pcm-2tetO1-*cas9* et pEC750CgRNA_*upp*-upp_del avant exposition à l'aTc. 4 & 5 : ATCC824 transformé avec pFW0001-Pcm-2tetO1-*cas9* et pEC750CgRNA_*upp*-upp_del avant exposition à l'aTc, isolé sur 2YTG+5-FU (2 transformants indépendants).

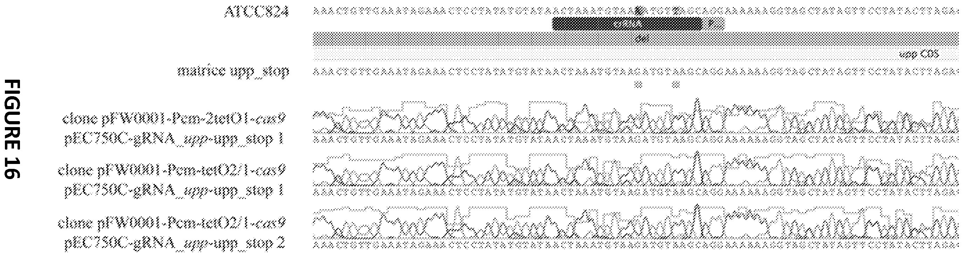

**Figure 16** **:** séquençage de la zone ciblée par cas9 chez les colonies isolées sur 2YTG+5-FU. NC_003030, séquence de Clostridium acetobutylicum ATCC824 (GenBank); crRNA, site reconnu par l'ARNg ; PAM, protospacer adjacent motif, intervenant dans la fixation de Cas9. CDS, coding sequence. SEQ ID NO : 24 correspond au fragment de la séquence génomique de la souche ATCC824 apparaissant sur la Figure 16 and SEQ ID NO : 25 correspond aux fragments apparaissant sur la Figure 16 des séquences identifiées respectivement comme « matrice upp_stop », « clone pFW0001-Pcm-2tetO1-*cas9* - pEC750C-gRNA*_upp-upp_*stop1 » et « clone pFW0001-Pcm-tetO2/1-*cas9* - pEC750C*-*gRNA*_upp-*upp*_*stop1 » et «clone pFW0001-Pcm-tetO2/1-*cas9* - pEC750C*-*gRNA_*upp-*upp_stop2 ».

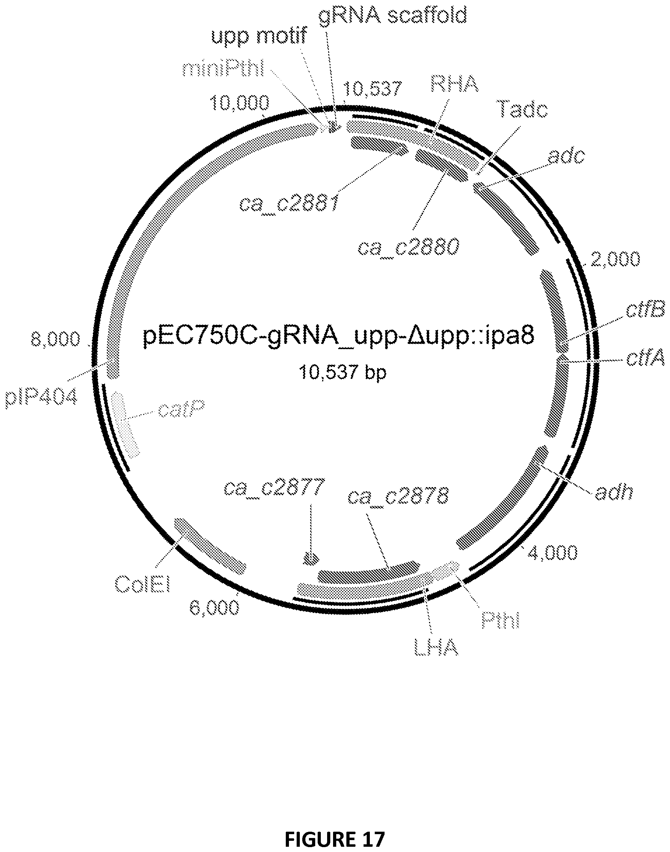

**Figure 17** **:** Plasmide pEC750C-gRNA_upp-Δ*upp*::ipa8. pIP404, origine de réplication chez *C*. *acetobutylicum.* ColE1, origine de réplication chez *E. coli.* acétyltransférase (gène de résistance au chloramphénicol/thiamphénicol). CDS, coding sequence. RHA/LHA : séquences flanquantes du gène *upp (ca_c2879)*

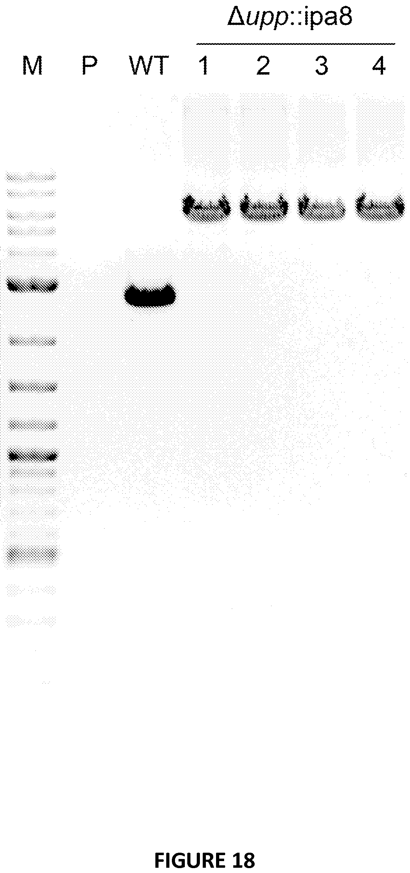

**Figure 18** **:** Résultats d'amplification avec les amorces CA_C2877 et CA_C2882. M, marqueur de taille 2-log (NEB). P, pEC750C-gRNA_upp-Δ*upp*::ipa8 ; WT, ATCC 824.

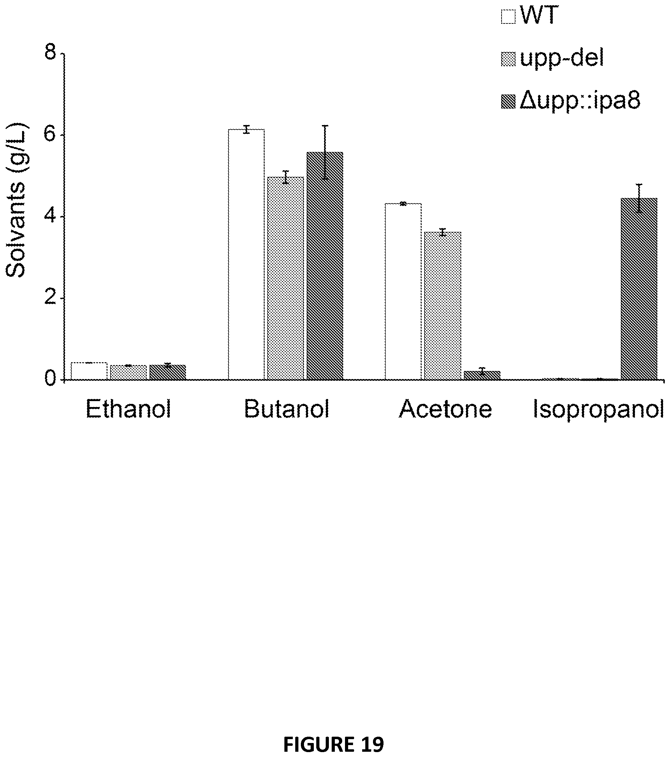

**Figure 19** **:** Production de solvants par ATCC 824 et par les mutants upp_del et Δ*upp*::ipa8*.* Les barres d'erreurs représentent l'écart-type d'expérimentations réalisées en duplicat. Les données obtenues pour upp_del et Δ*upp*::ipa8 sont les moyennes obtenues pour deux mutants biologiquement indépendants dans chaque cas.

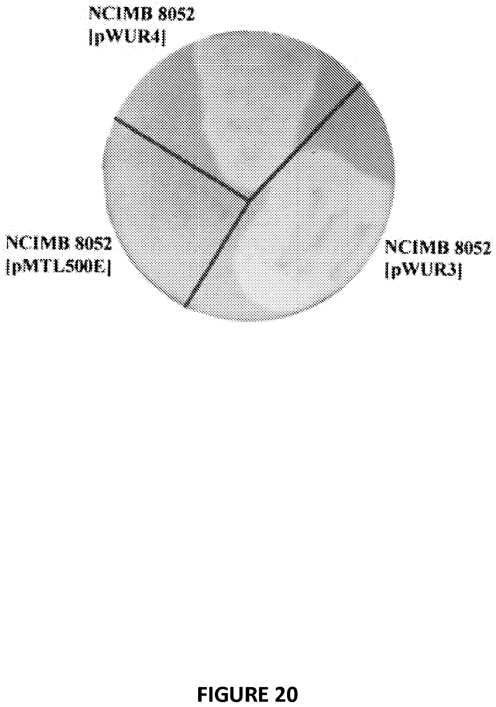

**Figure 20** : Mesure de l'activité endoglucanase sur milieu gélosé de différentes souches exprimant les endoglucanase CelA (pWUR3) CelD (pWUR 4) et de la souche contrôle exprimant le plasmide vide. Ces différentes souches sont mises à incuber pendant 48h sur des boites de Pétri contenant 0,2% CMC. Un halo d'hydrolyse révélé par une coloration au rouge Congo caractérise l'activité endoglucanase de chaque souche. Aucun halo n'est détectable sur la souche contrôle alors qu'il est clairement visible dans les souches exprimant les endoglucanases CelA ou CelD.

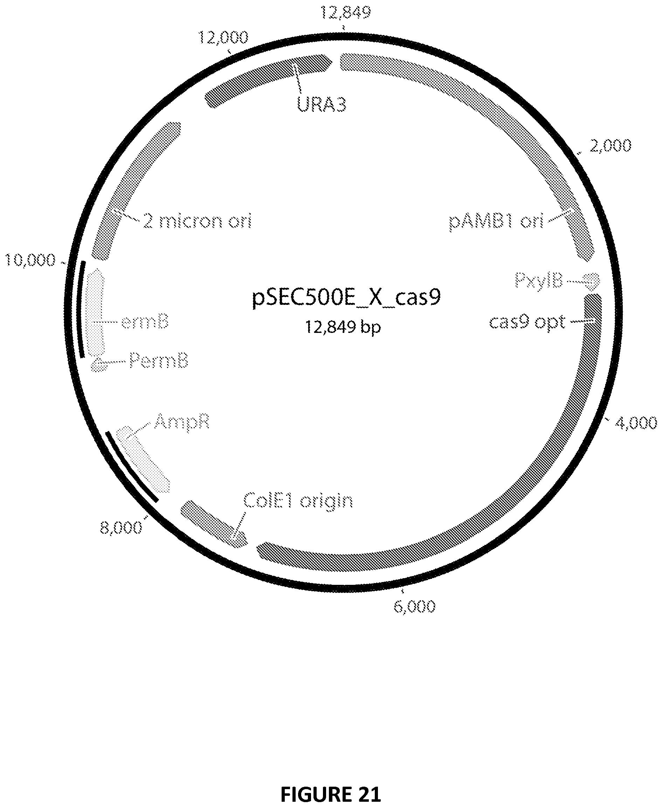

**Figure 21** **:** Plasmide pSEC500E_X_Cas9. pAMB1, origine de réplication chez *C. beijerinckii ;* PxylB, promoteur xylB ; ColEl origin, origine de réplication chez *E. coli* ; AmpR, gène de résistance à l'ampicilline ; PermB, promoteur du gène *ermB* ; ermB, gène de résistance à l'érythromycine ; 2 micron ori, origine de réplication chez la levure ; URA3, marqueur d'auxotrophie.

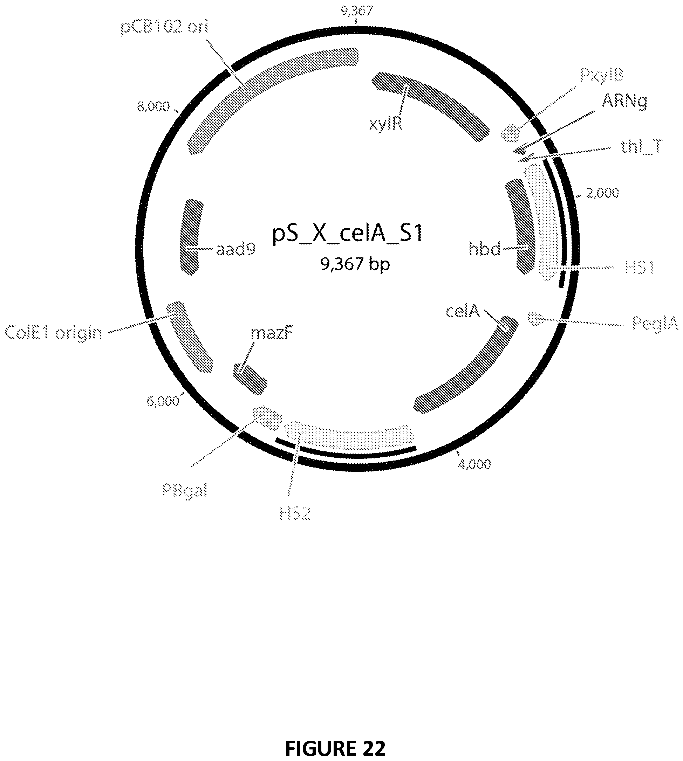

**Figure 22** **:** Plasmide pS_celAS1. HS1 et HS2, séquences d'homologie ; aad9, gène de résistance à la spectinomycine ; pCB102, origine de réplication chez *C*. *beijerinckii ;* ColE1 origin, origine de réplication chez *E. coli.* PeglA, promoteur du gène *eglA.*

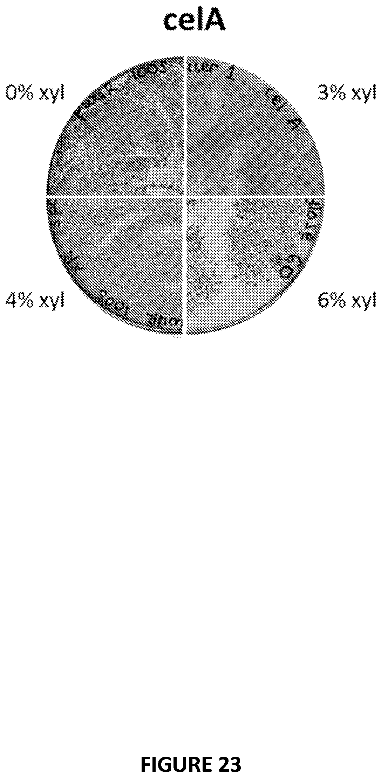

**Figure 23** **:** Sélection des souches recombinantes *C*. *beijerinckii* NCIMB8052 (pEC500E_Xcas9, pS_celAS1) sur milieu gélosé CGM contenant des concentrations croissantes de xylose.

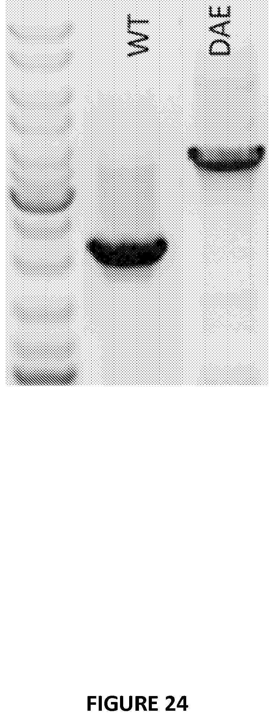

**Figure 24** : Contrôle par PCR de la souche NCIMB8052 et de la souche NCIMB8052 ayant intégrée le gène *celA.* M, GeneRuler 1kb DNA Ladder (ThermoFisher).

### EXEMPLES

Les inventeurs ont testé l'outil génétique décrit et revendiqué dans le présent texte sur deux cibles : les gènes *upp* et *adhE.*

### Inactivation du gène upp

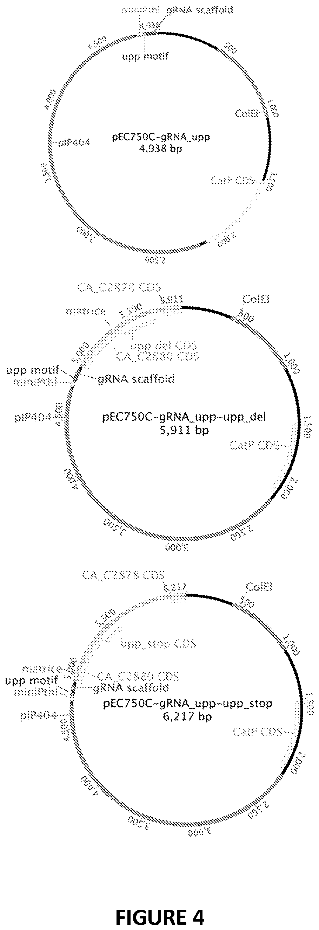

La première cible choisie permet de valider la technique de modification génétique conçue, par un screening simple. Le gène *upp* code une uracile phosphoribosyltransférase. Cette enzyme permet la formation d'uracile monophosphate (UMP) à partir d'uracile, mais également la formation de 5-fluorouracile monophosphate (5-FUMP), à partir de 5-fluorouracile (5-FU). Le 5-FUMP est un composé toxique pour la cellule qui bloque la synthèse d'ARN. De ce fait, une bactérie contenant dans son génome le gène *upp* ne pourra pas croître sur un milieu contenant du 5-FU, au contraire d'une souche n'exprimant pas ce gène.
Cibler ce gène permet de déterminer de façon simple et rapide, par simple observation phénotypique, si la stratégie de modification est efficace. Trois plasmides permettant de cibler *upp* ont été construits (cf. Figure 4 + SEQ ID NO : 9, 10 et 11). Tous trois contiennent le même ARNg ciblant le gène, et deux d'entre eux contiennent également des matrices de réparation différentes permettant de montrer les capacités de l'outil pour réaliser des délétions ou des mutations ponctuelles :
- La matrice upp_del (SEQ ID NO : 12) contient deux fragments de 500 nucléotides (nt) situés à 150nt de part et d'autre du site de coupure déterminé par l'ARNg. L'utilisation de cette matrice pour réparer la coupure provoque une délétion de 300nt à l'intérieur de la séquence codante du gène *upp* de sorte que ce dernier codera alors une protéine inactive.
- La matrice upp_stop (SEQ ID NO : 13)contient deux fragments de 650nt situés de part et d'autre du site de coupure, modifiés au niveau du site de reconnaissance de l'ARNg par la présence de mutations non-sens (induisant le replacement d'un codon codant un acide aminé par un codon stop) de telle manière que Cas9 ne puisse plus cibler le gène qui codera une protéine incomplète et inactive.

### Perte du plasmide pSOL

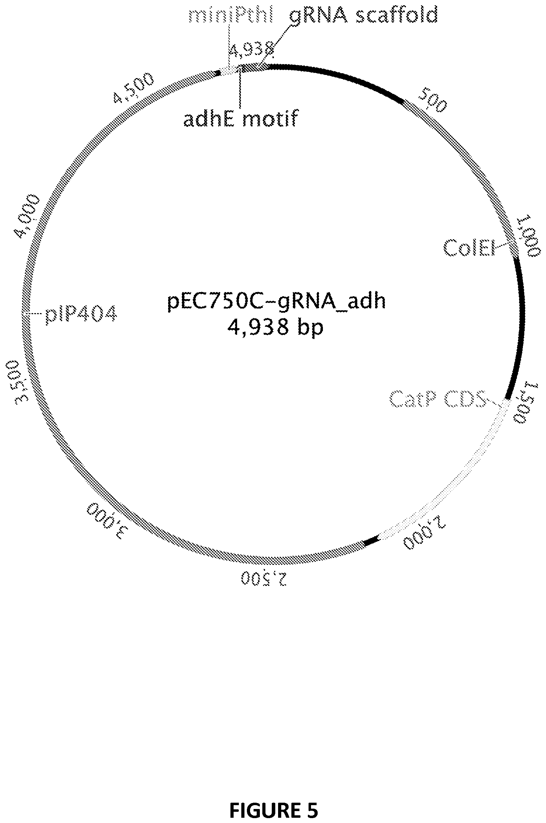

La deuxième cible choisie présente un intérêt dans le processus de fermentation: l'ensemble des gènes impliqués dans la solvantogénèse, en particulier *adhE,* est localisé sur le mégaplasmide pSOL, et il a été montré que sa perte abolit la production d'acétone et de butanol. Après avoir supprimé ces voies fermentaires à l'aide du plasmide de ciblage de pSOL (cf. Figure 5), il est possible de réintroduire les gènes d'intérêt directement dans le génome. Afin d'obtenir une souche ne contenant plus pSOL, un plasmide permettant de cibler *adhE* a été construit. Les inventeurs ont démontré que pSOL ne contient pas de fonctions essentielles pour la cellule de sorte que la cellule pourra survivre sans sa présence.

### Expression constitutive de cas9 dans C. acetobutylicum ATCC824

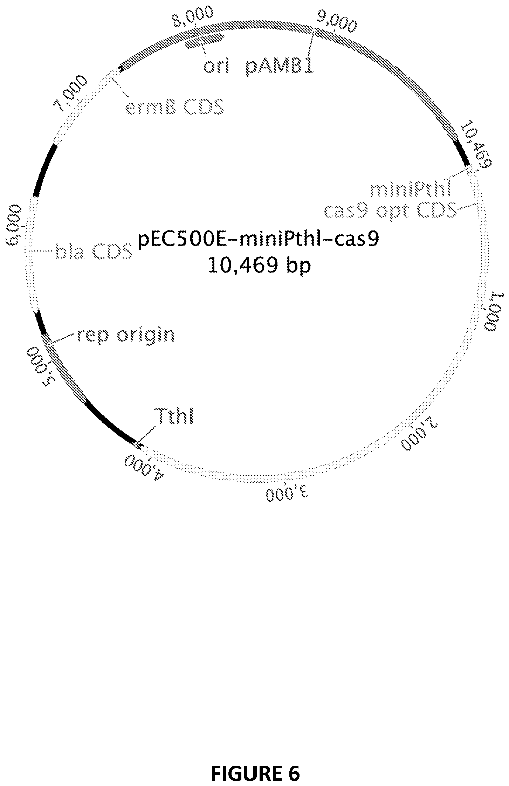

La stratégie choisie nécessite l'utilisation concomitante de deux plasmides :
- le vecteur d'expression de la nucléase de manière constitutive, dérivé du plasmide pEC500E : pEC500E-miniPthl-Cas9 (cf. figure 6 + SEQ ID NO : 5) ;
- l'un des vecteurs de ciblage, qui déterminent le site de coupure de la nucléase et permettent éventuellement la réparation de la coupure, dérivés de pEC750C :
   - pEC750C-gRNA_*upp* (SEQ ID NO : 9), contenant l'ARNg ciblant *upp* ;
   - pEC750C-gRNA_*upp*-upp_del (SEQ ID NO : 10), contenant l'ARNg ciblant *upp* et la matrice upp_del ;
   - pEC750C-gRNA_*upp*-upp_stop (SEQ ID NO : 11), contenant l'ARNg ciblant *upp* et la matrice upp_stop ;
   - pEC750C-gRNA_*adhE* (SEQ ID NO : 8), contenant l'ARNg ciblant *adhE.*

Le vecteur d'expression pEC500E-miniPthl-Cas9 a été introduit dans la souche ATCC824, ainsi qu'un plasmide de contrôle correspondant à un vecteur vide (pEC500E). Les deux souches obtenues ont ensuite été transformées avec les vecteurs de ciblage, dérivés du vecteur pEC750C, servant de contrôle. Les résultats de cette deuxième étape de transformation sont indiqués ci-dessous dans le tableau 1.

**Tableau 1 : résultats de transformations. ++, nombreux transformants obtenus (entre 10² et 10³ colonies obtenues/transformation) ; -, pas de transformants obtenus.**

| | pEC750C | pEC750C-gRNA_*upp* | pEC750 _*upp-*upp_del | pEC750C-gRNA_*upp-*upp_stop | pEC750C-gRNA_*adhE* |
|---|---|---|---|---|---|
| pEC500E | ++ | ++ | ++ | ++ | ++ |
| pEC500E-miniPthl *cas9* | ++ | - | - | + | ++ |

Les résultats de transformation obtenus indiquent que Cas9 est fonctionnel. En effet, lorsque la nucléase est exprimée et que le gène *upp* est ciblé par l'ARNg, aucun transformant n'est obtenu, en raison de la coupure provoquée dans l'ADN génomique et de l'incapacité de la bactérie à réaliser la réparation du génome (transformation avec pEC500E-miniPthl-*cas*9 et pEC750C-gRNA_*upp*) en l'absence de matrice de réparation.

### Ciblage de upp

Les résultats obtenus lorsque les vecteurs de ciblage sont introduits dans la souche contenant pEC500E montrent que le génome de la souche ATCC824 ne contient pas d'homologue de *cas9,* puisque des transformants sont obtenus avec chaque vecteur de ciblage.

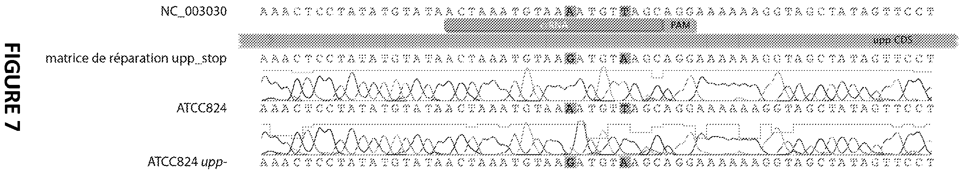

Un transformant contenant pEC500E-miniPthl-*cas*9 et pEC750C-gRNA_*upp*-upp_stop a ensuite été repiqué à plusieurs reprises sur un milieu non sélectif afin qu'il perde les plasmides qu'il contenait. Une fois les colonies curées de leurs plasmides et sensibles aux antibiotiques, le gène *upp* (SEQ ID NO : 3) a été séquencé (cf. figure 7).

Les modifications désirées sont bien présentes. L'outil génétique CRISPR-Cas9 comprenant l'introduction de deux plasmides et l'expression du gène *cas9* par un promoteur fort et constitutif est donc bien fonctionnel.

### Ciblage de adhE

Des transformants sont obtenus lors des transformations de la souche sauvage avec les plasmides pEC500E-miniPthl-*cas*9 et pEC750C_gRNA_*adhE.* Puisque *cas9* est actif, ce résultat indique qu'une coupure dans le mégaplasmide pSOL n'affecte pas la viabilité d'ATCC824.

Afin de confirmer l'éventuelle perte de pSOL, différents tests ont été réalisés :

### - Détection par PCR d'un gène présent sur le pSOL : ctfB

La PCR catP_fwd x catP_rev permet de détecter le gène de résistance au thiamphénicol, présent sur les plasmides pEC750C. Sa détection confirme que les vecteurs de ciblage sont présents.

La PCR RH_ctfB_R x V-CTFA-CAC2707_R permet de détecter une partie du gène *ctfB,* présent sur le mégaplasmide pSOL, et permet de savoir si ce dernier est présent dans la cellule.

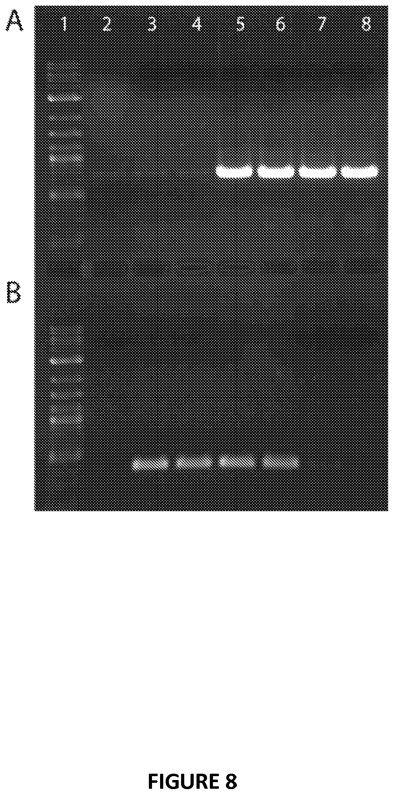

L'amplification semble montrer que le mégaplasmide pSOL n'est plus présent chez les clones transformés avec pEC500E-miniPthl-*cas9* et pEC750C-gRNA_*adhE* (cf. figure 8).

### - Détection d'une activité enzymatique codée par un gène présent sur le pSOL

Parmi les gènes contenus sur le mégaplasmide pSOL, *amyP* code une enzyme extracellulaire à activité α -amylase. Cette activité peut être détectée sur un milieu solide contenant de l'amidon et du glucose (Sabathé *et al.,* 2002). Des dilutions de cultures liquides ont été déposées sur une boîte de gélose contenant 0,2% de glucose et 2% d'amidon et incubées 72h à 37°C. L'activité α-amylase est ensuite révélée par une coloration à l'iode. Les halos clairs autours des spots de bactéries indiquent la présence d'une activité α-amylase. L'absence d'activité autour de ATCC824 contenant pEC500E-miniPthl-*cas9* et pEC750C-gRNA*_adhE* indique que le gène *amyP* n'est pas exprimé dans cette souche, confirmant que le mégaplasmide n'est plus présent (cf. Figure 9).

### - Bilan fermentaire

La souche sauvage ATCC824 ainsi qu'un transformant ont été cultivés pendant 24h dans un milieu GAPES afin d'établir les bilans fermentaires des deux souches. Le bilan fermentaire obtenu montre une diminution de la production d'éthanol ainsi qu'une abolition de la production de butanol et d'acétate en raison de l'absence des gènes *adhE, adhE1* et *adc* (présents sur le mégaplasmide pSOL) chez le transformant (cf. Figure 10).

Cas9 est donc capable d'agir sur le chromosome ou sur le plasmide naturel de la souche ATCC824 ce qui permet d'élargir son action au chromosome et à tout matériel génétique extra-chromosomique présent dans la souche (plasmide bactériophage, etc.).

### Expression inductible de cas9 dans C. acetobutylicum ATCC824

Afin de permettre les événements de recombinaison homologue entre le génome et les matrices de réparation, il faut augmenter le nombre de cellules dans lesquelles la nucléase est active (jusqu'à 10³ lorsque la souche ATCC824 contenant pEC500E-miniPthl-*cas9* est transformée avec un vecteur de ciblage). Dans ce but, un système dans lequel l'expression de la nucléase est contrôlée est nécessaire. Deux vecteurs dans lesquels le gène *cas9* est placé sous le contrôle d'un promoteur inductible à l'anhydrotétracycline ont été construits, dérivés du vecteur pFW0001 :
- pFW0001-Pcm-2tetO1-*cas9* (cf. Figure 11A + SEQ ID NO:6);
- pFW0001-Pcm-tetO2/1-*cas9* (cf. Figure 11B + SEQ ID NO:7);
Les promoteurs contrôlant l'expression de *cas9* contiennent des séquences opératrices, tetO1 et tetO2, sur lesquelles se fixe le répresseur transcriptionnel TetR. Cette répression est levée par la présence d'anhydrotétracycline (aTc). Ce système permet une expression contrôlée, avec peu de fuites. En présence d'aTc, la synthèse est plus importante à partir du promoteur Pcm-2tetO1 (cf. Figure 12).

### Transformation de C. acetobutylicum ATCC824 :

Les vecteurs d'expression ainsi que le vecteur vide pFW0001 ont été introduits dans ATCC824. Par la suite, les plasmides suivants ont été introduits dans chaque type de transformant (cf. tableau 2) :
- pEC750C-gRNA_*upp,* contenant l'ARNg ciblant *upp ;*
- pEC750C-gRNA*_upp-*upp*_*del*,* contenant l'ARNg ciblant *upp* et la matrice upp_del ;
- pEC750C-gRNA_*upp-*upp*_*stop*,* contenant l'ARNg ciblant *upp* et la matrice upp_stop ;
Les colonies transformées ont été étalées sur différents milieux solides, à des dilutions différentes :
- 2YTG + érythromycine pour vérifier la viabilité cellulaire, dilution d'un facteur 10⁶ ;
- 2YTG + érythromycine + thiamphénicol pour sélectionner les transformants ;
- 2YTG + érythromycine + thiamphénicol + aTc (200ng/mL) pour sélectionner les transformants en présence de l'inducteur.

**Tableau 2 : nombre de colonies obtenues sur chaque type de milieu pour chaque transformation. ery, érythromycine. thiam, thiamphénicol. aTc, anhydrotétracycline. Entre parenthèses, facteurs de dilutions. ND, non dilué. -, non testé.**

| | pEC750C | | | pEC750C-gRNA_*upp* | | | pEC750C-gRNA-*upp-*upp_del | | | pEC750C-gRNA_*upp-*upp_stop | | |
|---|---|---|---|---|---|---|---|---|---|---|---|---|
| | ery (10⁻⁸) | ery thiam (ND) | ery thiam aTc (ND) | ery (10⁻⁴) | ery thiam (ND) | ery thiam aTc (ND) | ery (10⁻⁶) | ery thiam (ND) | ery thiam aTc (ND) | ery (10⁻⁶) | ery thiam {ND} | ery thiam aTc (ND) |
| pFW0001 | 78 | 26 | - | 126 | 83 | - | 87 | 12 | - | 61 | 13 | - |
| pFW0001-Pcm-2tetO1-*cas9* | 229 | 129 | 3 | 157 | 27 | 0 | 232 | 5 | 0 | 169 | 7 | 0 |
| pF-W0001-Pcm-tetO2/1 - *cas9* | 210 | 400 | 3 | 367 | 130 | 0 | 227 | 21 | 0 | 177 | 18 | 0 |

On observe un effet toxique de l'aTc, puisque peu de transformants sont obtenus lorsqu'il est présent, même lorsque le vecteur de ciblage vide (pEC750C, contrôle) est utilisé. Conformément à ce qui est attendu, aucun transformant n'est obtenu lorsqu'un pEC750C contenant la cassette gRNA_*upp* est introduite dans une cellule exprimant *cas9,* sur un milieu contenant de l'aTc. En revanche, de nombreux transformants sont obtenus pour chaque combinaison de plasmides sur milieu sans aTc, indiquant que *cas9* n'est pas exprimée.

### Expression de cas9 en présence d'aTc :

Les différents transformants obtenus sur boîtes contenant de l'érythromycine et du thiamphénicol ont été repiqués sur le même type de milieu, puis utilisés pour ensemencer des précultures liquides contenant les deux antibiotiques. Ces précultures ont ensuite été utilisées pour ensemencer d'autres cultures liquides contenant des concentrations variables d'aTc afin de déterminer si le système est fonctionnel.

### Induction de cas9 :

Trois transformants ont été utilisés pour analyser la capacité d'induction de l'expression de cas9 en présence d'aTc :
ATCC824 contenant pFW0001 et pEC750C-gRNA_*upp* ;
ATCC824 contenant pFW0001-Pcm-2tetO1-*cas9* et pEC750C-gRNA_*upp* ;
ATCC824 contenant pFW0001-Pcm-tetO2/1-*cas9* et pEC750C-gRNA_*upp* ;
Des milieux liquides contenant de l'érythromycine, du thiamphénicol et des concentrations croissantes d'aTc ont été ensemencés à partir de précultures liquides de ces transformants (cf. figure 13). La capacité de croissance des cellules est évaluée par mesure des densités optiques après 72h de culture. Le transformant n'exprimant pas la nucléase n'est pas ou peu affecté par la présence d'aTc. En revanche, même à des faibles concentrations d'aTc, le transformant contenant le plasmide exprimant *cas9* via le promoteur Pcm-2tetO1 (pFW0001-Pcm-2tetO1-cas9) et le plasmide contenant uniquement le gRNA (pEC750C-gRNA_upp) présentent un important retard de croissance. Le transformant contenant le plasmide exprimant *cas9* via le promoteur Pcm-tetO2/1 (pFW0001-Pcm-tetO2/1-*cas9*) et le plasmide contenant uniquement le gRNA (pEC750C-gRNA_*upp*) n'est pas affecté à de faibles concentrations d'aTc. Cependant, à
partir de 150 ng/mL un fort retard de croissance est observée, et aucune croissance n'est observée à 300ng/mL. Le promoteur Pcm-tetO2/1 semble donc permettre une meilleure répression de l'expression que Pcm-2tetO1 en absence d'inducteur.

### Génération de mutants

Des cultures liquides des transformants contenant les plasmides de ciblage permettant la réparation des cassures double brin ont également été réalisées, en absence ou en présence (100ng/mL) d'aTc. Les transformants utilisés contenaient l'une des douze combinaisons de plasmides apparaissant dans le tableau 3.

**Tableau 3 : Combinaisons de plasmides présents dans les transformants.**

| Plasmide d'expression | | Plasmides de ciblage |
|---|---|---|
| pFW0001 | | pEC750C |
| pFW0001-Pcm-2tetO1-*cas9* | x | pEC750C-gRNA_*upp* |
| pFW0001-Pcm-tetO2/1-*cas9* | | pEC750C-gRNA_*upp-*upp_del |
| | | pEC750C-gRNA_*upp*-upp_stop |

Après 72h de culture, des aliquots ont été déposés sur différents milieux solides:
- 2YTG contenant du thiamphénicol et de l'érythromycine ;
- 2YTG contenant du thiamphénicol, de l'érythromycine, et 100ng/mL d'aTc ;
- 2YTG contenant du 5-fluorouracile.
Seuls les transformants dans lesquels des événements de recombinaison homologue ont permis l'insertion de la matrice de réparation sont capables de pousser sur 2YTG + 5-FU (cf. figure 14).

### Analyse des transformants upp_del :

Les clones isolés sur 2YTG + 5-FU ont été analysés par PCR (cf. figure 15).
La PCR catP_fwd x catP_rev permet de détecter le gène de résistance au thiamphénicol, présent sur les plasmides pEC750C. Sa détection confirme que les vecteurs de ciblage sont présents.
La PCR LHA_upp_fwd x RHA_upp_rev permet d'amplifier le gène *upp* ainsi que les régions flanquantes. Les amorces apparaissant ci-dessous ont servi à la construction de la matrice de réparation upp_del (cf. figure 15 + SEQ ID N0 : 14-21) :

**Tableau n°4 :**

| Nom | Séquence (5'-3') |
|---|---|
| catP_fwd | GTGGGCAAGTTGAAAAATTCAC |
| catP_rev | TTAACTATTTATCAATTCCTGCAATTCG |
| RH_ctfB_R | CTTGAGACTTTGCCGTGAGGG |
| V_ctfA_CAC2707_R | TAGTTGGAATGGGCGCTAGT |
| LHA_upp_fwd | ATGAAGATAGCAATAGGTAGTGATCATGC |
| RHA_upp_rev | ACGCTTATATTATCAATATTATTTAGCTTTATAG |
| upp_matrice_fwd | TGTCCAACCTTAGCAGCAGG |
| upp_matrice_rev | GTAGAAGAAGTAGCAATGCTAATGGC |

La PCR upp_matrice_fwd x upp_matrice_rev permet d'amplifier un fragment interne du gène *upp,* absent de la matrice de réparation upp_del.
Les résultats obtenus confirment la délétion à l'intérieur du gène *upp* dans les transformants analysés.

### Analyse des mutants upp_stop :

Le gène *upp* a été séquencé chez trois clones isolés sur 2YTG+5-FU après exposition à l'aTc (cf. Figure 16) :
- Un contenant le plasmide exprimant *cas9* via le promoteur Pcm-2tetO1 (pFW0001-Pcm-2tetO1-*cas9*) et le plasmide contenant le gRNA ainsi que la matrice de réparation upp_stop (pEC750CgRNA*_upp-*upp*_*stop) ;
- Deux contenant le plasmide exprimant *cas9* via le promoteur Pcm-tetO2/1 (pFW0001-Pcm-tetO2/1-*cas9*) et le plasmide contenant le gRNA ainsi que la matrice de réparation upp_stop (pEC750CgRNA*_upp-*upp*_*stop)*.*

La stratégie visant à développer un système de modification génétique par l'utilisation du gène *cas9* sous contrôle d'un promoteur inductible et du gRNA présent dans un second plasmide est donc fonctionnelle. Par rapport à l'utilisation du promoteur constitutif miniPthl, l'induction du gène *cas9* permet de maîtriser l'action de l'enzyme et de faciliter la sélection de transformants ayant subis les modifications souhaitées.

### Remplacement du gène upp par l'opéron ipa8

La modification apportée consiste en l'insertion au sein du génome de *C*. *acetobutylicum* ATCC 824 de l'opéron ipa8 contenant le gène *adh* de *C*. *beijerinckii* DSM6423 (permettant la conversion d'acétone en isopropanol) et les gènes *adc, ctfA, ctfB* de la souche ATCC 824 (permettant la ré-assimilation des acides produits et la formation d'acétone) sous contrôle du promoteur constitutif du gène *thl.* Cet opéron de 3614 pb est inséré à la place du gène *upp.*

Une matrice de réparation constituée de l'opéron flanqué des séquences de 1 kb situées de part et d'autre du gène *upp* a été insérée dans le plasmide pEC750-gRNA_upp pour obtenir le plasmide pEC750C-gRNA_upp-Δ*upp*::ipa8 (cf. Figure 17 et SEQ ID NO : 26).

Ce plasmide a été introduit dans des cellules compétentes d'ATCC 824 contenant le plasmide pFW0001-Pcm-tetO2/1-*cas9*. L'induction de l'expression de *cas9* a été effectuée sur milieu 2YTG contenant du thiamphénicol, de l'érythromycine, et l'inducteur aTc.

Les colonies obtenues ont été analysées par PCR avec le couple d'amorces CA_C2877 et CA_C2882 qui permet l'amplification d'un produit de 2720 pb chez la souche ATCC 824 :
CA_C2877 : 5'-CTTTTTAAAAAAGTTAAATAAGGAAGG-3' (SEQ ID NO : 27);
CA_C2882 : 5'-GTTTAACTTAAGTTACAGAAAAGCTAGG-3' (SEQ ID NO : 28)
Les résultats de PCR conduites sur les différents contrôles et sur 4 colonies indépendantes obtenues après induction confirment le remplacement du gène *upp* par l'opéron ipa8 (Figure 18).

Des fermentations ont été conduites dans du milieu GAPES (Gapes *et al,* 1996) pendant 72h à 34°C et 150 rpm sur les mutants obtenus, ainsi que sur la souche WT et des mutants Δ*upp* utilisés comme contrôles. Les surnageants de fermentations ont été analysés par HPLC en utilisant une solution de propanol à 0,5 g/L comme standard interne. Les concentrations en carbohydrates ont été quantifiées sur colonne Aminex®HXP-87P (Biorad, 300 mm X 7.8 mm) équipée d'un réfracteur différentiel (Varian 350 RI). La température de colonne était de 80°C et l'éluent consistait en de l'acide sulfurique à un débit de 0,4 mL/min (Spectra System RI 150).
Les résultats obtenus montrent que les mutants Δ*upp*::ipa8 sont capables de réduire l'acétone en isopropanol, au contraire de la souche WT ou d'un mutant Δ*upp* (Figure 19).

### Insertion du gène celA dans le génome de Clostridium beijerinckii NCIMB8052

La modification apportée consiste en l'insertion au sein du génome de *C*. *beijerinckii* NCIMB8052 du gène *celA* de *Neocallismatix patriciarum* sous contrôle du promoteur *eglA* de *Clostridium saccharobutylicum* NCP262. Ce gène code une enzyme capable de dégrader un substrat cellulosique nommé CMC (carboxymethyl cellulose). Le gène et son promoteur, d'une taille de 1667 pb, sont insérés après le gène *hbd,* et permettent à la souche de dégrader le composé CMC (Figure 20, Lopez-contreras *et al,* 2001).
Pour réaliser cette insertion, deux plasmides ont été utilisés :
- le plasmide pEC500E_X_cas9 : exprimant le gène *cas9* sous contrôle du promoteur inductible *xylB* de *Clostridium difficile* 630 (Nariya *et al,* 2011) (cf. Figure 21 et SEQ ID NO : 29).
- le plasmide pS_XR_celAS1 exprimant un ARN guide sous le contrôle du promoteur *xylB* et ciblant le gène *hbd* de *C*. *beijerinckii* NCIMB8052. Le plasmide contient également une matrice de réparation constituée du gène *celA* sous contrôle du promoteur *egl2,* flanqué de deux régions d'homologie de 1001 et 1017 paires de base situées de part et d'autre de la région ciblée par l'ARN guide. (cf. Figure 22 et SEQ ID NO : 30).

Ces deux plasmides ont été introduits séquentiellement dans NCIMB8052. L'induction de l'expression de *cas9* a été effectuée sur milieu CGM (Extrait de levure 6,25 g/L ; MgSO₄, 7H₂O 0,5 g/L ; KH₂HPO₄ 0,95 g/L ; K₂HPO₄ 0,95 g/L ; MnSO₄, 7H₂O 0,013 g/L ; FeSO₄, 7H₂O 0,013 g/L ; NaCl 1,25 g/L ; (NH₄)₂SO₄ 2,5 g/L ; Asparagine 2,5 g/L) contenant de la spectinomycine et de l'erythromycine ainsi que des concentrations croissantes d'inducteur xylose (Figure 24).
Les colonies obtenues après induction sur milieu CGM contenant 6% xylose ont été analysées par PCR avec le couple d'amorces Cbei_325_F et Cbei_325_R qui permet l'amplification d'un produit de 2070 paires de base chez la souche NCIMB8052 et de 3718 paires de base en cas d'intégration du gène *cel*A :

| | |
|---|---|
| Cbei_325_F (celA) | 5' - AGATAATTATGAAGTTAATCCTTAG - 3' (SEQ ID NO : 31) |
| Cbei_326_R (celA) | 5' - CATTTGCTTTCAGGTCTTCTTTTGCTG -3'(SEQ ID NO : 32) |

Les résultats de PCR conduites sur la souche contrôle et sur une colonie indépendante obtenue après induction confirment l'insertion du gène *celA* dans NCIMB8052 (Figure 25).

### REFERENCES

- Al-Hinai, M. A. et al. Novel system for efficient isolation of Clostridium double-crossover allelic exchange mutants enabling markerless chromosomal gene deletions and DNA intégration. Appl. Environ Microbiol. 2012. 78(22): 8112-21.
- Ran, F. A. et al. Genome engineering using the CRISPR-Cas9 system. Nature protocols. 2013. 8(11): 2281-308.
- Barrangou, R. et al. CRISPR provides acquired resistance against viruses in prokaryotes. Science. 2007, 315(5819): 1709-12.
- Banerjee, A. et al. Lactose-inducible system for metabolic engineering of Clostridium ljungdahlii, Appl. Environ. Microbiol. 2014.80(8): 2410-6.
- Cartman, S. T. et al. Precise manipulation of the Clostridium difficile chromosome reveals a lack of association between the tcdC genotype and toxin production. Appl. Environ. Microbiol. 2012. 78(13): 4683-90.
- Currie, D.H. et al. Functional heterologous expression of an engineered full length CipA from Clostridium thermocellum in Thermoanaerobacterium saccharolyticum, Biotechnol. Biofuels. 2013. 6(1): 32.
- DiCarlo, J. E. et al. Genome engineering in Saccharomyces cerevisiae using CRISPR-Cas systems. Nucleic Acids Res. 2013, 41(7): 4336-43
- Dong, H. et al. Development of an anhydrotetracycline-inducible gene expression system for solvent-producing Clostridium acetobutylicum: A useful tool for strain engineering. Metab. Eng. 2012. 14(1): 59-67.
- D'Urzo, N. et al. High-level intracellular expression of heterologous proteins in Brevibacillus choshinensis SP3 under the control of a xylose inducible promoter. Microb. Cell Fact. 2013. 12: 12
- Egholm, M. et al. Peptide nucleic acids (PNA). Oligonucleotide analogs with an achiral peptide backbone. J. Am. Chem. Soc. 1992. 114(5): 1895-7.
- Fonfara, I. et al. Phylogeny of Cas9 détermines functional exchangeability of dual-RNA and Cas9 among orthologous type II CRISPR-Cas systems. Nucleic acids res. 2013, 42(4): 2577-90.
- Gapes, J. R., Nimcevic, D., & Friedl, A. (1996). Long-Term Continuous Cultivation of Clostridium beijerinckii in a Two-Stage Chemostat with On-Line Solvent Removal. Applied and environmental microbiology, 62(9), 3210-3219.
- Hartman, A.H. et al. Construction and characterization of a lactose-inducible promoter system for controlled gene expression in Clostridium perfringens. Appl. Environ. Microbiol. 77(2): 471-8
- Jinek, M. et al. A programmable dual-RNA-guided DNA endonuclease in adaptive bacterial immunity. Science 2012. 337(6096): 816-21.
- Lee, J. et al. Metabolic engineering of Clostridium acetobutylicum ATCC 824 for isopropanol-butanol-ethanol fermentation. Appl. Environ. Microbiol; 2012. 78(5): 1416-23.
- Lopez-Contreras AM, Smidt H, van der Oost J, Claassen PAM, Mooibroek H, de Vos WM. 2001. Clostridium beijerinckii Cells Expressing Neocallimastix patriciarum Glycoside Hydrolases Show Enhanced Lichenan Utilization and Solvent Production. Appl Environ Microbiol 67:5127-5133.
- Lütke-Eversloh, T. Application of new metabolic engineering tools for Clostridium acetobutylicum. Appl. Microbiol. Biotechnol. 2014. 98(13): 5823-37.
- Mali, P. et al. Cas9 as a versatile tool for engineering biology. Nat. methods. 2013, 10(10): 957-63.
- Makarova, K. S., et al. Evolution and classification of the CRISPR-Cas systems. Nat. Rev. Microbiol. 2011, 9(6): 467-77.
- Mearls, E.B. et al. Development of a regulatable plasmid-based gene expression system for Clostridium thermocellum, Appl. Microbiol. Biotechnol. 2015.99(18): 7589-99.
- Nariya, H. et al., Development and characterization of a xylose-inducible gene expression system for Clostridium perfringens, Appl. Environ. Microbiol. 2011.77(23): 8439-41.
- Nariya H, Miyata S, Kuwahara T, Okabe A. 2011. Development and characterization of a xylose-inducible gene expression system for Clostridium perfringens. Appl Environ Microbiol 77:8439-8441.
- Newcomb, M. et al. Co-transcription of the celC gene cluster in Clostridium thermocellum, Appl. Microbiol. Biotechnol. 2011. 90(2): 625-34.
- Wang, Y. et al. Markerless chromosomal gene deletion in Clostridium beijerinckii using CRISPR/Cas9 system. J. Biotechnol.2015. 200: 1-5.
- Xu, T. et al. Efficient genome editing in Clostridium cellulolyticum via CRISPR-Cas9 nickase. Appl. Environ. Microbiol. 2015 AEM-00873.
- Zhang, N. et al. I-SceI-mediated scarless gene modification via allelic exchange in Clostridium. J. Microbiol. Methods. 2015, 108: 49-60.
- Zhang, J. et al. A novel arabinose-inducible genetic operation system developed for Clostridium cellulolyticum, Biotechnol. Biofuels. 2015.8: 36.

## Revendications

1. Outil génétique permettant la transformation par recombinaison homologue d'une bactérie solvantogène du genre *Clostridium* **caractérisé en ce qu'**il comprend :
- un premier acide nucléique codant au moins Cas9, dans lequel la séquence codant Cas9 est placée sous le contrôle d'un promoteur, et
- au moins un deuxième acide nucléique contenant une matrice de réparation permettant, par un mécanisme de recombinaison homologue, le remplacement d'une portion de l'ADN bactérien ciblée par Cas9 par une séquence d'intérêt,
**en ce que** i) au moins l'un desdits acides nucléiques code en outre un ou plusieurs ARN guides (ARNg) ou ii) l'outil génétique comprend en outre un ou plusieurs ARN guides, chaque ARN guide comprenant une structure ARN de fixation à l'enzyme Cas9 et une séquence complémentaire de la portion ciblée de l'ADN bactérien, ladite séquence complémentaire comprenant au moins 10 nucléotides, **en ce que** Cas9 provoque la coupure double-brins de l'ADN bactérien, **en ce que** l'ADN bactérien est l'ADN d'une bactérie solvantogène du genre *Clostridium,* et **en ce que** chacun des premier et deuxième acides nucléiques présents au sein de l'outil appartient à un vecteur distinct.

2. Outil selon la revendication 1, **caractérisé en ce que** la séquence codant le ou lesdits ARN guides est précédée d'un promoteur et **en ce que** ledit promoteur ou le promoteur contrôlant Cas9 est un promoteur inductible.

3. Outil selon la revendication 1 ou 2, **caractérisé en ce que** la portion ciblée de l'ADN bactérien comprend un gène essentiel ou une portion de gène essentielle à la survie bactérienne.

4. Outil selon l'une des revendications 1 à 3, **caractérisé en ce que** la bactérie solvantogène du genre *Clostridium* est sélectionnée parmi *C. acetobutylicum, C. cellulolyticum, C. phytofermentans, C. beijerinckii, C. saccharobutylicum, C. saccharoperbutylacetonicum, C. sporogenes, C. butyricum, C. aurantibutyricum et C. tyrobutyricum.*

5. Outil selon la revendication 4, **caractérisé en ce que** lorsque la bactérie solvantogène est *C*. *acetobutylicum* ladite bactérie *C*. *acetobutylicum* est la souche ATCC824, et lorsque la bactérie solvantogène est *C. beijerinckii* ladite bactérie *C. beijerinckii* est la souche DSM 6423.

6. Outil selon l'une des revendications 1 à 5, **caractérisé en ce que** la protéine Cas9 comprend la séquence SEQ ID NO : 1.

7. Outil selon l'une des revendications 1 à 6, **caractérisé en ce que** le promoteur de Cas9 est un promoteur inductible.

8. Outil selon l'une des revendications 1 à 7, **caractérisé en ce que** la séquence d'ADN d'intérêt code au moins un produit favorisant la production de solvant, typiquement au moins une enzyme impliquée dans la conversion des aldéhydes en alcool, une protéine membranaire, un facteur de transcription, ou une combinaison de ceux-ci.

9. Outil selon l'une des revendications 1 à 8, **caractérisé en ce que** le vecteur est un plasmide.

10. Procédé pour transformer par recombinaison homologue une bactérie solvantogène du genre *Clostridium,* **caractérisé en ce qu'**il comprend une étape d'introduction dans la bactérie d'un outil génétique selon l'une des revendications 1 à 9.

11. Procédé selon la revendication 10, **caractérisé en ce qu'**il comprend les étapes suivantes :
a) l'introduction dans la bactérie d'un outil génétique selon l'une des revendications 1 à 8 comprenant au moins un promoteur inductible, et
b) l'induction de l'expression du promoteur inductible permettant de modifier génétiquement la bactérie.

12. Procédé selon la revendication 10 ou 11, **caractérisé en ce qu'**il comprend une ou plusieurs étapes additionnelles, postérieures à l'étape b) lorsqu'elle est présente, d'introduction d'un énième acide nucléique codant i) une matrice de réparation distincte de celle(s) déjà introduite(s) et ii) un ou plusieurs ARN guides permettant leur intégration dans une zone ciblée du génome de la bactérie, chaque étape additionnelle étant précédée d'une étape d'élimination de l'acide nucléique codant la matrice de réparation préalablement introduite, et de préférence d'élimination du/des ARNs guides ou séquences codant le/les ARNs guides préalablement introduits.

13. Bactérie solvantogène du genre *Clostridium* transformée à l'aide du procédé selon l'une des revendications 10 à 12.

14. Kit pour transformer une bactérie du genre *Clostridium* ou pour produire au moins un solvant à l'aide d'une bactérie du genre *Clostridium* comprenant les éléments de l'outil génétique tels que décrits dans l'une des revendications 1 à 9 et éventuellement un ou plusieurs inducteurs adaptés au(x) promoteur(s) inductible(s) sélectionné(s) utilisé(s) au sein de l'outil.

15. Utilisation de l'outil génétique selon l'une des revendications 1 à 9, du procédé selon l'une des revendications 10 à 12, ou d'une bactérie transformée selon la revendication 13, pour permettre la production d'un solvant ou d'un mélange de solvants à l'échelle industrielle, de préférence d'acétone, de butanol, d'éthanol, d'isopropanol ou d'un mélange de ceux-ci, typiquement d'un mélange isopropanol/butanol.

## Patentansprüche

1. Genetisches Werkzeug, das die Transformation eines lösemittelproduzierenden Bakteriums der Gattung *Clostridium* mittels homologer Rekombination erlaubt, **dadurch gekennzeichnet, dass** es umfasst:
- eine erste Nukleinsäure, die wenigstens Cas9 codiert, wobei die Cas9-codierende Sequenz unter der Kontrolle eines Promotors steht, und
- wenigstens eine zweite Nukleinsäure, die ein Repair-Template enthält, das mithilfe eines homologen Rekombinationsmechanismus das Ersetzen eines Abschnitts der Cas9-gerichteten bakteriellen DNA durch eine Sequenz von Interesse erlaubt,
dass i) wenigstens eine der besagten Nukleinsäuren außerdem eine oder mehrere guide-RNAs (gRNAs) codiert oder ii) das genetische Werkzeug außerdem ein oder mehrere guide-RNAs umfasst, wobei jede guide-RNA eine Cas9-Enzym-bindende RNA-Struktur und eine komplementäre Sequenz des gerichteten Abschnitts der bakteriellen DNA umfasst, wobei besagte komplementäre Sequenz wenigstens 10 Nukleotide umfasst,
dass Cas9 das Schneiden der doppelsträngigen bakteriellen DNA bewirkt, dass die bakterielle DNA eine DNA eines lösemittelproduzierenden Bakteriums der Gattung *Clostridium* ist und dass jede der ersten und zweiten Nukleinsäuren, die im Werkzeug vorhanden sind, zu einem unterschiedlichen Vektor gehört.

2. Werkzeug gemäß Anspruch 1, **dadurch gekennzeichnet, dass** der Sequenz, die eine oder mehrere besagte guide-RNAs codiert, ein Promotor vorausgeht und dass besagter Promotor oder der Cas9-kontrollierende Promotor ein induzierbarer Promotor ist.

3. Werkzeug gemäß Anspruch 1 oder 2, **dadurch gekennzeichnet, dass** der gerichtete Abschnitt der bakteriellen DNA ein für das Überleben des Bakteriums essentielles Gen oder einen Abschnitt eines essentiellen Gens umfasst.

4. Werkzeug gemäß einem der Ansprüche 1 bis 3, **dadurch gekennzeichnet, dass** das lösemittelproduzierende Bakterium der Gattung *Clostridium* aus *C. acetobutylicum, C. cellulolyticum, C. phytofermentans, C. beijerinckii, C. saccharobutylicum, C. saccharoperbutylacetonicum, C. sporongenes, C. butyricum, C. aurantibutyricum* und *C*. *tyrobutyricum* ausgewählt ist.

5. Werkzeug gemäß Anspruch 4, **dadurch gekennzeichnet, dass**, wenn das lösemittelproduzierende Bakterium *C*. *acetobutylicum* ist, besagtes Bakterium *C*. *acetobutylicum* Stamm ATCC824 ist, und wenn das lösemittelproduzierende Bakterium *C*. *beijerinckii* ist, besagtes Bakterium C*. beijerinckii* Stamm DSM 6423 ist.

6. Werkzeug gemäß einem der Ansprüche 1 bis 5, **dadurch gekennzeichnet, dass** das Cas9-Protein die Sequenz SEQ ID NO: 1 umfasst.

7. Werkzeug gemäß einem der Ansprüche 1 bis 6, **dadurch gekennzeichnet, dass** der Cas9-Promotor ein induzierbarer Promotor ist.

8. Werkzeug gemäß einem der Ansprüche 1 bis 7, **dadurch gekennzeichnet, dass** die DNA-Sequenz von Interesse wenigstens ein Produkt codiert, das die Produktion des Lösemittels fördert, üblicherweise wenigstens ein Enzym, das an der Umwandlung von Aldehyden in Alkohol beteiligt ist, ein Membranprotein, einen Transkriptionsfaktor oder eine Kombination davon.

9. Werkzeug gemäß einem der Ansprüche 1 bis 8, **dadurch gekennzeichnet, dass** der Vektor ein Plasmid ist.

10. Verfahren zur Transformation eines lösemittelproduzierenden Bakteriums der Gattung *Clostridium* mittels homologer Rekombination, **dadurch gekennzeichnet, dass** es einen Schritt der Einführung in das Bakterium eines genetischen Werkzeuges gemäß einem der Ansprüche 1 bis 9 umfasst.

11. Verfahren gemäß Anspruch 10, **dadurch gekennzeichnet, dass** es die folgenden Schritte umfasst:
a) Einführung in das Bakterium eines genetischen Werkzeuges gemäß einem der Ansprüche 1 bis 8, das wenigstens einen induzierbaren Promotor umfasst, und
b) Induktion der Expression des induzierbaren Promotors zur genetischen Veränderung des Bakteriums.

12. Verfahren gemäß Anspruch 10 oder 11, **dadurch gekennzeichnet, dass** es nach Schritt b), sofern vorhanden, einen oder mehrere zusätzliche Schritte der Einführung einer weiteren Nukleinsäure umfasst, codierend i) ein Repair-Template, das sich von dem oder den bereits eingeführten Repair-Templates unterscheidet, und ii) ein oder mehrere guide-RNAs, die ihre Integration in eine gerichtete Zone des bakteriellen Genoms erlauben, wobei jedem zusätzlichen Schritt ein Schritt der Entfernung der Nukleinsäure, die das zuvor eingeführte Repair-Template codiert, und vorzugsweise der Entfernung der guide-RNA oder guide-RNAs oder Sequenzen vorausgeht, die die zuvor eingeführte guide-RNA oder eingeführten guide-RNAs codieren.

13. Lösemittelproduzierendes Bakterium der Gattung *Clostridium,* transformiert mithilfe des Verfahrens gemäß einem der Ansprüche 10 bis 12.

14. Kit zur Transformation eines Bakteriums der Gattung *Clostridium* oder zur Produktion wenigstens eines Lösemittels mithilfe eines Bakteriums der Gattung *Clostridium,* umfassend die Elemente des genetischen Werkzeuges wie in einem der Ansprüche 1 bis 9 beschrieben und gegebenenfalls ein oder mehrere Induktoren, die für den oder die im Werkzeug verwendeten ausgewählten induzierbaren Promotoren geeignet sind.

15. Verwendung des genetischen Werkzeuges gemäß einem der Ansprüche 1 bis 9, des Verfahrens gemäß einem der Ansprüche 10 bis 12 oder eines Bakteriums, transformiert gemäß Anspruch 13, um die Produktion eines Lösemittels oder einer Mischung von Lösemitteln in industriellem Maßstab, vorzugsweise Aceton, Butanol, Ethanol, Isopropanol oder einer Mischung davon, üblicherweise einer Isopropanol/Butanol-Mischung zu erlauben.

## Claims

1. A genetic tool allowing the transformation by homologous recombination of a solvent-forming bacterium of the genus *Clostridium,* **characterized in that** it comprises:
- a first nucleic acid encoding at least Cas9, wherein the sequence encoding Cas9 is placed under the control of a promoter, and
- at least one second nucleic acid containing a repair matrix allowing, by a homologous recombination mechanism, the replacement of a portion of the bacterial DNA targeted by Cas9 by a sequence of interest,
**in that** i) at least one of said nucleic acids further encodes one or more guide RNAs (gRNAs) or ii) the genetic tool further comprises one or more guide RNAs, each guide RNA comprising a Cas9 enzyme binding RNA structure and a sequence complementary to the targeted portion of the bacterial DNA, said complementary sequence comprising at least 10 nucleotides,
**in that** Cas9 causes double-stranded cleavage of the bacterial DNA, **in that** the bacterial DNA is the DNA of a solvent-forming bacterium of the genus *Clostridium,* and
**in that** each of the first and second nucleic acids present in the tool belongs to a distinct vector.

2. The tool as claimed in claim 1, **characterized in that** the sequence encoding the said guide RNA(s) is preceded by a promoter and **in that** the said promoter or the promoter controlling Cas9 is an inducible promoter.

3. The tool as claimed in claim 1 or 2, **characterized in that** the targeted portion of the bacterial DNA comprises an essential gene or gene portion essential for bacterial survival.

4. The tool as claimed in one of claims 1 to 3, **characterized in that** the solvent-forming bacterium of the genus *Clostridium* is selected from *C*. *acetobutylicum, C. cellulolyticum, C. phytofermentans, C. beijerinckii, C. saccharobutylicum, C. saccharoperbutylacetonicum, C. sporogenes, C. butyricum, C. aurantibutyricum* and *C. tyrobutyricum.*

5. The tool as claimed in claim 4, **characterized in that** when the solvent-forming bacterium is *C*. *acetobutylicum* said *C*. *acetobutylicum* bacterium is strain ATCC824, and when the solvent-forming bacterium is *C*. *beijerinckii* said *C*. *beijerinckii* bacterium is strain DSM 6423.

6. The tool as claimed in one of claims 1 to 5, **characterized in that** the Cas9 protein comprises the sequence SEQ ID NO: 1.

7. The tool as claimed in one of claims 1 to 6, **characterized in that** the Cas9 promoter is an inducible promoter.

8. The tool as claimed in one of claims 1 to 7, **characterized in that** the DNA sequence of interest encodes at least one solvent-producing product, typically at least one enzyme involved in the conversion of aldehydes to alcohol, a membrane protein, a transcription factor, or a combination thereof.

9. The tool as claimed in one of claims 1 to 8, **characterized in that** the vector is a plasmid.

10. A process for transforming by homologous recombination a solvent-forming bacterium of the genus *Clostridium,* **characterized in that** it comprises a step of introducing into the bacterium a genetic tool as claimed in one of claims 1 to 9.

11. The process as claimed in claim 10, **characterized in that** it comprises the following steps:
a) introduction into the bacterium of a genetic tool as claimed in one of claims 1 to 8 comprising at least one inducible promoter, and
b)induction of the expression of the inducible promoter enabling the bacterium to be genetically modified.

12. The process as claimed in claim 10 or 11, **characterized in that** it comprises one or more additional steps, subsequent to step b) when it is present, of introduction of an n^{th} nucleic acid encoding i) a repair matrix distinct from the one(s) already introduced and ii) one or more guide RNAs allowing their integration into a targeted zone of the genome of the bacterium, each additional step being preceded by a step of removal of the nucleic acid encoding the previously introduced repair matrix, and preferably of removal of the guide RNA(s) or sequences encoding the previously introduced guide RNA(s).

13. A solvent-forming bacterium of the genus *Clostridium* transformed by the process as claimed in one of claims 10 to 12.

14. A kit for transforming a bacterium of the genus *Clostridium* or for producing at least one solvent using a bacterium of the genus *Clostridium* comprising the elements of the genetic tool as described in one of claims 1 to 9 and optionally one or more inductors adapted to the selected inducible promoter(s) used within the tool.

15. Use of the genetic tool as claimed in one of claims 1 to 9, of the process as claimed in one of claims 10 to 12, or of a transformed bacterium as claimed in claim 13, for the production of a solvent or solvent mixture on an industrial scale, preferably acetone, butanol, ethanol, isopropanol or a mixture thereof, typically an isopropanol/butanol mixture.


### SEQUENCE LISTING

<110> IFP Energies Nouvelles
<120> OUTIL GENETIQUE DE TRANSFORMATION DE BACTERIES CLOSTRIDIUM
<130> B2115PC00
<160> 32
<170> PatentIn version 3.5
<210> 1
   <211> 1368
   <212> PRT
   <213> Streptococcus pyogenes
<400> 1
<210> 2
   <211> 4107
   <212> DNA
   <213> Streptococcus pyogenes
<400> 2
   atggataaga aatactcaat aggcttagat atcggcacaa atagcgtcgg atgggcggtg 60
   atcactgatg aatataaggt tccgtctaaa aagttcaagg ttctgggaaa tacagaccgc 120
   cacagtatca aaaaaaatct tataggggct cttttatttg acagtggaga gacagcggaa 180
   gcgactcgtc tcaaacggac agctcgtaga aggtatacac gtcggaagaa tcgtatttgt      240
   tatctacagg agattttttc aaatgagatg gcgaaagtag atgatagttt ctttcatcga       300
   cttgaagagt cttttttggt ggaagaagac aagaagcatg aacgtcatcc tatttttgga       360
   aatatagtag atgaagttgc ttatcatgag aaatatccaa ctatctatca tctgcgaaaa      420
   aaattggtag attctactga taaagcggat ttgcgcttaa tctatttggc cttagcgcat      480
   atgattaagt ttcgtggtca ttttttgatt gagggagatt taaatcctga taatagtgat      540
   gtggacaaac tatttatcca gttggtacaa acctacaatc aattatttga agaaaaccct      600
   attaacgcaa gtggagtaga tgctaaagcg attctttctg cacgattgag taaatcaaga      660
   cgattagaaa atctcattgc tcagctcccc ggtgagaaga aaaatggctt atttgggaat      720
   ctcattgctt tgtcattggg tttgacccct aattttaaat caaattttga tttggcagaa       780
   gatgctaaat tacagctttc aaaagatact tacgatgatg atttagataa tttattggcg      840
   caaattggag atcaatatgc tgatttgttt ttggcagcta agaatttatc agatgctatt      900
   ttactttcag atatcctaag agtaaatact gaaataacta aggctcccct atcagcttca      960
   atgattaaac gctacgatga acatcatcaa gacttgactc ttttaaaagc tttagttcga     1020
   caacaacttc cagaaaagta taaagaaatc ttttttgatc aatcaaaaaa cggatatgca     1080
   ggttatattg atgggggagc tagccaagaa gaattttata aatttatcaa accaatttta     1140
   gaaaaaatgg atggtactga ggaattattg gtgaaactaa atcgtgaaga tttgctgcgc     1200
   aagcaacgga cctttgacaa cggctctatt ccccatcaaa ttcacttggg tgagctgcat     1260
   gctattttga gaagacaaga agacttttat ccatttttaa aagacaatcg tgagaagatt     1320
   gaaaaaatct tgacttttcg aattccttat tatgttggtc cattggcgcg tggcaatagt     1380
   cgttttgcat ggatgactcg gaagtctgaa gaaacaatta ccccatggaa ttttgaagaa     1440
   gttgtcgata aaggtgcttc agctcaatca tttattgaac gcatgacaaa ctttgataaa     1500
   aatcttccaa atgaaaaagt actaccaaaa catagtttgc tttatgagta ttttacggtt     1560
   tataacgaat tgacaaaggt caaatatgtt actgaaggaa tgcgaaaacc agcatttctt     1620
   tcaggtgaac agaagaaagc cattgttgat ttactcttca aaacaaatcg aaaagtaacc     1680
   gttaagcaat taaaagaaga ttatttcaaa aaaatagaat gttttgatag tgttgaaatt     1740
   tcaggagttg aagatagatt taatgcttca ttaggtacct accatgattt gctaaaaatt     1800
   attaaagata aagatttttt ggataatgaa gaaaatgaag atatcttaga ggatattgtt     1860
   ttaacattga ccttatttga agatagggag atgattgagg aaagacttaa aacatatgct     1920
   cacctctttg atgataaggt gatgaaacag cttaaacgtc gccgttatac tggttgggga     1980
   cgtttgtctc gaaaattgat taatggtatt agggataagc aatctggcaa aacaatatta     2040
   gattttttga aatcagatgg ttttgccaat cgcaatttta tgcagctgat ccatgatgat 2100
   agtttgacat ttaaagaaga cattcaaaaa gcacaagtgt ctggacaagg cgatagttta 2160
   catgaacata ttgcaaattt agctggtagc cctgctatta aaaaaggtat tttacagact 2220
   gtaaaagttg ttgatgaatt ggtcaaagta atggggcggc ataagccaga aaatatcgtt 2280
   attgaaatgg cacgtgaaaa tcagacaact caaaagggcc agaaaaattc gcgagagcgt 2340
   atgaaacgaa tcgaagaagg tatcaaagaa ttaggaagtc agattcttaa agagcatcct 2400
   gttgaaaata ctcaattgca aaatgaaaag ctctatctct attatctcca aaatggaaga 2460
   gacatgtatg tggaccaaga attagatatt aatcgtttaa gtgattatga tgtcgatcac 2520
   attgttccac aaagtttcct taaagacgat tcaatagaca ataaggtctt aacgcgttct 2580
   gataaaaatc gtggtaaatc ggataacgtt ccaagtgaag aagtagtcaa aaagatgaaa 2640
   aactattgga gacaacttct aaacgccaag ttaatcactc aacgtaagtt tgataattta 2700
   acgaaagctg aacgtggagg tttgagtgaa cttgataaag ctggttttat caaacgccaa 2760
   ttggttgaaa ctcgccaaat cactaagcat gtggcacaaa ttttggatag tcgcatgaat 2820
   actaaatacg atgaaaatga taaacttatt cgagaggtta aagtgattac cttaaaatct 2880
   aaattagttt ctgacttccg aaaagatttc caattctata aagtacgtga gattaacaat 2940
   taccatcatg cccatgatgc gtatctaaat gccgtcgttg gaactgcttt gattaagaaa 3000
   tatccaaaac ttgaatcgga gtttgtctat ggtgattata aagtttatga tgttcgtaaa 3060
   atgattgcta agtctgagca agaaataggc aaagcaaccg caaaatattt cttttactct 3120
   aatatcatga acttcttcaa aacagaaatt acacttgcaa atggagagat tcgcaaacgc 3180
   cctctaatcg aaactaatgg ggaaactgga gaaattgtct gggataaagg gcgagatttt 3240
   gccacagtgc gcaaagtatt gtccatgccc caagtcaata ttgtcaagaa aacagaagta 3300
   cagacaggcg gattctccaa ggagtcaatt ttaccaaaaa gaaattcgga caagcttatt 3360
   gctcgtaaaa aagactggga tccaaaaaaa tatggtggtt ttgatagtcc aacggtagct 3420
   tattcagtcc tagtggttgc taaggtggaa aaagggaaat cgaagaagtt aaaatccgtt 3480
   aaagagttac tagggatcac aattatggaa agaagttcct ttgaaaaaaa tccgattgac 3540
   tttttagaag ctaaaggata taaggaagtt aaaaaagact taatcattaa actacctaaa 3600
   tatagtcttt ttgagttaga aaacggtcgt aaacggatgc tggctagtgc cggagaatta 3660
   caaaaaggaa atgagctggc tctgccaagc aaatatgtga attttttata tttagctagt 3720
   cattatgaaa agttgaaggg tagtccagaa gataacgaac aaaaacaatt gtttgtggag 3780
   cagcataagc attatttaga tgagattatt gagcaaatca gtgaattttc taagcgtgtt 3840
   attttagcag atgccaattt agataaagtt cttagtgcat ataacaaaca tagagacaaa 3900
   ccaatacgtg aacaagcaga aaatattatt catttattta cgttgacgaa tcttggagct 3960
   cccgctgctt ttaaatattt tgatacaaca attgatcgta aacgatatac gtctacaaaa 4020
   gaagttttag atgccactct tatccatcaa tccatcactg gtctttatga aacacgcatt 4080
   gatttgagtc agctaggagg tgactga 4107
<210> 3
   <211> 630
   <212> DNA
   <213> clostridium
<400> 3
   atgagtaaag ttacacaaat atcacatcca cttatattac acaaattagc atttatgaga 60
   gataaaaaaa caggatctaa agattttaga gagatggtag aagaagtagc aatgctaatg 120
   gcatatgaag taacaagaga aatgcagctt gaaactgttg aaatagaaac tcctatatgt 180
   ataactaaat gtaaaatgtt agcaggaaaa aaggtagcta tagttcctat acttagagca 240
   ggacttggaa tggtaaatgg agtattaaaa ttaatacctg ctgctaaggt tggacatata 300
   ggattatata gagatgaaaa gacattaaaa cctgtagaat acttctgtaa acttcctcaa 360
   gatatagagg aaagagacat aatagtaact gatccaatgc ttgcaactgg tgggtcagca 420
   atagatgcaa taacacttct taagaaaaga ggagcaaaat acataagact tatgtgtctt 480
   ataggagcac cagaaggtat agcagcagta caagaagcac atccagatgt agatatatac 540
   ctcgcatcaa tagatgaaaa gttagatgaa aatggatata tagttcctgg tcttggagat 600
   gctggagata gattattcgg tacaaaataa 630
<210> 4
   <211> 2589
   <212> DNA
   <213> clostridium
<400> 4
   atgaaagtca caacagtaaa ggaattagat gaaaaactca aggtaattaa agaagctcaa      60
   aaaaaattct cttgttactc gcaagaaatg gttgatgaaa tctttagaaa tgcagcaatg     120
   gcagcaatcg acgcaaggat agagctagca aaagcagctg ttttggaaac cggtatgggc     180
   ttagttgaag acaaggttat aaaaaatcat tttgcaggcg aatacatcta taacaaatat     240
   aaggatgaaa aaacctgcgg tataattgaa cgaaatgaac cctacggaat tacaaaaata     300
   gcagaaccta taggagttgt agctgctata atccctgtaa caaaccccac atcaacaaca     360
   atatttaaat ccttaatatc ccttaaaact agaaatggaa ttttcttttc gcctcaccca     420
   agggcaaaaa aatccacaat actagcagct aaaacaatac ttgatgcagc cgttaagagt     480
   ggtgccccgg aaaatataat aggttggata gatgaacctt caattgaact aactcaatat     540
   ttaatgcaaa aagcagatat aacccttgca actggtggtc cctcactagt taaatctgct     600
   tattcttccg gaaaaccagc aataggtgtt ggtccgggta acaccccagt aataattgat 660
   gaatctgctc atataaaaat ggcagtaagt tcaattatat tatccaaaac ctatgataat 720
   ggtgttatat gtgcttctga acaatctgta atagtcttaa aatccatata taacaaggta 780
   aaagatgagt tccaagaaag aggagcttat ataataaaga aaaacgaatt ggataaagtc 840
   cgtgaagtga tttttaaaga tggatccgta aaccctaaaa tagtcggaca gtcagcttat 900
   actatagcag ctatggctgg cataaaagta cctaaaacca caagaatatt aataggagaa 960
   gttacctcct taggtgaaga agaacctttt gcccacgaaa aactatctcc tgttttggct 1020
   atgtatgagg ctgacaattt tgatgatgct ttaaaaaaag cagtaactct aataaactta 1080
   ggaggcctcg gccatacctc aggaatatat gcagatgaaa taaaagcacg agataaaata 1140
   gatagattta gtagtgccat gaaaaccgta agaacctttg taaatatccc aacctcacaa 1200
   ggtgcaagtg gagatctata taattttaga ataccacctt ctttcacgct tggctgcgga 1260
   ttttggggag gaaattctgt ttccgagaat gttggtccaa aacatctttt gaatattaaa 1320
   accgtagctg aaaggagaga aaacatgctt tggtttagag ttccacataa agtatatttt 1380
   aagttcggtt gtcttcaatt tgctttaaaa gatttaaaag atctaaagaa aaaaagagcc 1440
   tttatagtta ctgatagtga cccctataat ttaaactatg ttgattcaat aataaaaata 1500
   cttgagcacc tagatattga ttttaaagta tttaataagg ttggaagaga agctgatctt 1560
   aaaaccataa aaaaagcaac tgaagaaatg tcctccttta tgccagacac tataatagct 1620
   ttaggtggta cccctgaaat gagctctgca aagctaatgt gggtactata tgaacatcca 1680
   gaagtaaaat ttgaagatct tgcaataaaa tttatggaca taagaaagag aatatatact 1740
   ttcccaaaac tcggtaaaaa ggctatgtta gttgcaatta caacttctgc tggttccggt 1800
   tctgaggtta ctccttttgc tttagtaact gacaataaca ctggaaataa gtacatgtta 1860
   gcagattatg aaatgacacc aaatatggca attgtagatg cagaacttat gatgaaaatg 1920
   ccaaagggat taaccgctta ttcaggtata gatgcactag taaatagtat agaagcatac 1980
   acatccgtat atgcttcaga atacacaaac ggactagcac tagaggcaat acgattaata 2040
   tttaaatatt tgcctgaggc ttacaaaaac ggaagaacca atgaaaaagc aagagagaaa 2100
   atggctcacg cttcaactat ggcaggtatg gcatccgcta atgcatttct aggtctatgt 2160
   cattccatgg caataaaatt aagttcagaa cacaatattc ctagtggcat tgccaatgca 2220
   ttactaatag aagaagtaat aaaatttaac gcagttgata atcctgtaaa acaagcccct 2280
   tgcccacaat ataagtatcc aaacaccata tttagatatg ctcgaattgc agattatata 2340
   aagcttggag gaaatactga tgaggaaaag gtagatctct taattaacaa aatacatgaa 2400
   ctaaaaaaag ctttaaatat accaacttca ataaaggatg caggtgtttt ggaggaaaac 2460
   ttctattcct cccttgatag aatatctgaa cttgcactag atgatcaatg cacaggcgct 2520
   aatcctagat ttcctcttac aagtgagata aaagaaatgt atataaattg ttttaaaaaa     2580
   caaccttaa                                                             2589
<210> 5
   <211> 10469
   <212> DNA
   <213> Artificial Sequence
<220>
   <223> plasmide
<400> 5
   catggataaa aagtacagta ttggtctaga cataggaact aactctgttg ggtgggctgt      60
   tataacagat gaatataaag ttccatcaaa aaaatttaaa gtattaggaa acactgatag     120
   acattcaata aaaaaaaact tgataggtgc tttattattc gattcaggag agactgctga     180
   agctacacgt ttaaaaagaa cagctagacg tagatataca agaagaaaaa ataggatatg     240
   ttatcttcaa gaaattttta gtaatgaaat ggcaaaagtt gatgattcat tctttcacag     300
   actagaagaa agtttcttag ttgaagaaga taagaagcat gaaagacacc ctatttttgg     360
   taatatcgta gatgaagtag catatcatga gaagtatcca actatctatc atttaagaaa     420
   gaaattagtt gattctacag ataaagctga tctgagatta atatatttag ctttagctca     480
   tatgattaaa tttagaggac attttttaat agaaggtgat ttaaacccag acaacagcga      540
   tgtagataaa ttatttatcc aattagttca aacttataat caattattcg aagagaatcc     600
   aattaatgca agtggtgtag acgctaaggc tatattatca gctagattat caaaatctag     660
   aagattagaa aatctaatag ctcaacttcc tggagaaaag aaaaatggac tttttgggaa     720
   cctaatagct ctctcactcg gactaacacc aaattttaaa agcaattttg atcttgctga     780
   agacgcaaag ttacaactat caaaggatac atacgatgat gatttagata atttgttagc     840
   tcaaataggt gatcaatatg ctgatttgtt tcttgcagca aaaaacttaa gtgatgcaat     900
   tttactatca gatatactta gagtaaatac agaaataaca aaggctcctt tatcagcaag     960
   tatgattaaa cgatatgatg agcatcatca agatttaaca ttattaaagg cacttgtaag    1020
   acaacaatta ccagaaaaat ataaagaaat tttctttgat caatctaaaa atggatatgc    1080
   tggatatata gacggtggag caagtcaaga agagttttat aaatttataa agcctatttt    1140
   agaaaaaatg gatggaactg aagaattact tgttaaactt aacagagaag atttacttag    1200
   aaaacaaaga acttttgata atggttcaat tcctcaccaa attcatttag gagaattaca    1260
   tgctatacta agaagacaag aagattttta tccatttctt aaagataata gagaaaaaat    1320
   tgaaaaaatt ttaactttta gaataccata ttatgtagga ccacttgcaa ggggaaattc    1380
   aagatttgca tggatgacta gaaaatcaga agaaactata accccgtgga attttgaaga    1440
   agtagtagat aaaggagcta gtgctcaatc atttatagaa agaatgacaa attttgataa 1500
   gaatcttcct aacgaaaagg ttttgccaaa gcatagcctt ctttatgagt attttacagt 1560
   ttataatgag cttactaaag taaaatacgt tacagaagga atgagaaaac cagcattttt 1620
   gtctggtgaa caaaagaaag caatagtaga cctattattt aaaacaaata ggaaggttac 1680
   cgtaaagcaa cttaaagaag attacttcaa aaaaattgaa tgctttgata gtgttgaaat 1740
   atcaggagtt gaagatagat ttaatgcttc acttggtaca tatcacgatc tcttaaaaat 1800
   tataaaagat aaggattttt tagataatga agaaaatgaa gatattcttg aagatatagt 1860
   attaacattg acactttttg aagatagaga aatgatagaa gaaagattaa aaacatatgc 1920
   acatcttttt gatgataagg ttatgaagca acttaaaaga agaagatata caggttgggg 1980
   acgtttgtca agaaagctaa ttaatggtat tagagataaa caatcaggaa agactattct 2040
   cgattttctt aaatcagatg gatttgctaa tagaaacttt atgcaattaa ttcatgatga 2100
   ttctcttact ttcaaagagg atattcaaaa ggctcaagtt tctggacaag gcgatagctt 2160
   acacgaacac attgctaacc ttgcagggag ccccgctatc aaaaaaggaa ttttacaaac 2220
   agttaaagtt gtagatgaac ttgttaaagt tatgggaaga cacaaacctg agaatatagt 2280
   tatagaaatg gccagagaaa atcaaacaac acaaaaagga caaaaaaatt ctagagagag 2340
   aatgaagaga attgaagaag gaataaaaga gctaggatca caaatattaa aagaacatcc 2400
   agttgaaaat actcaattgc aaaatgaaaa gttatatttg tattacttac aaaatggaag 2460
   agatatgtat gttgatcaag aactcgatat taatagatta agtgactatg atgttgatca 2520
   tattgttcct caatcatttt taaaagatga ttcaatcgat aacaaagtat taactagatc 2580
   agataaaaat agaggaaagt cagataatgt accatctgaa gaagttgtta aaaaaatgaa 2640
   gaactattgg agacaacttt taaatgcaaa gctaattaca caaagaaaat ttgacaattt 2700
   aacaaaagca gaaagaggag gattaagcga attagacaaa gctggattta taaaaagaca 2760
   acttgttgag acaagacaaa taactaagca tgttgctcaa atacttgatt caagaatgaa 2820
   tacaaaatat gatgaaaatg ataaattaat cagagaagta aaagtaataa cattaaagtc 2880
   aaaattagta tcagatttca gaaaggattt tcaattttac aaagttcgtg aaataaataa 2940
   ctatcatcat gctcatgatg catacttaaa tgctgttgta ggaactgctc ttattaagaa 3000
   atatcctaaa ctagaaagcg aatttgttta tggagattat aaagtttatg atgtgcgcaa 3060
   aatgatcgcg aaatccgaac aagaaatcgg taaggctaca gcaaaatatt tcttttatag 3120
   taatataatg aattttttta agacagaaat aactttggct aatggtgaaa tcagaaaaag 3180
   accacttatc gaaacaaatg gagagacagg agaaatagta tgggataaag gaagagattt 3240
   tgctactgtt agaaaagtac taagtatgcc acaagtaaat atcgtaaaga aaactgaagt 3300
   tcaaactgga ggtttctcta aggaatcaat tttacctaag agaaattcag ataagttaat 3360
   tgcaaggaaa aaagattggg acccaaaaaa atacggtggt tttgatagtc caacagttgc 3420
   ctatagtgtt cttgtagtag cgaaagttga gaaaggtaag tcaaaaaagt tgaaaagcgt 3480
   aaaagaactt cttggtatca caattatgga aagatcttca tttgaaaaaa atccaattga 3540
   ctttttagaa gctaagggtt ataaagaagt taaaaaggat ttaatcataa aactaccaaa 3600
   gtatagtcta tttgaactcg aaaacggaag aaaacgaatg ctcgctagcg caggagaact 3660
   tcaaaaagga aatgaacttg cgctgccatc aaagtatgta aatttcttat atttagcttc 3720
   tcattatgag aaattaaaag gatcaccaga ggataatgaa caaaagcaac tatttgtaga 3780
   acaacacaaa cattatttag atgaaataat agaacaaata tctgaatttt ctaaaagagt 3840
   tatacttgcc gacgcaaatc tagataaggt gctttcagcg tataataaac acagagataa 3900
   accaataaga gaacaagcag aaaacattat ccatcttttt acattaacta atcttggtgc 3960
   accagctgca tttaagtact ttgatacaac aatagataga aaaagataca catctactaa 4020
   agaagtatta gacgcaactt taatacatca atctattaca gggctttatg aaacaagaat 4080
   tgatttaagt caactaggcg gagattaagt cgacaaagta ttgttaaaaa taactctgta 4140
   gaattataaa ttagttctac agagttattt tttgacccgg gtaccgagct cgaattcgta 4200
   atcatggtca tagctgtttc ctgtgtgaaa ttgttatccg ctcacaattc cacacaacat 4260
   acgagccgga agcataaagt gtaaagcctg gggtgcctaa tgagtgagct aactcacatt 4320
   aattgcgttg cgctcactgc ccgctttcca gtcgggaaac ctgtcgtgcc agctgcatta 4380
   atgaatcggc caacgcgcgg ggagaggcgg tttgcgtatt gggcgctctt ccgcttcctc 4440
   gctcactgac tcgctgcgct cggtcgttcg gctgcggcga gcggtatcag ctcactcaaa 4500
   ggcggtaata cggttatcca cagaatcagg ggataacgca ggaaagaaca tgtgagcaaa 4560
   aggccagcaa aaggccagga accgtaaaaa ggccgcgttg ctggcgtttt tccataggct 4620
   ccgcccccct gacgagcatc acaaaaatcg acgctcaagt cagaggtggc gaaacccgac 4680
   aggactataa agataccagg cgtttccccc tggaagctcc ctcgtgcgct ctcctgttcc 4740
   gaccctgccg cttaccggat acctgtccgc ctttctccct tcgggaagcg tggcgctttc 4800
   tcatagctca cgctgtaggt atctcagttc ggtgtaggtc gttcgctcca agctgggctg 4860
   tgtgcacgaa ccccccgttc agcccgaccg ctgcgcctta tccggtaact atcgtcttga 4920
   gtccaacccg gtaagacacg acttatcgcc actggcagca gccactggta acaggattag 4980
   cagagcgagg tatgtaggcg gtgctacaga gttcttgaag tggtggccta actacggcta 5040
   cactagaagg acagtatttg gtatctgcgc tctgctgaag ccagttacct tcggaaaaag 5100
   agttggtagc tcttgatccg gcaaacaaac caccgctggt agcggtggtt tttttgtttg 5160
   caagcagcag attacgcgca gaaaaaaagg atctcaagaa gatcctttga tcttttctac 5220
   ggggtctgac gctcagtgga acgaaaactc acgttaaggg attttggtca tgagattatc 5280
   aaaaaggatc ttcacctaga tccttttaaa ttaaaaatga agttttaaat caatctaaag 5340
   tatatatgag taaacttggt ctgacagtta ccaatgctta atcagtgagg cacctatctc 5400
   agcgatctgt ctatttcgtt catccatagt tgcctgactc cccgtcgtgt agataactac 5460
   gatacgggag ggcttaccat ctggccccag tgctgcaatg ataccgcgag acccacgctc 5520
   accggctcca gatttatcag caataaacca gccagccgga agggccgagc gcagaagtgg 5580
   tcctgcaact ttatccgcct ccatccagtc tattaattgt tgccgggaag ctagagtaag 5640
   tagttcgcca gttaatagtt tgcgcaacgt tgttgccatt gctacaggca tcgtggtgtc 5700
   acgctcgtcg tttggtatgg cttcattcag ctccggttcc caacgatcaa ggcgagttac 5760
   atgatccccc atgttgtgca aaaaagcggt tagctccttc ggtcctccga tcgttgtcag 5820
   aagtaagttg gccgcagtgt tatcactcat ggttatggca gcactgcata attctcttac 5880
   tgtcatgcca tccgtaagat gcttttctgt gactggtgag tactcaacca agtcattctg 5940
   agaatagtgt atgcggcgac cgagttgctc ttgcccggcg tcaatacggg ataataccgc 6000
   gccacatagc agaactttaa aagtgctcat cattggaaaa cgttcttcgg ggcgaaaact 6060
   ctcaaggatc ttaccgctgt tgagatccag ttcgatgtaa cccactcgtg cacccaactg 6120
   atcttcagca tcttttactt tcaccagcgt ttctgggtga gcaaaaacag gaaggcaaaa 6180
   tgccgcaaaa aagggaataa gggcgacacg gaaatgttga atactcatac tcttcctttt 6240
   tcaatattat tgaagcattt atcagggtta ttgtctcatg agcggataca tatttgaatg 6300
   tatttagaaa aataaacaaa taggggttcc gcgcacattt ccccgaaaag tgccacctga 6360
   ctgccgggcc tcttgcggga tcaaaagaaa aacgaaatga tacaccaatc agtgcaaaaa 6420
   aagatataat gggagataag acggttcgtg ttcgtgctga cttgcaccat atcataaaaa 6480
   tcgaaacagc aaagaatggc ggaaacgtaa aagaagttat ggaaataaga cttagaagca 6540
   aacttaagag tgtgttgata gtgcagtatc ttaaaatttt gtataatagg aattgaagtt 6600
   aaattagatg ctaaaaattt gtaattaaga aggagtgatt acatgaacaa aaatataaaa 6660
   tattctcaaa actttttaac gagtgaaaaa gtactcaacc aaataataaa acaattgaat 6720
   ttaaaagaaa ccgataccgt ttacgaaatt ggaacaggta aagggcattt aacgacgaaa 6780
   ctggctaaaa taagtaaaca ggtaacgtct attgaattag acagtcatct attcaactta 6840
   tcgtcagaaa aattaaaact gaatactcgt gtcactttaa ttcaccaaga tattctacag 6900
   tttcaattcc ctaacaaaca gaggtataaa attgttggga gtattcctta ccatttaagc 6960
   acacaaatta ttaaaaaagt ggtttttgaa agccatgcgt ctgacatcta tctgattgtt 7020
   gaagaaggat tctacaagcg taccttggat attcaccgaa cactagggtt gctcttgcac 7080
   actcaagtct cgattcagca attgcttaag ctgccagcgg aatgctttca tcctaaacca 7140
   aaagtaaaca gtgtcttaat aaaacttacc cgccatacca cagatgttcc agataaatat 7200
   tggaagctat atacgtactt tgtttcaaaa tgggtcaatc gagaatatcg tcaactgttt 7260
   actaaaaatc agtttcatca agcaatgaaa cacgccaaag taaacaattt aagtaccgtt 7320
   acttatgagc aagtattgtc tatttttaat agttatctat tatttaacgg gaggaaataa 7380
   ttctatgagt ccctaggccc aactaactca acgctagtag tggatttaat cccaaatgag 7440
   ccaacagaac cagaaccaga aacagaatca gaacaagtaa cattggattt agaaatggaa 7500
   gaagaaaaaa gcaatgactt cgtgtgaata atgcacgaaa tcgttgctta ttttttttta 7560
   aaagcggtat actagatata acgaaacaac gaactgaata gaaacgaaaa aagagccatg 7620
   acacatttat aaaatgtttg acgacatttt ataaatgcat agcccgataa gattgccaaa 7680
   ccaacgctta tcagttagtc agatgaactc ttccctcgta agaagttatt taattaactt 7740
   tgtttgaaga cggtatataa ccgtactatc attatatagg gaaatcagag agttttcaag 7800
   tatctaagct actgaattta agaattgtta agcaatcaat cggaaatcgt ttgattgctt 7860
   tttttgtatt catttataga aggtggagtt tgtatgaatc atgatgaatg taaaacttat 7920
   ataaaaaata gtttattgga gataagaaaa ttagcaaata tctatacact agaaacgttt 7980
   aagaaagagt tagaaaagag aaatatctac ttagaaacaa aatcagataa gtatttttct 8040
   tcggaggggg aagattatat atataagtta atagaaaata acaaaataat ttattcgatt 8100
   agtggaaaaa aattgactta taaaggaaaa aaatcttttt caaaacatgc aatattgaaa 8160
   cagttgaatg aaaaagcaaa ccaagttaat taaacaacct attttatagg atttatagga 8220
   aaggagaaca gctgaatgaa tatccctttt gttgtagaaa ctgtgcttca tgacggcttg 8280
   ttaaagtaca aatttaaaaa tagtaaaatt cgctcaatca ctaccaagcc aggtaaaagc 8340
   aaaggggcta tttttgcgta tcgctcaaaa tcaagcatga ttggcggtcg tggtgttgtt 8400
   ctgacttccg aggaagcgat tcaagaaaat caagatacat ttacacattg gacacccaac 8460
   gtttatcgtt atggaacgta tgcagacgaa aaccgttcat acacgaaagg acattctgaa 8520
   aacaatttaa gacaaatcaa taccttcttt attgattttg atattcacac ggcaaaagaa 8580
   actatttcag caagcgatat tttaacaacc gctattgatt taggttttat gcctactatg 8640
   attatcaaat ctgataaagg ttatcaagca tattttgttt tagaaacgcc agtctatgtg 8700
   acttcaaaat cagaatttaa atctgtcaaa gcagccaaaa taatttcgca aaatatccga 8760
   gaatattttg gaaagtcttt gccagttgat ctaacgtgta atcattttgg tattgctcgc 8820
   ataccaagaa cggacaatgt agaatttttt gatcctaatt accgttattc tttcaaagaa 8880
   tggcaagatt ggtctttcaa acaaacagat aataagggct ttactcgttc aagtctaacg 8940
   gttttaagcg gtacagaagg caaaaaacaa gtagatgaac cctggtttaa tctcttattg 9000
   cacgaaacga aattttcagg agaaaagggt ttaatagggc gtaataacgt catgtttacc      9060
   ctctctttag cctactttag ttcaggctat tcaatcgaaa cgtgcgaata taatatgttt      9120
   gagtttaata atcgattaga tcaaccctta gaagaaaaag aagtaatcaa aattgttaga      9180
   agtgcctatt cagaaaacta tcaaggggct aatagggaat acattaccat tctttgcaaa      9240
   gcttgggtat caagtgattt aaccagtaaa gatttatttg tccgtcaagg gtggtttaaa      9300
   ttcaagaaaa aaagaagcga acgtcaacgt gttcatttgt cagaatggaa agaagattta      9360
   atggcttata ttagcgaaaa aagcgatgta tacaagcctt atttagtgac gaccaaaaaa      9420
   gagattagag aagtgctagg cattcctgaa cggacattag ataaattgct gaaggtactg      9480
   aaggcgaatc aggaaatttt ctttaagatt aaaccaggaa gaaatggtgg cattcaactt      9540
   gctagtgtta aatcattgtt gctatcgatc attaaagtaa aaaaagaaga aaaagaaagc      9600
   tatataaagg cgctgacaaa ttcttttgac ttagagcata cattcattca agagacttta      9660
   aacaagctag cagaacgccc taaaacggac acacaactcg atttgtttag ctatgataca      9720
   ggctgaaaat aaaacccgca ctatgccatt acatttatat ctatgatacg tgtttgtttt      9780
   ttctttgctg tttagcgaat gattagcaga aatatacaga gtaagatttt aattaattat      9840
   tagggggaga aggagagagt agcccgaaaa cttttagttg gcttggactg aacgaagtga      9900
   gggaaaggct actaaaacgt cgaggggcag tgagagcgaa gcgaacactt gattttttaa      9960
   ttttctatct tttataggtc attagagtat acttatttgt cctataaact atttagcagc     10020
   ataatagatt tattgaatag gtcatttaag ttgagcatat tagaggagga aaatcttgga     10080
   gaaatatttg aagaacccga ttacatggat tggattagtt cttgtggtta cgtggttttt     10140
   aactaaaagt agtgaatttt tgatttttgg tgtgtgtgtc ttgttgttag tatttgctag     10200
   tcaaagtgat taaatagaat tctagcgcca ttcgccattc aggctgcgca actgttggga     10260
   agggcgatcg gtgcgggcct cttcgctatt acgccagctg gcgaaagggg gatgtgctgc     10320
   aaggcgatta agttgggtaa cgccagggtt ttcccagtca cgacgttgta aaacgacggc     10380
   cagtgccaag cttgcatgcc tgcaggcctc gagtatattg ataaaaataa taatagtggg     10440
   tataattaag ttgttaggag gttagttac                                      10469
<210> 6
   <211> 8876
   <212> DNA
   <213> Artificial Sequence
<220>
   <223> plasmide
<400> 6
   tcgagactct atcattgata gagtttgaaa ctctatcatt gatagagtat aatatctttg      60
   ttcattagag cgataaactt gaatttgaga gggaacttcc atggataaaa agtacagtat     120
   tggtctagac ataggaacta actctgttgg gtgggctgtt ataacagatg aatataaagt 180
   tccatcaaaa aaatttaaag tattaggaaa cactgataga cattcaataa aaaaaaactt 240
   gataggtgct ttattattcg attcaggaga gactgctgaa gctacacgtt taaaaagaac 300
   agctagacgt agatatacaa gaagaaaaaa taggatatgt tatcttcaag aaatttttag 360
   taatgaaatg gcaaaagttg atgattcatt ctttcacaga ctagaagaaa gtttcttagt 420
   tgaagaagat aagaagcatg aaagacaccc tatttttggt aatatcgtag atgaagtagc 480
   atatcatgag aagtatccaa ctatctatca tttaagaaag aaattagttg attctacaga 540
   taaagctgat ctgagattaa tatatttagc tttagctcat atgattaaat ttagaggaca 600
   ttttttaata gaaggtgatt taaacccaga caacagcgat gtagataaat tatttatcca 660
   attagttcaa acttataatc aattattcga agagaatcca attaatgcaa gtggtgtaga 720
   cgctaaggct atattatcag ctagattatc aaaatctaga agattagaaa atctaatagc 780
   tcaacttcct ggagaaaaga aaaatggact ttttgggaac ctaatagctc tctcactcgg 840
   actaacacca aattttaaaa gcaattttga tcttgctgaa gacgcaaagt tacaactatc 900
   aaaggataca tacgatgatg atttagataa tttgttagct caaataggtg atcaatatgc 960
   tgatttgttt cttgcagcaa aaaacttaag tgatgcaatt ttactatcag atatacttag 1020
   agtaaataca gaaataacaa aggctccttt atcagcaagt atgattaaac gatatgatga 1080
   gcatcatcaa gatttaacat tattaaaggc acttgtaaga caacaattac cagaaaaata 1140
   taaagaaatt ttctttgatc aatctaaaaa tggatatgct ggatatatag acggtggagc 1200
   aagtcaagaa gagttttata aatttataaa gcctatttta gaaaaaatgg atggaactga 1260
   agaattactt gttaaactta acagagaaga tttacttaga aaacaaagaa cttttgataa 1320
   tggttcaatt cctcaccaaa ttcatttagg agaattacat gctatactaa gaagacaaga 1380
   agatttttat ccatttctta aagataatag agaaaaaatt gaaaaaattt taacttttag 1440
   aataccatat tatgtaggac cacttgcaag gggaaattca agatttgcat ggatgactag 1500
   aaaatcagaa gaaactataa ccccgtggaa ttttgaagaa gtagtagata aaggagctag 1560
   tgctcaatca tttatagaaa gaatgacaaa ttttgataag aatcttccta acgaaaaggt 1620
   tttgccaaag catagccttc tttatgagta ttttacagtt tataatgagc ttactaaagt 1680
   aaaatacgtt acagaaggaa tgagaaaacc agcatttttg tctggtgaac aaaagaaagc 1740
   aatagtagac ctattattta aaacaaatag gaaggttacc gtaaagcaac ttaaagaaga 1800
   ttacttcaaa aaaattgaat gctttgatag tgttgaaata tcaggagttg aagatagatt 1860
   taatgcttca cttggtacat atcacgatct cttaaaaatt ataaaagata aggatttttt 1920
   agataatgaa gaaaatgaag atattcttga agatatagta ttaacattga cactttttga 1980
   agatagagaa atgatagaag aaagattaaa aacatatgca catctttttg atgataaggt 2040
   tatgaagcaa cttaaaagaa gaagatatac aggttgggga cgtttgtcaa gaaagctaat 2100
   taatggtatt agagataaac aatcaggaaa gactattctc gattttctta aatcagatgg 2160
   atttgctaat agaaacttta tgcaattaat tcatgatgat tctcttactt tcaaagagga 2220
   tattcaaaag gctcaagttt ctggacaagg cgatagctta cacgaacaca ttgctaacct 2280
   tgcagggagc cccgctatca aaaaaggaat tttacaaaca gttaaagttg tagatgaact 2340
   tgttaaagtt atgggaagac acaaacctga gaatatagtt atagaaatgg ccagagaaaa 2400
   tcaaacaaca caaaaaggac aaaaaaattc tagagagaga atgaagagaa ttgaagaagg 2460
   aataaaagag ctaggatcac aaatattaaa agaacatcca gttgaaaata ctcaattgca 2520
   aaatgaaaag ttatatttgt attacttaca aaatggaaga gatatgtatg ttgatcaaga 2580
   actcgatatt aatagattaa gtgactatga tgttgatcat attgttcctc aatcattttt 2640
   aaaagatgat tcaatcgata acaaagtatt aactagatca gataaaaata gaggaaagtc 2700
   agataatgta ccatctgaag aagttgttaa aaaaatgaag aactattgga gacaactttt 2760
   aaatgcaaag ctaattacac aaagaaaatt tgacaattta acaaaagcag aaagaggagg 2820
   attaagcgaa ttagacaaag ctggatttat aaaaagacaa cttgttgaga caagacaaat 2880
   aactaagcat gttgctcaaa tacttgattc aagaatgaat acaaaatatg atgaaaatga 2940
   taaattaatc agagaagtaa aagtaataac attaaagtca aaattagtat cagatttcag 3000
   aaaggatttt caattttaca aagttcgtga aataaataac tatcatcatg ctcatgatgc 3060
   atacttaaat gctgttgtag gaactgctct tattaagaaa tatcctaaac tagaaagcga 3120
   atttgtttat ggagattata aagtttatga tgtgcgcaaa atgatcgcga aatccgaaca 3180
   agaaatcggt aaggctacag caaaatattt cttttatagt aatataatga atttttttaa 3240
   gacagaaata actttggcta atggtgaaat cagaaaaaga ccacttatcg aaacaaatgg 3300
   agagacagga gaaatagtat gggataaagg aagagatttt gctactgtta gaaaagtact 3360
   aagtatgcca caagtaaata tcgtaaagaa aactgaagtt caaactggag gtttctctaa 3420
   ggaatcaatt ttacctaaga gaaattcaga taagttaatt gcaaggaaaa aagattggga 3480
   cccaaaaaaa tacggtggtt ttgatagtcc aacagttgcc tatagtgttc ttgtagtagc 3540
   gaaagttgag aaaggtaagt caaaaaagtt gaaaagcgta aaagaacttc ttggtatcac 3600
   aattatggaa agatcttcat ttgaaaaaaa tccaattgac tttttagaag ctaagggtta 3660
   taaagaagtt aaaaaggatt taatcataaa actaccaaag tatagtctat ttgaactcga 3720
   aaacggaaga aaacgaatgc tcgctagcgc aggagaactt caaaaaggaa atgaacttgc 3780
   gctgccatca aagtatgtaa atttcttata tttagcttct cattatgaga aattaaaagg 3840
   atcaccagag gataatgaac aaaagcaact atttgtagaa caacacaaac attatttaga 3900
   tgaaataata gaacaaatat ctgaattttc taaaagagtt atacttgccg acgcaaatct 3960
   agataaggtg ctttcagcgt ataataaaca cagagataaa ccaataagag aacaagcaga 4020
   aaacattatc catcttttta cattaactaa tcttggtgca ccagctgcat ttaagtactt 4080
   tgatacaaca atagatagaa aaagatacac atctactaaa gaagtattag acgcaacttt 4140
   aatacatcaa tctattacag ggctttatga aacaagaatt gatttaagtc aactaggcgg 4200
   agattaagtc gacaaagtat tgttaaaaat aactctgtag aattataaat tagttctaca 4260
   gagttatttt ttgacccggg tatattgata aaaataataa tagtgggtat aattaagttg 4320
   ttaggaggtt agttagaatg atgtcaagat tagataaaag taaagtgatt aacagcgcat 4380
   tagagctgct taatgaggtc ggaatcgaag gtttaacaac ccgtaaactc gcccagaagc 4440
   taggtgtaga gcagcctaca ttgtattggc atgtaaaaaa taagcgggct ttgctcgacg 4500
   ccttagccat tgagatgtta gataggcacc atactcactt ttgcccttta gaaggggaaa 4560
   gctggcaaga ttttttacgt aataacgcta aaagttttag atgtgcttta ctaagtcatc 4620
   gcgatggagc aaaagtacat ttaggtacac ggcctacaga aaaacagtat gaaactctcg 4680
   aaaatcaatt agccttttta tgccaacaag gtttttcact agagaatgca ttatatgcac 4740
   tcagcgctgt ggggcatttt actttaggtt gcgtattgga agatcaagag catcaagtcg 4800
   ctaaagaaga aagggaaaca cctactactg atagtatgcc gccattatta cgacaagcta 4860
   tcgaattatt tgatcaccaa ggtgcagagc cagccttctt attcggcctt gaattgatca 4920
   tatgcggatt agaaaaacaa cttaaatgtg aaagtgggtc ttaaaagcag cataaccttt 4980
   ttccgtgatg gtaacttcac ggtaaccaag atgtcgagtt gagctcgaat tcgtaatcat 5040
   ggtcatagct gtttcctgtg tgaaattgtt atccgctcac aattccacac aacatacgag 5100
   ccggaagcat aaagtgtaaa gcctggggtg cctaatgagt gagctaactc acattaattg 5160
   cgttgcgctc actgcccgct ttccagtcgg gaaacctgtc gtgccagctg cattaatgaa 5220
   tcggccaacg cgcggggaga ggcggtttgc gtattgggcg ctcttccgct tcctcgctca 5280
   ctgactcgct gcgctcggtc gttcggctgc ggcgagcggt atcagctcac tcaaaggcgg 5340
   taatacggtt atccacagaa tcaggggata acgcaggaaa gaacatgtga gcaaaaggcc 5400
   agcaaaaggc caggaaccgt aaaaaggccg cgttgctggc gtttttccat aggctccgcc 5460
   cccctgacga gcatcacaaa aatcgacgct caagtcagag gtggcgaaac ccgacaggac 5520
   tataaagata ccaggcgttt ccccctggaa gctccctcgt gcgctctcct gttccgaccc 5580
   tgccgcttac cggatacctg tccgcctttc tcccttcggg aagcgtggcg ctttctcata 5640
   gctcacgctg taggtatctc agttcggtgt aggtcgttcg ctccaagctg ggctgtgtgc 5700
   acgaaccccc cgttcagccc gaccgctgcg ccttatccgg taactatcgt cttgagtcca 5760
   acccggtaag acacgactta tcgccactgg cagcagccac tggtaacagg attagcagag 5820
   cgaggtatgt aggcggtgct acagagttct tgaagtggtg gcctaactac ggctacacta 5880
   gaaggacagt atttggtatc tgcgctctgc tgaagccagt taccttcgga aaaagagttg 5940
   gtagctcttg atccggcaaa caaaccaccg ctggtagcgg tggttttttt gtttgcaagc 6000
   agcagattac gcgcagaaaa aaaggatctc aagaagatcc tttgatcttt tctacggggt 6060
   ctgacgctca gtggaacgaa aactcacgtt aagggatttt ggtcatgaga ttatcaaaaa 6120
   ggatcttcac ctagatcctt ttaaattaaa aatgaagttt taaatcaatc taaagtatat 6180
   atgagtaaac ttggtctgac agttaccagg tccactgccg ggcctcttgc gggatcaaaa 6240
   gaaaaacgaa atgatacacc aatcagtgca aaaaaagata taatgggaga taagacggtt 6300
   cgtgttcgtg ctgacttgca ccatatcata aaaatcgaaa cagcaaagaa tggcggaaac 6360
   gtaaaagaag ttatggaaat aagacttaga agcaaactta agagtgtgtt gatagtgcag 6420
   tatcttaaaa ttttgtataa taggaattga agttaaatta gatgctaaaa atttgtaatt 6480
   aagaaggagt gattacatga acaaaaatat aaaatattct caaaactttt taacgagtga 6540
   aaaagtactc aaccaaataa taaaacaatt gaatttaaaa gaaaccgata ccgtttacga 6600
   aattggaaca ggtaaagggc atttaacgac gaaactggct aaaataagta aacaggtaac 6660
   gtctattgaa ttagacagtc atctattcaa cttatcgtca gaaaaattaa aactgaatac 6720
   tcgtgtcact ttaattcacc aagatattct acagtttcaa ttccctaaca aacagaggta 6780
   taaaattgtt gggagtattc cttaccattt aagcacacaa attattaaaa aagtggtttt 6840
   tgaaagccat gcgtctgaca tctatctgat tgttgaagaa ggattctaca agcgtacctt 6900
   ggatattcac cgaacactag ggttgctctt gcacactcaa gtctcgattc agcaattgct 6960
   taagctgcca gcggaatgct ttcatcctaa accaaaagta aacagtgtct taataaaact 7020
   tacccgccat accacagatg ttccagataa atattggaag ctatatacgt actttgtttc 7080
   aaaatgggtc aatcgagaat atcgtcaact gtttactaaa aatcagtttc atcaagcaat 7140
   gaaacacgcc aaagtaaaca atttaagtac cgttacttat gagcaagtat tgtctatttt 7200
   taatagttat ctattattta acgggaggaa ataattctat gagtccctag gcaggcctcc 7260
   gccattattt ttttgaacaa ttgacaattc atttcttatt ttttattaag tgatagtcaa 7320
   aaggcataac agtgctgaat agaaagaaat ttacagaaaa gaaaattata gaatttagta 7380
   tgattaatta tactcattta tgaatgttta attgaataca aaaaaaaata cttgttatgt 7440
   attcaattac gggttaaaat atagacaagt tgaaaaattt aataaaaaaa taagtcctca 7500
   gctcttatat attaagctac caacttagta tataagccaa aacttaaatg tgctaccaac 7560
   acatcaagcc gttagagaac tctatctata gcaatatttc aaatgtaccg acatacaaga 7620
   gaaacattaa ctatatatat tcaatttatg agattatctt aacagatata aatgtaaatt 7680
   gcaataagta agatttagaa gtttatagcc tttgtgtatt ggaagcagta cgcaaaggct      7740
   tttttatttg ataaaaatta gaagtatatt tattttttca taattaattt atgaaaatga      7800
   aagggggtga gcaaagtgac agaggaaagc agtatcttat caaataacaa ggtattagca      7860
   atatcattat tgactttagc agtaaacatt atgactttta tagtgcttgt agctaagtag      7920
   tacgaaaggg ggagctttaa aaagctcctt ggaatacata gaattcataa attaatttat      7980
   gaaaagaagg gcgtatatga aaacttgtaa aaattgcaaa gagtttatta aagatactga      8040
   aatatgcaaa atacattcgt tgatgattca tgataaaaca gtagcaacct attgcagtaa      8100
   atacaatgag tcaagatgtt tacataaagg gaaagtccaa tgtattaatt gttcaaagat      8160
   gaaccgatat ggatggtgtg ccataaaaat gagatgtttt acagaggaag aacagaaaaa      8220
   agaacgtaca tgcattaaat attatgcaag gagctttaaa aaagctcatg taaagaagag      8280
   taaaaagaaa aaataattta tttattaatt taatattgag agtgccgaca cagtatgcac      8340
   taaaaaatat atctgtggtg tagtgagccg atacaaaagg atagtcactc gcattttcat      8400
   aatacatctt atgttatgat tatgtgtcgg tgggacttca cgacgaaaac ccacaataaa      8460
   aaaagagttc ggggtagggt taagcatagt tgaggcaact aaacaatcaa gctaggatat      8520
   gcagtagcag accgtaaggt cgttgtttag gtgtgttgta atacatacgc tattaagatg      8580
   taaaaatacg gataccaatg aagggaaaag tataattttt ggatgtagtt tgtttgttca      8640
   tctatgggca aactacgtcc aaagccgttt ccaaatctgc taaaaagtat atcctttcta      8700
   aaatcaaagt caagtatgaa atcataaata aagtttaatt ttgaagttat tatgatatta      8760
   tgtttttcta ttaaaataaa ttaagtatat agaatagttt aataatagta tatacttaat      8820
   gtgataagtg tctgacagtg tcacagaaag gatgattgtt atggattata agcggc          8876
<210> 7
   <211> 8874
   <212> DNA
   <213> Artificial Sequence
<220>
   <223> plasmide
<400> 7
   catggataaa aagtacagta ttggtctaga cataggaact aactctgttg ggtgggctgt      60
   tataacagat gaatataaag ttccatcaaa aaaatttaaa gtattaggaa acactgatag     120
   acattcaata aaaaaaaact tgataggtgc tttattattc gattcaggag agactgctga     180
   agctacacgt ttaaaaagaa cagctagacg tagatataca agaagaaaaa ataggatatg     240
   ttatcttcaa gaaattttta gtaatgaaat ggcaaaagtt gatgattcat tctttcacag     300
   actagaagaa agtttcttag ttgaagaaga taagaagcat gaaagacacc ctatttttgg     360
   taatatcgta gatgaagtag catatcatga gaagtatcca actatctatc atttaagaaa      420
   gaaattagtt gattctacag ataaagctga tctgagatta atatatttag ctttagctca      480
   tatgattaaa tttagaggac attttttaat agaaggtgat ttaaacccag acaacagcga      540
   tgtagataaa ttatttatcc aattagttca aacttataat caattattcg aagagaatcc      600
   aattaatgca agtggtgtag acgctaaggc tatattatca gctagattat caaaatctag      660
   aagattagaa aatctaatag ctcaacttcc tggagaaaag aaaaatggac tttttgggaa      720
   cctaatagct ctctcactcg gactaacacc aaattttaaa agcaattttg atcttgctga      780
   agacgcaaag ttacaactat caaaggatac atacgatgat gatttagata atttgttagc      840
   tcaaataggt gatcaatatg ctgatttgtt tcttgcagca aaaaacttaa gtgatgcaat      900
   tttactatca gatatactta gagtaaatac agaaataaca aaggctcctt tatcagcaag      960
   tatgattaaa cgatatgatg agcatcatca agatttaaca ttattaaagg cacttgtaag     1020
   acaacaatta ccagaaaaat ataaagaaat tttctttgat caatctaaaa atggatatgc     1080
   tggatatata gacggtggag caagtcaaga agagttttat aaatttataa agcctatttt     1140
   agaaaaaatg gatggaactg aagaattact tgttaaactt aacagagaag atttacttag     1200
   aaaacaaaga acttttgata atggttcaat tcctcaccaa attcatttag gagaattaca     1260
   tgctatacta agaagacaag aagattttta tccatttctt aaagataata gagaaaaaat     1320
   tgaaaaaatt ttaactttta gaataccata ttatgtagga ccacttgcaa ggggaaattc     1380
   aagatttgca tggatgacta gaaaatcaga agaaactata accccgtgga attttgaaga     1440
   agtagtagat aaaggagcta gtgctcaatc atttatagaa agaatgacaa attttgataa     1500
   gaatcttcct aacgaaaagg ttttgccaaa gcatagcctt ctttatgagt attttacagt     1560
   ttataatgag cttactaaag taaaatacgt tacagaagga atgagaaaac cagcattttt     1620
   gtctggtgaa caaaagaaag caatagtaga cctattattt aaaacaaata ggaaggttac     1680
   cgtaaagcaa cttaaagaag attacttcaa aaaaattgaa tgctttgata gtgttgaaat     1740
   atcaggagtt gaagatagat ttaatgcttc acttggtaca tatcacgatc tcttaaaaat     1800
   tataaaagat aaggattttt tagataatga agaaaatgaa gatattcttg aagatatagt     1860
   attaacattg acactttttg aagatagaga aatgatagaa gaaagattaa aaacatatgc     1920
   acatcttttt gatgataagg ttatgaagca acttaaaaga agaagatata caggttgggg     1980
   acgtttgtca agaaagctaa ttaatggtat tagagataaa caatcaggaa agactattct     2040
   cgattttctt aaatcagatg gatttgctaa tagaaacttt atgcaattaa ttcatgatga     2100
   ttctcttact ttcaaagagg atattcaaaa ggctcaagtt tctggacaag gcgatagctt     2160
   acacgaacac attgctaacc ttgcagggag ccccgctatc aaaaaaggaa ttttacaaac     2220
   agttaaagtt gtagatgaac ttgttaaagt tatgggaaga cacaaacctg agaatatagt     2280
   tatagaaatg gccagagaaa atcaaacaac acaaaaagga caaaaaaatt ctagagagag 2340
   aatgaagaga attgaagaag gaataaaaga gctaggatca caaatattaa aagaacatcc 2400
   agttgaaaat actcaattgc aaaatgaaaa gttatatttg tattacttac aaaatggaag 2460
   agatatgtat gttgatcaag aactcgatat taatagatta agtgactatg atgttgatca 2520
   tattgttcct caatcatttt taaaagatga ttcaatcgat aacaaagtat taactagatc 2580
   agataaaaat agaggaaagt cagataatgt accatctgaa gaagttgtta aaaaaatgaa 2640
   gaactattgg agacaacttt taaatgcaaa gctaattaca caaagaaaat ttgacaattt 2700
   aacaaaagca gaaagaggag gattaagcga attagacaaa gctggattta taaaaagaca 2760
   acttgttgag acaagacaaa taactaagca tgttgctcaa atacttgatt caagaatgaa 2820
   tacaaaatat gatgaaaatg ataaattaat cagagaagta aaagtaataa cattaaagtc 2880
   aaaattagta tcagatttca gaaaggattt tcaattttac aaagttcgtg aaataaataa 2940
   ctatcatcat gctcatgatg catacttaaa tgctgttgta ggaactgctc ttattaagaa 3000
   atatcctaaa ctagaaagcg aatttgttta tggagattat aaagtttatg atgtgcgcaa 3060
   aatgatcgcg aaatccgaac aagaaatcgg taaggctaca gcaaaatatt tcttttatag 3120
   taatataatg aattttttta agacagaaat aactttggct aatggtgaaa tcagaaaaag 3180
   accacttatc gaaacaaatg gagagacagg agaaatagta tgggataaag gaagagattt 3240
   tgctactgtt agaaaagtac taagtatgcc acaagtaaat atcgtaaaga aaactgaagt 3300
   tcaaactgga ggtttctcta aggaatcaat tttacctaag agaaattcag ataagttaat 3360
   tgcaaggaaa aaagattggg acccaaaaaa atacggtggt tttgatagtc caacagttgc 3420
   ctatagtgtt cttgtagtag cgaaagttga gaaaggtaag tcaaaaaagt tgaaaagcgt 3480
   aaaagaactt cttggtatca caattatgga aagatcttca tttgaaaaaa atccaattga 3540
   ctttttagaa gctaagggtt ataaagaagt taaaaaggat ttaatcataa aactaccaaa 3600
   gtatagtcta tttgaactcg aaaacggaag aaaacgaatg ctcgctagcg caggagaact 3660
   tcaaaaagga aatgaacttg cgctgccatc aaagtatgta aatttcttat atttagcttc 3720
   tcattatgag aaattaaaag gatcaccaga ggataatgaa caaaagcaac tatttgtaga 3780
   acaacacaaa cattatttag atgaaataat agaacaaata tctgaatttt ctaaaagagt 3840
   tatacttgcc gacgcaaatc tagataaggt gctttcagcg tataataaac acagagataa 3900
   accaataaga gaacaagcag aaaacattat ccatcttttt acattaacta atcttggtgc 3960
   accagctgca tttaagtact ttgatacaac aatagataga aaaagataca catctactaa 4020
   agaagtatta gacgcaactt taatacatca atctattaca gggctttatg aaacaagaat 4080
   tgatttaagt caactaggcg gagattaagt cgacaaagta ttgttaaaaa taactctgta 4140
   gaattataaa ttagttctac agagttattt tttgacccgg gtatattgat aaaaataata 4200
   atagtgggta taattaagtt gttaggaggt tagttagaat gatgtcaaga ttagataaaa 4260
   gtaaagtgat taacagcgca ttagagctgc ttaatgaggt cggaatcgaa ggtttaacaa 4320
   cccgtaaact cgcccagaag ctaggtgtag agcagcctac attgtattgg catgtaaaaa 4380
   ataagcgggc tttgctcgac gccttagcca ttgagatgtt agataggcac catactcact 4440
   tttgcccttt agaaggggaa agctggcaag attttttacg taataacgct aaaagtttta 4500
   gatgtgcttt actaagtcat cgcgatggag caaaagtaca tttaggtaca cggcctacag 4560
   aaaaacagta tgaaactctc gaaaatcaat tagccttttt atgccaacaa ggtttttcac 4620
   tagagaatgc attatatgca ctcagcgctg tggggcattt tactttaggt tgcgtattgg 4680
   aagatcaaga gcatcaagtc gctaaagaag aaagggaaac acctactact gatagtatgc 4740
   cgccattatt acgacaagct atcgaattat ttgatcacca aggtgcagag ccagccttct 4800
   tattcggcct tgaattgatc atatgcggat tagaaaaaca acttaaatgt gaaagtgggt 4860
   cttaaaagca gcataacctt tttccgtgat ggtaacttca cggtaaccaa gatgtcgagt 4920
   tgagctcgaa ttcgtaatca tggtcatagc tgtttcctgt gtgaaattgt tatccgctca 4980
   caattccaca caacatacga gccggaagca taaagtgtaa agcctggggt gcctaatgag 5040
   tgagctaact cacattaatt gcgttgcgct cactgcccgc tttccagtcg ggaaacctgt 5100
   cgtgccagct gcattaatga atcggccaac gcgcggggag aggcggtttg cgtattgggc 5160
   gctcttccgc ttcctcgctc actgactcgc tgcgctcggt cgttcggctg cggcgagcgg 5220
   tatcagctca ctcaaaggcg gtaatacggt tatccacaga atcaggggat aacgcaggaa 5280
   agaacatgtg agcaaaaggc cagcaaaagg ccaggaaccg taaaaaggcc gcgttgctgg 5340
   cgtttttcca taggctccgc ccccctgacg agcatcacaa aaatcgacgc tcaagtcaga 5400
   ggtggcgaaa cccgacagga ctataaagat accaggcgtt tccccctgga agctccctcg 5460
   tgcgctctcc tgttccgacc ctgccgctta ccggatacct gtccgccttt ctcccttcgg 5520
   gaagcgtggc gctttctcat agctcacgct gtaggtatct cagttcggtg taggtcgttc 5580
   gctccaagct gggctgtgtg cacgaacccc ccgttcagcc cgaccgctgc gccttatccg 5640
   gtaactatcg tcttgagtcc aacccggtaa gacacgactt atcgccactg gcagcagcca 5700
   ctggtaacag gattagcaga gcgaggtatg taggcggtgc tacagagttc ttgaagtggt 5760
   ggcctaacta cggctacact agaaggacag tatttggtat ctgcgctctg ctgaagccag 5820
   ttaccttcgg aaaaagagtt ggtagctctt gatccggcaa acaaaccacc gctggtagcg 5880
   gtggtttttt tgtttgcaag cagcagatta cgcgcagaaa aaaaggatct caagaagatc 5940
   ctttgatctt ttctacgggg tctgacgctc agtggaacga aaactcacgt taagggattt 6000
   tggtcatgag attatcaaaa aggatcttca cctagatcct tttaaattaa aaatgaagtt 6060
   ttaaatcaat ctaaagtata tatgagtaaa cttggtctga cagttaccag gtccactgcc 6120
   gggcctcttg cgggatcaaa agaaaaacga aatgatacac caatcagtgc aaaaaaagat 6180
   ataatgggag ataagacggt tcgtgttcgt gctgacttgc accatatcat aaaaatcgaa 6240
   acagcaaaga atggcggaaa cgtaaaagaa gttatggaaa taagacttag aagcaaactt 6300
   aagagtgtgt tgatagtgca gtatcttaaa attttgtata ataggaattg aagttaaatt 6360
   agatgctaaa aatttgtaat taagaaggag tgattacatg aacaaaaata taaaatattc 6420
   tcaaaacttt ttaacgagtg aaaaagtact caaccaaata ataaaacaat tgaatttaaa 6480
   agaaaccgat accgtttacg aaattggaac aggtaaaggg catttaacga cgaaactggc 6540
   taaaataagt aaacaggtaa cgtctattga attagacagt catctattca acttatcgtc 6600
   agaaaaatta aaactgaata ctcgtgtcac tttaattcac caagatattc tacagtttca 6660
   attccctaac aaacagaggt ataaaattgt tgggagtatt ccttaccatt taagcacaca 6720
   aattattaaa aaagtggttt ttgaaagcca tgcgtctgac atctatctga ttgttgaaga 6780
   aggattctac aagcgtacct tggatattca ccgaacacta gggttgctct tgcacactca 6840
   agtctcgatt cagcaattgc ttaagctgcc agcggaatgc tttcatccta aaccaaaagt 6900
   aaacagtgtc ttaataaaac ttacccgcca taccacagat gttccagata aatattggaa 6960
   gctatatacg tactttgttt caaaatgggt caatcgagaa tatcgtcaac tgtttactaa 7020
   aaatcagttt catcaagcaa tgaaacacgc caaagtaaac aatttaagta ccgttactta 7080
   tgagcaagta ttgtctattt ttaatagtta tctattattt aacgggagga aataattcta 7140
   tgagtcccta ggcaggcctc cgccattatt tttttgaaca attgacaatt catttcttat 7200
   tttttattaa gtgatagtca aaaggcataa cagtgctgaa tagaaagaaa tttacagaaa 7260
   agaaaattat agaatttagt atgattaatt atactcattt atgaatgttt aattgaatac 7320
   aaaaaaaaat acttgttatg tattcaatta cgggttaaaa tatagacaag ttgaaaaatt 7380
   taataaaaaa ataagtcctc agctcttata tattaagcta ccaacttagt atataagcca 7440
   aaacttaaat gtgctaccaa cacatcaagc cgttagagaa ctctatctat agcaatattt 7500
   caaatgtacc gacatacaag agaaacatta actatatata ttcaatttat gagattatct 7560
   taacagatat aaatgtaaat tgcaataagt aagatttaga agtttatagc ctttgtgtat 7620
   tggaagcagt acgcaaaggc ttttttattt gataaaaatt agaagtatat ttattttttc 7680
   ataattaatt tatgaaaatg aaagggggtg agcaaagtga cagaggaaag cagtatctta 7740
   tcaaataaca aggtattagc aatatcatta ttgactttag cagtaaacat tatgactttt 7800
   atagtgcttg tagctaagta gtacgaaagg gggagcttta aaaagctcct tggaatacat 7860
   agaattcata aattaattta tgaaaagaag ggcgtatatg aaaacttgta aaaattgcaa 7920
   agagtttatt aaagatactg aaatatgcaa aatacattcg ttgatgattc atgataaaac 7980
   agtagcaacc tattgcagta aatacaatga gtcaagatgt ttacataaag ggaaagtcca 8040
   atgtattaat tgttcaaaga tgaaccgata tggatggtgt gccataaaaa tgagatgttt 8100
   tacagaggaa gaacagaaaa aagaacgtac atgcattaaa tattatgcaa ggagctttaa 8160
   aaaagctcat gtaaagaaga gtaaaaagaa aaaataattt atttattaat ttaatattga 8220
   gagtgccgac acagtatgca ctaaaaaata tatctgtggt gtagtgagcc gatacaaaag 8280
   gatagtcact cgcattttca taatacatct tatgttatga ttatgtgtcg gtgggacttc 8340
   acgacgaaaa cccacaataa aaaaagagtt cggggtaggg ttaagcatag ttgaggcaac 8400
   taaacaatca agctaggata tgcagtagca gaccgtaagg tcgttgttta ggtgtgttgt 8460
   aatacatacg ctattaagat gtaaaaatac ggataccaat gaagggaaaa gtataatttt 8520
   tggatgtagt ttgtttgttc atctatgggc aaactacgtc caaagccgtt tccaaatctg 8580
   ctaaaaagta tatcctttct aaaatcaaag tcaagtatga aatcataaat aaagtttaat 8640
   tttgaagtta ttatgatatt atgtttttct attaaaataa attaagtata tagaatagtt 8700
   taataatagt atatacttaa tgtgataagt gtctgacagt gtcacagaaa ggatgattgt 8760
   tatggattat aagcggctcg agtccctatc agtgatagat tgaaactcta tcattgatag 8820
   agtataatat ctttgttcat tagagcgata aacttgaatt tgagagggaa cttc 8874
<210> 8
   <211> 4938
   <212> DNA
   <213> Artificial Sequence
<220>
   <223> plasmide
<400> 8
   tcgactctag aggatccccg ggtaccgagc tcgaattcgt aatcatggtc atagctgttt      60
   cctgtgtgaa attgttatcc gctcacaatt ccacacaaca tacgagccgg aagcataaag     120
   tgtaaagcct ggggtgccta atgagtgagc taactcacat taattgcgtt gcgctcactg     180
   cccgctttcc agtcgggaaa cctgtcgtgc cagctgcatt aatgaatcgg ccaacgcgcg     240
   gggagaggcg gtttgcgtat tgggcgctct tccgcttcct cgctcactga ctcgctgcgc     300
   tcggtcgttc ggctgcggcg agcggtatca gctcactcaa aggcggtaat acggttatcc     360
   acagaatcag gggataacgc aggaaagaac atgtgagcaa aaggccagca aaaggccagg     420
   aaccgtaaaa aggccgcgtt gctggcgttt ttccataggc tccgcccccc tgacgagcat     480
   cacaaaaatc gacgctcaag tcagaggtgg cgaaacccga caggactata aagataccag     540
   gcgtttcccc ctggaagctc cctcgtgcgc tctcctgttc cgaccctgcc gcttaccgga     600
   tacctgtccg cctttctccc ttcgggaagc gtggcgcttt ctcatagctc acgctgtagg      660
   tatctcagtt cggtgtaggt cgttcgctcc aagctgggct gtgtgcacga accccccgtt      720
   cagcccgacc gctgcgcctt atccggtaac tatcgtcttg agtccaaccc ggtaagacac 780
   gacttatcgc cactggcagc agccactggt aacaggatta gcagagcgag gtatgtaggc 840
   ggtgctacag agttcttgaa gtggtggcct aactacggct acactagaag aacagtattt 900
   ggtatctgcg ctctgctgaa gccagttacc ttcggaaaaa gagttggtag ctcttgatcc 960
   ggcaaacaaa ccaccgctgg tagcggtggt ttttttgttt gcaagcagca gattacgcgc 1020
   agaaaaaaag gatctcaaga agatcctttg atcttttcta cggggtctga cgctcagtgg 1080
   aacgaaaact cacgttaagg gattttggtc atgagattat caaaaaggat cttcacctag 1140
   atccttttaa attaaaaatg aagttttaaa tcaatctaaa gtatatatga gtaaacttgg 1200
   tctgacagtt accaaagcta gcttaatact agtatatact taatgtgata agtgtctgac 1260
   agctgaccgg tctaaagagg tccctagcgc ctacggggaa tttgtatcga taaggggtac 1320
   aaattcccac taagcgctcg gccggggatc gatccccggg tacgtacccg gcagtttttc 1380
   tttttcggca agtgttcaag aagttattaa gtcgggagtg cagtcgaagt gggcaagttg 1440
   aaaaattcac aaaaatgtgg tataatatct ttgttcatta gagcgataaa cttgaatttg 1500
   agagggaact tagatggtat ttgaaaaaat tgataaaaat agttggaaca gaaaagagta 1560
   ttttgaccac tactttgcaa gtgtaccttg tacctacagc atgaccgtta aagtggatat 1620
   cacacaaata aaggaaaagg gaatgaaact atatcctgca atgctttatt atattgcaat 1680
   gattgtaaac cgccattcag agtttaggac ggcaatcaat caagatggtg aattggggat 1740
   atatgatgag atgataccaa gctatacaat atttcacaat gatactgaaa cattttccag 1800
   cctttggact gagtgtaagt ctgactttaa atcattttta gcagattatg aaagtgatac 1860
   gcaacggtat ggaaacaatc atagaatgga aggaaagcca aatgctccgg aaaacatttt 1920
   taatgtatct atgataccgt ggtcaacctt cgatggcttt aatctgaatt tgcagaaagg 1980
   atatgattat ttgattccta tttttactat ggggaaatat tataaagaag ataacaaaat 2040
   tatacttcct ttggcaattc aagttcatca cgcagtatgt gacggatttc acatttgccg 2100
   ttttgtaaac gaattgcagg aattgataaa tagttaactt caggtttgtc tgtaactaaa 2160
   aactagtatt taacctagga tcaaaaaaat ttccaataat cccactctaa gccacaaaca 2220
   cgccctataa aatcccgctt taatcccact ttgagacaca tgtaatatta ctttacgccc 2280
   tagtatagtg ataatttttt acattcaatg ccacgcaaaa aaataaaggg gcactataat 2340
   aaaagttcct tcggaactaa ctaaagtaaa aaattatctt tacaacctcc ccaaaaaaaa 2400
   gaacaggtac aaagtaccct ataatacaag cgtaaaaaaa atgagggtaa aaataaaaaa 2460
   ataaaaaaat aaaaaaataa aaaaataaaa aaataaaaaa ataaaaaaat ataaaaataa 2520
   aaaaatataa aaataaaaaa atataaaaat aaaaaaataa aaaaatataa aaataaaaaa 2580
   ataaaaaaat ataaaaatat tttttattta aagtttgaaa aaaatttttt tatattatat 2640
   aatctttgaa gaaaagaata taaaaaatga gcctttataa aagcccattt tttttcatat 2700
   acgtaatatg acgttctaat gtttttattg gtacttctaa cattagagta atttctttat 2760
   ttttaaagcc tttttcttta agggctttta ttttttttct taatacattt aattcctctt 2820
   tttttgttgc ttttccttta gcttttaatt gctcttgata atttttttta cctctaatat 2880
   tttctcttct cttatattcc tttttagaaa ttattattgt catatatttt tgttcttctt 2940
   ctgtaatttc taataactct ataagagttt cattcttata cttatattgc ttatttttat 3000
   ctaaataaca tctttcagca cttctagttg ctcttataac ttctctttca cttaaatgtt 3060
   gtctaaacat actattaagt tctaaaacat catttaatgc cttctcaatg tcttctgtaa 3120
   agctacaaag ataatatcta tataaaaata atataagctc tctgtgtcct tttaaatcat 3180
   attctcttag ttcacaaagt tttattatgt cttgtattct tccataatat aaacttcttt 3240
   ctctataaat ataatttatt ttgcttggtc tacccttttt cctttcatat ggttttaatt 3300
   caggtaaaaa tccattttgt atttctctta agtcataaat atattcgtac tcatctaata 3360
   tattgactac tgtttttgat ttagagttta tacttcctgg aactcttaat attctcgttg 3420
   catctaaggc ttgtctatct gctccaaagt attttaattg attatataaa tattcttgaa 3480
   ccgctttcca taatggtaat gctttactag gtactgcatt tattatccat attaaataca 3540
   ttcctcttcc actatctatt acatagtttg gtataggaat actttgatta aaataattct 3600
   tttctaagtc cattaatacc tggtctttag ttttgccagt tttataataa tccaagtcta 3660
   taaacagtgt atttaactct tttatatttt ctaatcgcct acacggctta taaaaggtat 3720
   ttagagttat atagatattt tcatcactca tatctaaatc ttttaattca gcgtatttat 3780
   agtgccattg gctatatcct tttttatcta taacgctcct ggttatccac cctttacttc 3840
   tactatgaat attatctata tagttctttt tattcagctt taatgcgttt ctcacttatt 3900
   cacctcccct tctgtaaaac taagaaaatt atatcatatt ttcaataatt attaactatt 3960
   cttaaactct taataaaaaa tagagtaagt ccccaattga aacttaatct attttttatg 4020
   ttttaattta ttatttttat taaaatattt taaactaaat taaatgattc tttttaattt 4080
   tttactattt cattccataa tatattacta taattattta caaataatat ttcttcattt 4140
   gtaatattta gatgatttac taattttagt ttttatatat taaataatta atgtataatt 4200
   tatataaaaa atcaaaggag cttataaatt atgattattt ccaaagatac taaagattta 4260
   atttttttca attttaacaa tactttttgt aatattatgt ttaaatttaa ttgtattttt 4320
   ttcatataat aaagccgttg aagtaaacca atccattttc cttatgatgt tattattaaa 4380
   tttaagtttt ataataatat ctttattata tttattgttt ttaaaaaaac tagtgaaatt 4440
   tctagtgaaa tttccggctt tattaaactt atttttagga attttatttt cattttcatc 4500
   tttacaggat ttgattatat ctttaaatat gttttatcaa atattatctt tttctaaatt 4560
   tatatatatt tttattatat ttattattat atatatttta tttttaagtt tctttctaac 4620
   agctattaaa aagaaactta aaaataaaaa cacgtactct aaaccaataa ataaaactat 4680
   ttttattatt gctgccttga ttggaatagt ttttagtaaa attaatttca atattccaca 4740
   atattatatt ataagctagc acgcctcgag tatattgata aaaataataa tagtgggtat 4800
   aattaagttg ttaggaggtt agttagactt aaaatccata tataacagtt ttagagctag 4860
   aaatagcaag ttaaaataag gctagtccgt tatcaacttg aaaaagtggc accgagtcgg 4920
   tgcttttttt gaagcttg 4938
<210> 9
   <211> 4938
   <212> DNA
   <213> Artificial Sequence
<220>
   <223> plasmide
<400> 9
   tcgactctag aggatccccg ggtaccgagc tcgaattcgt aatcatggtc atagctgttt      60
   cctgtgtgaa attgttatcc gctcacaatt ccacacaaca tacgagccgg aagcataaag     120
   tgtaaagcct ggggtgccta atgagtgagc taactcacat taattgcgtt gcgctcactg     180
   cccgctttcc agtcgggaaa cctgtcgtgc cagctgcatt aatgaatcgg ccaacgcgcg     240
   gggagaggcg gtttgcgtat tgggcgctct tccgcttcct cgctcactga ctcgctgcgc     300
   tcggtcgttc ggctgcggcg agcggtatca gctcactcaa aggcggtaat acggttatcc     360
   acagaatcag gggataacgc aggaaagaac atgtgagcaa aaggccagca aaaggccagg     420
   aaccgtaaaa aggccgcgtt gctggcgttt ttccataggc tccgcccccc tgacgagcat     480
   cacaaaaatc gacgctcaag tcagaggtgg cgaaacccga caggactata aagataccag     540
   gcgtttcccc ctggaagctc cctcgtgcgc tctcctgttc cgaccctgcc gcttaccgga     600
   tacctgtccg cctttctccc ttcgggaagc gtggcgcttt ctcatagctc acgctgtagg     660
   tatctcagtt cggtgtaggt cgttcgctcc aagctgggct gtgtgcacga accccccgtt     720
   cagcccgacc gctgcgcctt atccggtaac tatcgtcttg agtccaaccc ggtaagacac     780
   gacttatcgc cactggcagc agccactggt aacaggatta gcagagcgag gtatgtaggc     840
   ggtgctacag agttcttgaa gtggtggcct aactacggct acactagaag aacagtattt     900
   ggtatctgcg ctctgctgaa gccagttacc ttcggaaaaa gagttggtag ctcttgatcc     960
   ggcaaacaaa ccaccgctgg tagcggtggt ttttttgttt gcaagcagca gattacgcgc    1020
   agaaaaaaag gatctcaaga agatcctttg atcttttcta cggggtctga cgctcagtgg 1080
   aacgaaaact cacgttaagg gattttggtc atgagattat caaaaaggat cttcacctag 1140
   atccttttaa attaaaaatg aagttttaaa tcaatctaaa gtatatatga gtaaacttgg 1200
   tctgacagtt accaaagcta gcttaatact agtatatact taatgtgata agtgtctgac 1260
   agctgaccgg tctaaagagg tccctagcgc ctacggggaa tttgtatcga taaggggtac 1320
   aaattcccac taagcgctcg gccggggatc gatccccggg tacgtacccg gcagtttttc 1380
   tttttcggca agtgttcaag aagttattaa gtcgggagtg cagtcgaagt gggcaagttg 1440
   aaaaattcac aaaaatgtgg tataatatct ttgttcatta gagcgataaa cttgaatttg 1500
   agagggaact tagatggtat ttgaaaaaat tgataaaaat agttggaaca gaaaagagta 1560
   ttttgaccac tactttgcaa gtgtaccttg tacctacagc atgaccgtta aagtggatat 1620
   cacacaaata aaggaaaagg gaatgaaact atatcctgca atgctttatt atattgcaat 1680
   gattgtaaac cgccattcag agtttaggac ggcaatcaat caagatggtg aattggggat 1740
   atatgatgag atgataccaa gctatacaat atttcacaat gatactgaaa cattttccag 1800
   cctttggact gagtgtaagt ctgactttaa atcattttta gcagattatg aaagtgatac 1860
   gcaacggtat ggaaacaatc atagaatgga aggaaagcca aatgctccgg aaaacatttt 1920
   taatgtatct atgataccgt ggtcaacctt cgatggcttt aatctgaatt tgcagaaagg 1980
   atatgattat ttgattccta tttttactat ggggaaatat tataaagaag ataacaaaat 2040
   tatacttcct ttggcaattc aagttcatca cgcagtatgt gacggatttc acatttgccg 2100
   ttttgtaaac gaattgcagg aattgataaa tagttaactt caggtttgtc tgtaactaaa 2160
   aactagtatt taacctagga tcaaaaaaat ttccaataat cccactctaa gccacaaaca 2220
   cgccctataa aatcccgctt taatcccact ttgagacaca tgtaatatta ctttacgccc 2280
   tagtatagtg ataatttttt acattcaatg ccacgcaaaa aaataaaggg gcactataat 2340
   aaaagttcct tcggaactaa ctaaagtaaa aaattatctt tacaacctcc ccaaaaaaaa 2400
   gaacaggtac aaagtaccct ataatacaag cgtaaaaaaa atgagggtaa aaataaaaaa 2460
   ataaaaaaat aaaaaaataa aaaaataaaa aaataaaaaa ataaaaaaat ataaaaataa 2520
   aaaaatataa aaataaaaaa atataaaaat aaaaaaataa aaaaatataa aaataaaaaa 2580
   ataaaaaaat ataaaaatat tttttattta aagtttgaaa aaaatttttt tatattatat 2640
   aatctttgaa gaaaagaata taaaaaatga gcctttataa aagcccattt tttttcatat 2700
   acgtaatatg acgttctaat gtttttattg gtacttctaa cattagagta atttctttat 2760
   ttttaaagcc tttttcttta agggctttta ttttttttct taatacattt aattcctctt 2820
   tttttgttgc ttttccttta gcttttaatt gctcttgata atttttttta cctctaatat 2880
   tttctcttct cttatattcc tttttagaaa ttattattgt catatatttt tgttcttctt 2940
   ctgtaatttc taataactct ataagagttt cattcttata cttatattgc ttatttttat 3000
   ctaaataaca tctttcagca cttctagttg ctcttataac ttctctttca cttaaatgtt 3060
   gtctaaacat actattaagt tctaaaacat catttaatgc cttctcaatg tcttctgtaa 3120
   agctacaaag ataatatcta tataaaaata atataagctc tctgtgtcct tttaaatcat 3180
   attctcttag ttcacaaagt tttattatgt cttgtattct tccataatat aaacttcttt 3240
   ctctataaat ataatttatt ttgcttggtc tacccttttt cctttcatat ggttttaatt 3300
   caggtaaaaa tccattttgt atttctctta agtcataaat atattcgtac tcatctaata 3360
   tattgactac tgtttttgat ttagagttta tacttcctgg aactcttaat attctcgttg 3420
   catctaaggc ttgtctatct gctccaaagt attttaattg attatataaa tattcttgaa 3480
   ccgctttcca taatggtaat gctttactag gtactgcatt tattatccat attaaataca 3540
   ttcctcttcc actatctatt acatagtttg gtataggaat actttgatta aaataattct 3600
   tttctaagtc cattaatacc tggtctttag ttttgccagt tttataataa tccaagtcta 3660
   taaacagtgt atttaactct tttatatttt ctaatcgcct acacggctta taaaaggtat 3720
   ttagagttat atagatattt tcatcactca tatctaaatc ttttaattca gcgtatttat 3780
   agtgccattg gctatatcct tttttatcta taacgctcct ggttatccac cctttacttc 3840
   tactatgaat attatctata tagttctttt tattcagctt taatgcgttt ctcacttatt 3900
   cacctcccct tctgtaaaac taagaaaatt atatcatatt ttcaataatt attaactatt 3960
   cttaaactct taataaaaaa tagagtaagt ccccaattga aacttaatct attttttatg 4020
   ttttaattta ttatttttat taaaatattt taaactaaat taaatgattc tttttaattt 4080
   tttactattt cattccataa tatattacta taattattta caaataatat ttcttcattt 4140
   gtaatattta gatgatttac taattttagt ttttatatat taaataatta atgtataatt 4200
   tatataaaaa atcaaaggag cttataaatt atgattattt ccaaagatac taaagattta 4260
   atttttttca attttaacaa tactttttgt aatattatgt ttaaatttaa ttgtattttt 4320
   ttcatataat aaagccgttg aagtaaacca atccattttc cttatgatgt tattattaaa 4380
   tttaagtttt ataataatat ctttattata tttattgttt ttaaaaaaac tagtgaaatt 4440
   tctagtgaaa tttccggctt tattaaactt atttttagga attttatttt cattttcatc 4500
   tttacaggat ttgattatat ctttaaatat gttttatcaa atattatctt tttctaaatt 4560
   tatatatatt tttattatat ttattattat atatatttta tttttaagtt tctttctaac 4620
   agctattaaa aagaaactta aaaataaaaa cacgtactct aaaccaataa ataaaactat 4680
   ttttattatt gctgccttga ttggaatagt ttttagtaaa attaatttca atattccaca 4740
   atattatatt ataagctagc acgcctcgag tatattgata aaaataataa tagtgggtat 4800
   aattaagttg ttaggaggtt agttagaact aaatgtaaaa tgttagcgtt ttagagctag 4860
   aaatagcaag ttaaaataag gctagtccgt tatcaacttg aaaaagtggc accgagtcgg 4920
   tgcttttttt gaagcttg 4938
<210> 10
   <211> 5911
   <212> DNA
   <213> Artificial Sequence
<220>
   <223> plasmide
<400> 10
   aattcgtaat catggtcata gctgtttcct gtgtgaaatt gttatccgct cacaattcca      60
   cacaacatac gagccggaag cataaagtgt aaagcctggg gtgcctaatg agtgagctaa     120
   ctcacattaa ttgcgttgcg ctcactgccc gctttccagt cgggaaacct gtcgtgccag     180
   ctgcattaat gaatcggcca acgcgcgggg agaggcggtt tgcgtattgg gcgctcttcc     240
   gcttcctcgc tcactgactc gctgcgctcg gtcgttcggc tgcggcgagc ggtatcagct     300
   cactcaaagg cggtaatacg gttatccaca gaatcagggg ataacgcagg aaagaacatg     360
   tgagcaaaag gccagcaaaa ggccaggaac cgtaaaaagg ccgcgttgct ggcgtttttc     420
   cataggctcc gcccccctga cgagcatcac aaaaatcgac gctcaagtca gaggtggcga     480
   aacccgacag gactataaag ataccaggcg tttccccctg gaagctccct cgtgcgctct     540
   cctgttccga ccctgccgct taccggatac ctgtccgcct ttctcccttc gggaagcgtg     600
   gcgctttctc atagctcacg ctgtaggtat ctcagttcgg tgtaggtcgt tcgctccaag     660
   ctgggctgtg tgcacgaacc ccccgttcag cccgaccgct gcgccttatc cggtaactat     720
   cgtcttgagt ccaacccggt aagacacgac ttatcgccac tggcagcagc cactggtaac     780
   aggattagca gagcgaggta tgtaggcggt gctacagagt tcttgaagtg gtggcctaac     840
   tacggctaca ctagaagaac agtatttggt atctgcgctc tgctgaagcc agttaccttc     900
   ggaaaaagag ttggtagctc ttgatccggc aaacaaacca ccgctggtag cggtggtttt     960
   tttgtttgca agcagcagat tacgcgcaga aaaaaaggat ctcaagaaga tcctttgatc    1020
   ttttctacgg ggtctgacgc tcagtggaac gaaaactcac gttaagggat tttggtcatg    1080
   agattatcaa aaaggatctt cacctagatc cttttaaatt aaaaatgaag ttttaaatca    1140
   atctaaagta tatatgagta aacttggtct gacagttacc aaagctagct taatactagt    1200
   atatacttaa tgtgataagt gtctgacagc tgaccggtct aaagaggtcc ctagcgccta    1260
   cggggaattt gtatcgataa ggggtacaaa ttcccactaa gcgctcggcc ggggatcgat    1320
   ccccgggtac gtacccggca gtttttcttt ttcggcaagt gttcaagaag ttattaagtc    1380
   gggagtgcag tcgaagtggg caagttgaaa aattcacaaa aatgtggtat aatatctttg    1440
   ttcattagag cgataaactt gaatttgaga gggaacttag atggtatttg aaaaaattga 1500
   taaaaatagt tggaacagaa aagagtattt tgaccactac tttgcaagtg taccttgtac 1560
   ctacagcatg accgttaaag tggatatcac acaaataaag gaaaagggaa tgaaactata 1620
   tcctgcaatg ctttattata ttgcaatgat tgtaaaccgc cattcagagt ttaggacggc 1680
   aatcaatcaa gatggtgaat tggggatata tgatgagatg ataccaagct atacaatatt 1740
   tcacaatgat actgaaacat tttccagcct ttggactgag tgtaagtctg actttaaatc 1800
   atttttagca gattatgaaa gtgatacgca acggtatgga aacaatcata gaatggaagg 1860
   aaagccaaat gctccggaaa acatttttaa tgtatctatg ataccgtggt caaccttcga 1920
   tggctttaat ctgaatttgc agaaaggata tgattatttg attcctattt ttactatggg 1980
   gaaatattat aaagaagata acaaaattat acttcctttg gcaattcaag ttcatcacgc 2040
   agtatgtgac ggatttcaca tttgccgttt tgtaaacgaa ttgcaggaat tgataaatag 2100
   ttaacttcag gtttgtctgt aactaaaaac tagtatttaa cctaggatca aaaaaatttc 2160
   caataatccc actctaagcc acaaacacgc cctataaaat cccgctttaa tcccactttg 2220
   agacacatgt aatattactt tacgccctag tatagtgata attttttaca ttcaatgcca 2280
   cgcaaaaaaa taaaggggca ctataataaa agttccttcg gaactaacta aagtaaaaaa 2340
   ttatctttac aacctcccca aaaaaaagaa caggtacaaa gtaccctata atacaagcgt 2400
   aaaaaaaatg agggtaaaaa taaaaaaata aaaaaataaa aaaataaaaa aataaaaaaa 2460
   taaaaaaata aaaaaatata aaaataaaaa aatataaaaa taaaaaaata taaaaataaa 2520
   aaaataaaaa aatataaaaa taaaaaaata aaaaaatata aaaatatttt ttatttaaag 2580
   tttgaaaaaa atttttttat attatataat ctttgaagaa aagaatataa aaaatgagcc 2640
   tttataaaag cccatttttt ttcatatacg taatatgacg ttctaatgtt tttattggta 2700
   cttctaacat tagagtaatt tctttatttt taaagccttt ttctttaagg gcttttattt 2760
   tttttcttaa tacatttaat tcctcttttt ttgttgcttt tcctttagct tttaattgct 2820
   cttgataatt ttttttacct ctaatatttt ctcttctctt atattccttt ttagaaatta 2880
   ttattgtcat atatttttgt tcttcttctg taatttctaa taactctata agagtttcat 2940
   tcttatactt atattgctta tttttatcta aataacatct ttcagcactt ctagttgctc 3000
   ttataacttc tctttcactt aaatgttgtc taaacatact attaagttct aaaacatcat 3060
   ttaatgcctt ctcaatgtct tctgtaaagc tacaaagata atatctatat aaaaataata 3120
   taagctctct gtgtcctttt aaatcatatt ctcttagttc acaaagtttt attatgtctt 3180
   gtattcttcc ataatataaa cttctttctc tataaatata atttattttg cttggtctac 3240
   cctttttcct ttcatatggt tttaattcag gtaaaaatcc attttgtatt tctcttaagt 3300
   cataaatata ttcgtactca tctaatatat tgactactgt ttttgattta gagtttatac 3360
   ttcctggaac tcttaatatt ctcgttgcat ctaaggcttg tctatctgct ccaaagtatt 3420
   ttaattgatt atataaatat tcttgaaccg ctttccataa tggtaatgct ttactaggta 3480
   ctgcatttat tatccatatt aaatacattc ctcttccact atctattaca tagtttggta 3540
   taggaatact ttgattaaaa taattctttt ctaagtccat taatacctgg tctttagttt 3600
   tgccagtttt ataataatcc aagtctataa acagtgtatt taactctttt atattttcta 3660
   atcgcctaca cggcttataa aaggtattta gagttatata gatattttca tcactcatat 3720
   ctaaatcttt taattcagcg tatttatagt gccattggct atatcctttt ttatctataa 3780
   cgctcctggt tatccaccct ttacttctac tatgaatatt atctatatag ttctttttat 3840
   tcagctttaa tgcgtttctc acttattcac ctccccttct gtaaaactaa gaaaattata 3900
   tcatattttc aataattatt aactattctt aaactcttaa taaaaaatag agtaagtccc 3960
   caattgaaac ttaatctatt ttttatgttt taatttatta tttttattaa aatattttaa 4020
   actaaattaa atgattcttt ttaatttttt actatttcat tccataatat attactataa 4080
   ttatttacaa ataatatttc ttcatttgta atatttagat gatttactaa ttttagtttt 4140
   tatatattaa ataattaatg tataatttat ataaaaaatc aaaggagctt ataaattatg 4200
   attatttcca aagatactaa agatttaatt tttttcaatt ttaacaatac tttttgtaat 4260
   attatgttta aatttaattg tatttttttc atataataaa gccgttgaag taaaccaatc 4320
   cattttcctt atgatgttat tattaaattt aagttttata ataatatctt tattatattt 4380
   attgttttta aaaaaactag tgaaatttct agtgaaattt ccggctttat taaacttatt 4440
   tttaggaatt ttattttcat tttcatcttt acaggatttg attatatctt taaatatgtt 4500
   ttatcaaata ttatcttttt ctaaatttat atatattttt attatattta ttattatata 4560
   tattttattt ttaagtttct ttctaacagc tattaaaaag aaacttaaaa ataaaaacac 4620
   gtactctaaa ccaataaata aaactatttt tattattgct gccttgattg gaatagtttt 4680
   tagtaaaatt aatttcaata ttccacaata ttatattata agctagcacg cctcgagtat 4740
   attgataaaa ataataatag tgggtataat taagttgtta ggaggttagt tagaactaaa 4800
   tgtaaaatgt tagcgtttta gagctagaaa tagcaagtta aaataaggct agtccgttat 4860
   caacttgaaa aagtggcacc gagtcggtgc tttttttgaa gcttgtcgac atgaagatag 4920
   caataggtag tgatcatgca ggattttcat tgaaaaagga agttataaaa catttagaga 4980
   gtaaaaatat tgaggttaaa gattttggca ctctaactga tgaatcatgt gattatccag 5040
   attatgcatt aaaagtagca gaggaagttg ctcaaaaaaa ctttgagttt ggaatactca 5100
   tttgtggaac aggtatagga ataagcatat cagcaaataa ggtgccagga ataagagcag 5160
   ctgtatgtac agatacattc tgtgctcatg catcaagaga acataacaat gcaaatatac 5220
   ttgcaatggg agaaagagtt gtaggacctg gattagcaat tgatatagta gatacatttt 5280
   taaattcaaa atttcaggga gataggcatc aaagaagaat agacaagatt acacaaatag 5340
   aaaaaaaata caatggagga atgaaataat gagtaaagtt acacaaatat cacatccact 5400
   tatattacac aaacttcctc aagatataga ggaaagagac ataatagtaa ctgatccaat 5460
   gcttgcaact ggtgggtcag caatagatgc aataacactt cttaagaaaa gaggagcaaa 5520
   atacataaga cttatgtgtc ttataggagc accagaaggt atagcagcag tacaagaagc 5580
   acatccagat gtagatatat acctcgcatc aatagatgaa aagttagatg aaaatggata 5640
   tatagttcct ggtcttggag atgctggaga tagattattc ggtacaaaat aaattgcata 5700
   aataaaaagg gctgaaaaat aaatttcagt ccttttattt atattttaac tttattccat 5760
   gccactgcct cttctgataa taatagaaat tattaaagtt aatacagatg taagggcaac 5820
   tgttccacct ggagcacaat ttaagtaata agagctaaat aatccaacga gtacatctat 5880
   aaagctaaat aatattgata atataagcgt g 5911
<210> 11
   <211> 6217
   <212> DNA
   <213> Artificial Sequence
<220>
   <223> plasmide
<400> 11
   aattcgtaat catggtcata gctgtttcct gtgtgaaatt gttatccgct cacaattcca      60
   cacaacatac gagccggaag cataaagtgt aaagcctggg gtgcctaatg agtgagctaa     120
   ctcacattaa ttgcgttgcg ctcactgccc gctttccagt cgggaaacct gtcgtgccag     180
   ctgcattaat gaatcggcca acgcgcgggg agaggcggtt tgcgtattgg gcgctcttcc     240
   gcttcctcgc tcactgactc gctgcgctcg gtcgttcggc tgcggcgagc ggtatcagct     300
   cactcaaagg cggtaatacg gttatccaca gaatcagggg ataacgcagg aaagaacatg     360
   tgagcaaaag gccagcaaaa ggccaggaac cgtaaaaagg ccgcgttgct ggcgtttttc     420
   cataggctcc gcccccctga cgagcatcac aaaaatcgac gctcaagtca gaggtggcga     480
   aacccgacag gactataaag ataccaggcg tttccccctg gaagctccct cgtgcgctct     540
   cctgttccga ccctgccgct taccggatac ctgtccgcct ttctcccttc gggaagcgtg     600
   gcgctttctc atagctcacg ctgtaggtat ctcagttcgg tgtaggtcgt tcgctccaag     660
   ctgggctgtg tgcacgaacc ccccgttcag cccgaccgct gcgccttatc cggtaactat     720
   cgtcttgagt ccaacccggt aagacacgac ttatcgccac tggcagcagc cactggtaac     780
   aggattagca gagcgaggta tgtaggcggt gctacagagt tcttgaagtg gtggcctaac     840
   tacggctaca ctagaagaac agtatttggt atctgcgctc tgctgaagcc agttaccttc 900
   ggaaaaagag ttggtagctc ttgatccggc aaacaaacca ccgctggtag cggtggtttt 960
   tttgtttgca agcagcagat tacgcgcaga aaaaaaggat ctcaagaaga tcctttgatc 1020
   ttttctacgg ggtctgacgc tcagtggaac gaaaactcac gttaagggat tttggtcatg 1080
   agattatcaa aaaggatctt cacctagatc cttttaaatt aaaaatgaag ttttaaatca 1140
   atctaaagta tatatgagta aacttggtct gacagttacc aaagctagct taatactagt 1200
   atatacttaa tgtgataagt gtctgacagc tgaccggtct aaagaggtcc ctagcgccta 1260
   cggggaattt gtatcgataa ggggtacaaa ttcccactaa gcgctcggcc ggggatcgat 1320
   ccccgggtac gtacccggca gtttttcttt ttcggcaagt gttcaagaag ttattaagtc 1380
   gggagtgcag tcgaagtggg caagttgaaa aattcacaaa aatgtggtat aatatctttg 1440
   ttcattagag cgataaactt gaatttgaga gggaacttag atggtatttg aaaaaattga 1500
   taaaaatagt tggaacagaa aagagtattt tgaccactac tttgcaagtg taccttgtac 1560
   ctacagcatg accgttaaag tggatatcac acaaataaag gaaaagggaa tgaaactata 1620
   tcctgcaatg ctttattata ttgcaatgat tgtaaaccgc cattcagagt ttaggacggc 1680
   aatcaatcaa gatggtgaat tggggatata tgatgagatg ataccaagct atacaatatt 1740
   tcacaatgat actgaaacat tttccagcct ttggactgag tgtaagtctg actttaaatc 1800
   atttttagca gattatgaaa gtgatacgca acggtatgga aacaatcata gaatggaagg 1860
   aaagccaaat gctccggaaa acatttttaa tgtatctatg ataccgtggt caaccttcga 1920
   tggctttaat ctgaatttgc agaaaggata tgattatttg attcctattt ttactatggg 1980
   gaaatattat aaagaagata acaaaattat acttcctttg gcaattcaag ttcatcacgc 2040
   agtatgtgac ggatttcaca tttgccgttt tgtaaacgaa ttgcaggaat tgataaatag 2100
   ttaacttcag gtttgtctgt aactaaaaac tagtatttaa cctaggatca aaaaaatttc 2160
   caataatccc actctaagcc acaaacacgc cctataaaat cccgctttaa tcccactttg 2220
   agacacatgt aatattactt tacgccctag tatagtgata attttttaca ttcaatgcca 2280
   cgcaaaaaaa taaaggggca ctataataaa agttccttcg gaactaacta aagtaaaaaa 2340
   ttatctttac aacctcccca aaaaaaagaa caggtacaaa gtaccctata atacaagcgt 2400
   aaaaaaaatg agggtaaaaa taaaaaaata aaaaaataaa aaaataaaaa aataaaaaaa 2460
   taaaaaaata aaaaaatata aaaataaaaa aatataaaaa taaaaaaata taaaaataaa 2520
   aaaataaaaa aatataaaaa taaaaaaata aaaaaatata aaaatatttt ttatttaaag 2580
   tttgaaaaaa atttttttat attatataat ctttgaagaa aagaatataa aaaatgagcc 2640
   tttataaaag cccatttttt ttcatatacg taatatgacg ttctaatgtt tttattggta 2700
   cttctaacat tagagtaatt tctttatttt taaagccttt ttctttaagg gcttttattt 2760
   tttttcttaa tacatttaat tcctcttttt ttgttgcttt tcctttagct tttaattgct 2820
   cttgataatt ttttttacct ctaatatttt ctcttctctt atattccttt ttagaaatta 2880
   ttattgtcat atatttttgt tcttcttctg taatttctaa taactctata agagtttcat 2940
   tcttatactt atattgctta tttttatcta aataacatct ttcagcactt ctagttgctc 3000
   ttataacttc tctttcactt aaatgttgtc taaacatact attaagttct aaaacatcat 3060
   ttaatgcctt ctcaatgtct tctgtaaagc tacaaagata atatctatat aaaaataata 3120
   taagctctct gtgtcctttt aaatcatatt ctcttagttc acaaagtttt attatgtctt 3180
   gtattcttcc ataatataaa cttctttctc tataaatata atttattttg cttggtctac 3240
   cctttttcct ttcatatggt tttaattcag gtaaaaatcc attttgtatt tctcttaagt 3300
   cataaatata ttcgtactca tctaatatat tgactactgt ttttgattta gagtttatac 3360
   ttcctggaac tcttaatatt ctcgttgcat ctaaggcttg tctatctgct ccaaagtatt 3420
   ttaattgatt atataaatat tcttgaaccg ctttccataa tggtaatgct ttactaggta 3480
   ctgcatttat tatccatatt aaatacattc ctcttccact atctattaca tagtttggta 3540
   taggaatact ttgattaaaa taattctttt ctaagtccat taatacctgg tctttagttt 3600
   tgccagtttt ataataatcc aagtctataa acagtgtatt taactctttt atattttcta 3660
   atcgcctaca cggcttataa aaggtattta gagttatata gatattttca tcactcatat 3720
   ctaaatcttt taattcagcg tatttatagt gccattggct atatcctttt ttatctataa 3780
   cgctcctggt tatccaccct ttacttctac tatgaatatt atctatatag ttctttttat 3840
   tcagctttaa tgcgtttctc acttattcac ctccccttct gtaaaactaa gaaaattata 3900
   tcatattttc aataattatt aactattctt aaactcttaa taaaaaatag agtaagtccc 3960
   caattgaaac ttaatctatt ttttatgttt taatttatta tttttattaa aatattttaa 4020
   actaaattaa atgattcttt ttaatttttt actatttcat tccataatat attactataa 4080
   ttatttacaa ataatatttc ttcatttgta atatttagat gatttactaa ttttagtttt 4140
   tatatattaa ataattaatg tataatttat ataaaaaatc aaaggagctt ataaattatg 4200
   attatttcca aagatactaa agatttaatt tttttcaatt ttaacaatac tttttgtaat 4260
   attatgttta aatttaattg tatttttttc atataataaa gccgttgaag taaaccaatc 4320
   cattttcctt atgatgttat tattaaattt aagttttata ataatatctt tattatattt 4380
   attgttttta aaaaaactag tgaaatttct agtgaaattt ccggctttat taaacttatt 4440
   tttaggaatt ttattttcat tttcatcttt acaggatttg attatatctt taaatatgtt 4500
   ttatcaaata ttatcttttt ctaaatttat atatattttt attatattta ttattatata 4560
   tattttattt ttaagtttct ttctaacagc tattaaaaag aaacttaaaa ataaaaacac 4620
   gtactctaaa ccaataaata aaactatttt tattattgct gccttgattg gaatagtttt     4680
   tagtaaaatt aatttcaata ttccacaata ttatattata agctagcacg cctcgagtat     4740
   attgataaaa ataataatag tgggtataat taagttgtta ggaggttagt tagaactaaa     4800
   tgtaaaatgt tagcgtttta gagctagaaa tagcaagtta aaataaggct agtccgttat     4860
   caacttgaaa aagtggcacc gagtcggtgc tttttttgaa gcttgtcgac atgaagatag     4920
   caataggtag tgatcatgca ggattttcat tgaaaaagga agttataaaa catttagaga     4980
   gtaaaaatat tgaggttaaa gattttggca ctctaactga tgaatcatgt gattatccag     5040
   attatgcatt aaaagtagca gaggaagttg ctcaaaaaaa ctttgagttt ggaatactca     5100
   tttgtggaac aggtatagga ataagcatat cagcaaataa ggtgccagga ataagagcag     5160
   ctgtatgtac agatacattc tgtgctcatg catcaagaga acataacaat gcaaatatac     5220
   ttgcaatggg agaaagagtt gtaggacctg gattagcaat tgatatagta gatacatttt     5280
   taaattcaaa atttcaggga gataggcatc aaagaagaat agacaagatt acacaaatag     5340
   aaaaaaaata caatggagga atgaaataat gagtaaagtt acacaaatat cacatccact     5400
   tatattacac aaattagcat ttatgagaga taaaaaaaca ggatctaaag attttagaga     5460
   gatggtagaa gaagtagcaa tgctaatggc atatgaagta acaagagaaa tgcagcttga     5520
   aactgttgaa atagaaactc ctatatgtat aactaaatgt aagatgtaag caggaaaaaa     5580
   ggtagctata gttcctatac ttagagcagg acttggaatg gtaaatggag tattaaaatt     5640
   aatacctgct gctaaggttg gacatatagg attatataga gatgaaaaga cattaaaacc     5700
   tgtagaatac ttctgtaaac ttcctcaaga tatagaggaa agagacataa tagtaactga     5760
   tccaatgctt gcaactggtg ggtcagcaat agatgcaata acacttctta agaaaagagg     5820
   agcaaaatac ataagactta tgtgtcttat aggagcacca gaaggtatag cagcagtaca     5880
   agaagcacat ccagatgtag atatatacct cgcatcaata gatgaaaagt tagatgaaaa     5940
   tggatatata gttcctggtc ttggagatgc tggagataga ttattcggta caaaataaat     6000
   tgcataaata aaaagggctg aaaaataaat ttcagtcctt ttatttatat tttaacttta     6060
   ttccatgcca ctgcctcttc tgataataat agaaattatt aaagttaata cagatgtaag     6120
   ggcaactgtt ccacctggag cacaatttaa gtaataagag ctaaataatc caacgagtac     6180
   atctataaag ctaaataata ttgataatat aagcgtg                              6217
<210> 12
   <211> 1002
   <212> DNA
   <213> Artificial Sequence
<220>
   <223> upp_del
<400> 12
   catgaagata gcaataggta gtgatcatgc aggattttca ttgaaaaagg aagttataaa      60
   acatttagag agtaaaaata ttgaggttaa agattttggc actctaactg atgaatcatg     120
   tgattatcca gattatgcat taaaagtagc agaggaagtt gctcaaaaaa actttgagtt     180
   tggaatactc atttgtggaa caggtatagg aataagcata tcagcaaata aggtgccagg     240
   aataagagca gctgtatgta cagatacatt ctgtgctcat gcatcaagag aacataacaa     300
   tgcaaatata cttgcaatgg gagaaagagt tgtaggacct ggattagcaa ttgatatagt     360
   agatacattt ttaaattcaa aatttcaggg agataggcat caaagaagaa tagacaagat     420
   tacacaaata gaaaaaaaat acaatggagg aatgaaataa tgagtaaagt tacacaaata     480
   tcacatccac ttatattaca caaacttcct caagatatag aggaaagaga cataatagta     540
   actgatccaa tgcttgcaac tggtgggtca gcaatagatg caataacact tcttaagaaa     600
   agaggagcaa aatacataag acttatgtgt cttataggag caccagaagg tatagcagca     660
   gtacaagaag cacatccaga tgtagatata tacctcgcat caatagatga aaagttagat     720
   gaaaatggat atatagttcc tggtcttgga gatgctggag atagattatt cggtacaaaa     780
   taaattgcat aaataaaaag ggctgaaaaa taaatttcag tccttttatt tatattttaa     840
   ctttattcca tgccactgcc tcttctgata ataatagaaa ttattaaagt taatacagat     900
   gtaagggcaa ctgttccacc tggagcacaa tttaagtaat aagagctaaa taatccaacg     960
   agtacatcta taaagctaaa taatattgat aatataagcg tg                       1002
<210> 13
   <211> 1306
   <212> DNA
   <213> Artificial Sequence
<220>
   <223> upp_stop
<400> 13
   atgaagatag caataggtag tgatcatgca ggattttcat tgaaaaagga agttataaaa      60
   catttagaga gtaaaaatat tgaggttaaa gattttggca ctctaactga tgaatcatgt     120
   gattatccag attatgcatt aaaagtagca gaggaagttg ctcaaaaaaa ctttgagttt     180
   ggaatactca tttgtggaac aggtatagga ataagcatat cagcaaataa ggtgccagga     240
   ataagagcag ctgtatgtac agatacattc tgtgctcatg catcaagaga acataacaat     300
   gcaaatatac ttgcaatggg agaaagagtt gtaggacctg gattagcaat tgatatagta     360
   gatacatttt taaattcaaa atttcaggga gataggcatc aaagaagaat agacaagatt     420
   acacaaatag aaaaaaaata caatggagga atgaaataat gagtaaagtt acacaaatat     480
   cacatccact tatattacac aaattagcat ttatgagaga taaaaaaaca ggatctaaag     540
   attttagaga gatggtagaa gaagtagcaa tgctaatggc atatgaagta acaagagaaa      600
   tgcagcttga aactgttgaa atagaaactc ctatatgtat aactaaatgt aagatgtaag     660
   caggaaaaaa ggtagctata gttcctatac ttagagcagg acttggaatg gtaaatggag     720
   tattaaaatt aatacctgct gctaaggttg gacatatagg attatataga gatgaaaaga     780
   cattaaaacc tgtagaatac ttctgtaaac ttcctcaaga tatagaggaa agagacataa     840
   tagtaactga tccaatgctt gcaactggtg ggtcagcaat agatgcaata acacttctta     900
   agaaaagagg agcaaaatac ataagactta tgtgtcttat aggagcacca gaaggtatag     960
   cagcagtaca agaagcacat ccagatgtag atatatacct cgcatcaata gatgaaaagt    1020
   tagatgaaaa tggatatata gttcctggtc ttggagatgc tggagataga ttattcggta    1080
   caaaataaat tgcataaata aaaagggctg aaaaataaat ttcagtcctt ttatttatat    1140
   tttaacttta ttccatgcca ctgcctcttc tgataataat agaaattatt aaagttaata    1200
   cagatgtaag ggcaactgtt ccacctggag cacaatttaa gtaataagag ctaaataatc    1260
   caacgagtac atctataaag ctaaataata ttgataatat aagcgt                   1306
<210> 14
   <211> 22
   <212> DNA
   <213> Artificial Sequence
<220>
   <223> amorce
<400> 14
   gtgggcaagt tgaaaaattc ac 22
<210> 15
   <211> 28
   <212> DNA
   <213> Artificial Sequence
<220>
   <223> amorce
<400> 15
   ttaactattt atcaattcct gcaattcg 28
<210> 16
   <211> 21
   <212> DNA
   <213> Artificial Sequence
<220>
   <223> amorce
<400> 16
   cttgagactt tgccgtgagg g 21
<210> 17
   <211> 20
   <212> DNA
   <213> Artificial Sequence
<220>
   <223> amorce
<400> 17
   tagttggaat gggcgctagt 20
<210> 18
   <211> 29
   <212> DNA
   <213> Artificial Sequence
<220>
   <223> amorce
<400> 18
   atgaagatag caataggtag tgatcatgc 29
<210> 19
   <211> 34
   <212> DNA
   <213> Artificial Sequence
<220>
   <223> amorce
<400> 19
   acgcttatat tatcaatatt atttagcttt atag 34
<210> 20
   <211> 20
   <212> DNA
   <213> Artificial Sequence
<220>
   <223> amorce
<400> 20
   tgtccaacct tagcagcagg 20
<210> 21
   <211> 26
   <212> DNA
   <213> Artificial Sequence
<220>
   <223> amorce
<400> 21
   gtagaagaag tagcaatgct aatggc 26
<210> 22
   <211> 62
   <212> DNA
   <213> Clostridium acetobutylicum
<400> 22
   aaactcctat atgtataact aaatgtaaaa tgttagcagg aaaaaaggta gctatagttc 60
   ct 62
<210> 23
   <211> 62
   <212> DNA
   <213> Artificial Sequence
<220>
   <223> fragment upp_stop fig7
<400> 23
   aaactcctat atgtataact aaatgtaaga tgtaagcagg aaaaaaggta gctatagttc 60
   ct 62
<210> 24
   <211> 86
   <212> DNA
   <213> Clostridium acetobutylicum
<400> 24
   aaactgttga aatagaaact cctatatgta taactaaatg taaaatgtta gcaggaaaaa 60
   aggtagctat agttcctata cttaga 86
<210> 25
   <211> 86
   <212> DNA
   <213> Artificial Sequence
<220>
   <223> fragment upp_stop fig16
<400> 25
   aaactgttga aatagaaact cctatatgta taactaaatg taagatgtaa gcaggaaaaa 60
   aggtagctat agttcctata cttaga 86
<210> 26
   <211> 10537
   <212> DNA
   <213> Artificial Sequence
<220>
   <223> plasmide
<400> 26
   tcgactctag aggatcctac ttggttttat agagatttta aggaggaagc tatgaagata 60
   ctttttgttt gtactggtaa tacatgtaga agctgtatgg cagaagctat gtttaatagt 120
   atgtgtaata tagatggtat agaagctttt tcagcaggtg catctgctat tcatggaagc 180
   aaaacttcct taaattcggc tgtggtagtt agggagaact taggcaaaga tatatcaaat 240
   agaaaggcta tacagttgac tactttttta gttgatgaat gtgatttagt tttaacaatg 300
   acatctttat taagtgacgt gcttagaagt caaatggaga agaactctaa taagatatat 360
   tcattaagtg aatatacagg tgtagagggt gacattactg acccatatgg tagaagtgta 420
   gagatctaca gacaaactta taaagacatt gaaaagaggc taaaaatact tattaaaaaa 480
   ctaaatgaag atagaagtat ttaatcatat actcttatct tctttttttg cgattatgta 540
   ttaagtggag gtgtttttat gaagatagca ataggtagtg atcatgcagg attttcattg 600
   aaaaaggaag ttataaaaca tttagagagt aaaaatattg aggttaaaga ttttggcact 660
   ctaactgatg aatcatgtga ttatccagat tatgcattaa aagtagcaga ggaagttgct 720
   caaaaaaact ttgagtttgg aatactcatt tgtggaacag gtataggaat aagcatatca 780
   gcaaataagg tgccaggaat aagagcagct gtatgtacag atacattctg tgctcatgca 840
   tcaagagaac ataacaatgc aaatatactt gcaatgggag aaagagttgt aggacctgga 900
   ttagcaattg atatagtaga tacattttta aattcaaaat ttcagggaga taggcatcaa 960
   agaagaatag acaagattac acaaatagaa aaaaaataca atggaggaat gaaataaaaa 1020
   aataagagtt accatttaag gtaactctta tttttattac ttaagataat catatataac 1080
   ttcagctcta ggcaatatta tatctgcaag aatgtgagag ctagaaacaa tctcttttac 1140
   tggcaaatca ttaagtggcg ccatagcgtg atcaaataac tgcagtcgag ttggtcctgt 1200
   ccaagcttca tgtacggtaa catctgtgat tttcgcattt ataagctcac atattctagg 1260
   gcttccatca taattgggta ttattttcaa catataatta gggcgacaaa tttgatcctt 1320
   tgcttcatta gcatctaagg ctttatgttt gtaccccatt gtagctgtcg caactctaag 1380
   ttttccatag tctaaagttc ctactaaagt atctgaatcc acaaaaagct ttggataccc 1440
   gagcttttta ggatatgcac ttaattccct tcctactgca attgcaggct cattatctaa 1500
   atacatcata tgaagataat ctcccttaac tccattaaag cttacgggaa tagcctgtcc 1560
   gctttctgta taacaaccaa gtccactcgt atcatgcatt gccataattt caaacctgac 1620
   taagggctca tcaatttcta aaggctctgg cacaacttta cgaagtgcat ccatatctgt 1680
   acgatataca atgttaaaat actcacgatt atgaaattta tagggtcctc taggaaatgc 1740
   aggcgaagtt aatggcgtgc taatttgttt aattacttca tcctttaaca taaaagtcac 1800
   cttcctaaat ttaataatgt ttagcttttc taacatactt tatcttcaca ttataaatcg 1860
   cctctagtcc tatttattta tatctagtta ttttttgtcg actgtttcat agtatttctt 1920
   tctaaacagc catgggtcta agttcattgg atatgagtaa atctgcagca gttaaagacc 1980
   ttatttcatc aatggttgtg tttttattaa tttcagtgag aagtaaacca tcattaataa 2040
   cctcaattac tccaagttct gttacaatta gatttgcttg agactttgcc gtgaggggaa 2100
   gtgtacattt ttttaaaatt ttaggttgac ctttatttgt atgtctcatt gcaattatta 2160
   ctttcttagc tccatttact aaatccatag ctccacccat accagagagc atttttccag 2220
   gaacaatcca attggctata ttaccctttt catctacctg gagagcccct aaaacagtaa 2280
   catctacgtg accaccacgg attagtgaaa acgaaactga gctatcgaaa aatgtgccgt 2340
   caggaagtac tgttgtatag tctcctcctg catttactac atctttatct gcctcattta 2400
   ttttaggact agcgcccatt ccaactattc cgttttctga ttggaaagta attttgaaat 2460
   tttttggtat ataatctgca accatggtag gaagacctac acctaagttt acaagttgac 2520
   cattttttaa ttctcttgca actcttttgg ctattatttc tttcgctagg tttttatcat 2580
   taatcatttt atgcaggctc ctttactata taatttataa gaactccggg ggtcattgct 2640
   ttttcctttt ctagtttttc acagctaact aaattttcag cttcaactat tacggtttta 2700
   gctgccattg ccatataggg attaaagttt ttagtagtac ctttatagaa ggtgtttccg 2760
   gcctcatcta caatactacc tttaattaat gctacatcgg ctgtaagagg tagctctaac 2820
   aaatattccg ttccatttat agatattttt ttctttcctt tttcaatcaa agttcctaaa 2880
   cctgttttag ttagtacacc acctaagcca gatccgcctg cacgtattct ttccactaga 2940
   gttccttggg gagagagctc tacttcaagt tcattattaa aaagtttttt gccagtatct 3000
   gggttgctgc ctatatatga agcaataagc ttttttactt gattatttga tattaactta 3060
   ccaatacctg tattaggata acatgtatca ttacttataa tcgttaaatt ctttatattt 3120
   aaattaacta aaaaatcaat taatttggtt ggagtgccac agtttaaaaa acctccaatc 3180
   ataattgtca tcccatcttt aaagaatgac cttaaatttt caaatctaat tattttagag 3240
   ttcattttaa tccctccttt taaattctct agaaaaatta tctcgaggta taatcctcca 3300
   tgatctatta tgttataata taactactgc tttaattaag tcttttggct tgtctttcat 3360
   taataacagt gcttcttcta tgtgatcaaa tccatgatat acatgtgtaa ctaatttact 3420
   tagatcaaca cgattatata ctaccatatc tcttaacatt tctgctctca aacgtccccc 3480
   aggacaaaga cctcctttta tagtcttgtg agccattcca catccccatt ctacacgtgg 3540
   tattagtaaa gcatctccac ttccatgata atttatatta gaaattattc ctcctggttt 3600
   aaccatagat actgcttggg ataatgtttc agaaccaccg cctgccataa ttacgcggtc 3660
   aacgcctttt ccattcgtta atttcataac ttgatcaact atatgaccat ttttataatt 3720
   tagaatatct gttgctccat aaaattttgc agcctcaaca caaatcggcc tgctccccac 3780
   tccaattatt ctacctgctc cacgtaattt agcacctgct attcccatta agccaacagc 3840
   tccaatgcca attaccacaa cacttgaacc catttgaata tctgcaagtt ctgctccatg 3900
   aaatccagta gtcatcatat ctgttatcat aacagcattt tctaatggca tgtctttagg 3960
   tagaatcgca agattcatat ccgcatcatt tacatgaaaa tattcaccaa aaactccatc 4020
   cttgaaattt gaaaatttcc atcctgcgag cataccgttt gagtgctgtt gaaaaccagc 4080
   ttgaacttcc aaagatctcc aatctggagt tgtacaagga actataactc tgtcaccagg 4140
   tttaaaatcc ttcacttcac ttcctacttc aacaacttca cctacagctt catgccctaa 4200
   aatcatattc ttcctatctc caagagctcc ctcaaaaaca gtatgtatat ctgatgtaca 4260
   cggagatact gctaatgggc gtacaatagc atcatatgaa cccgcaactg gcctttcttt 4320
   ttcgatccat cctaacttat taatacctag cattgcaaaa cctttcataa aatatgttcc 4380
   tccttaaaaa tattccttta atagtctaag ggcccccata gtttatccct aatttatacg 4440
   ttttctctaa caacttaatt atacccacta ttattatttt tatcaatata ttttgttaaa 4500
   aaaaacacaa agtttaatat tttttaacaa aaaaattaaa cttatgcata tgctaacccc 4560
   cttaaaatca tgttttagca taaatacata agtttatatt ttaactatat taatgtaata 4620
   aaaaatactt gattgcataa ataaaaaggg ctgaaaaata aatttcagtc cttttattta 4680
   tattttaact ttattccatg ccactgcctc ttctgataat aatagaaatt attaaagtta 4740
   atacagatgt aagggcaact gttccacctg gagcacaatt taagtaataa gagctaaata 4800
   atccaacgag tacatctata aagctaaata atattgataa tataagcgtt gacttaaatc 4860
   cctttttaaa ctgcatagca gcagctacag gtacagctaa tagtgaagag acaactaata 4920
   tccctgttat acgtattgaa acggatataa cgaaaccgac aacgatagaa aatatgtaat 4980
   ttaaaaattt tgtgtttatg ccagataccc ttgcaccttg ctcatcaaat gttgtatata 5040
   aaaagttgtt ataaaatata gctataagaa atatagatac tacacttatt attagtataa 5100
   gtacgagatc acttttacca acagttaaaa tacttccaaa aagaaaagat tcaatgtttc 5160
   cgcttgcacg tccacttgtt gttaaggtta tagctattcc aacagataga gttaatataa 5220
   ttggaagtat taagtccgaa tactgcttaa aggagtttct aagtaattct attaagacag 5280
   cacaaatagt tacaaaaata aaggttgtta tcataggatt tacgccaata aagataccta 5340
   tagctacacc ggcaaaagaa gcatgtgata atgcatcacc taccattgag tgtcttttta 5400
   aaactaagaa aatacctata agtggacaca aaattgcaat cattaccgag gcaagtaaag 5460
   cgttttgcat aaaacttaga cgaaatagtt caatcattat ttaacaacct ccatattgct 5520
   ttttaaaaaa tcgttaatgt taagcaaaga gccactttct ccatggaaat tgaaaatatg 5580
   agttgaatta gaataagcag cctttaaatt atgttctaca catattattg tgaattcgta 5640
   atcatggtca tagctgtttc ctgtgtgaaa ttgttatccg ctcacaattc cacacaacat 5700
   acgagccgga agcataaagt gtaaagcctg gggtgcctaa tgagtgagct aactcacatt 5760
   aattgcgttg cgctcactgc ccgctttcca gtcgggaaac ctgtcgtgcc agctgcatta 5820
   atgaatcggc caacgcgcgg ggagaggcgg tttgcgtatt gggcgctctt ccgcttcctc 5880
   gctcactgac tcgctgcgct cggtcgttcg gctgcggcga gcggtatcag ctcactcaaa 5940
   ggcggtaata cggttatcca cagaatcagg ggataacgca ggaaagaaca tgtgagcaaa 6000
   aggccagcaa aaggccagga accgtaaaaa ggccgcgttg ctggcgtttt tccataggct 6060
   ccgcccccct gacgagcatc acaaaaatcg acgctcaagt cagaggtggc gaaacccgac 6120
   aggactataa agataccagg cgtttccccc tggaagctcc ctcgtgcgct ctcctgttcc 6180
   gaccctgccg cttaccggat acctgtccgc ctttctccct tcgggaagcg tggcgctttc 6240
   tcatagctca cgctgtaggt atctcagttc ggtgtaggtc gttcgctcca agctgggctg 6300
   tgtgcacgaa ccccccgttc agcccgaccg ctgcgcctta tccggtaact atcgtcttga 6360
   gtccaacccg gtaagacacg acttatcgcc actggcagca gccactggta acaggattag 6420
   cagagcgagg tatgtaggcg gtgctacaga gttcttgaag tggtggccta actacggcta 6480
   cactagaaga acagtatttg gtatctgcgc tctgctgaag ccagttacct tcggaaaaag 6540
   agttggtagc tcttgatccg gcaaacaaac caccgctggt agcggtggtt tttttgtttg 6600
   caagcagcag attacgcgca gaaaaaaagg atctcaagaa gatcctttga tcttttctac 6660
   ggggtctgac gctcagtgga acgaaaactc acgttaaggg attttggtca tgagattatc 6720
   aaaaaggatc ttcacctaga tccttttaaa ttaaaaatga agttttaaat caatctaaag 6780
   tatatatgag taaacttggt ctgacagtta ccaaagctag cttaatacta gtatatactt 6840
   aatgtgataa gtgtctgaca gctgaccggt ctaaagaggt ccctagcgcc tacggggaat 6900
   ttgtatcgat aaggggtaca aattcccact aagcgctcgg ccggggatcg atccccgggt 6960
   acgtacccgg cagtttttct ttttcggcaa gtgttcaaga agttattaag tcgggagtgc 7020
   agtcgaagtg ggcaagttga aaaattcaca aaaatgtggt ataatatctt tgttcattag 7080
   agcgataaac ttgaatttga gagggaactt agatggtatt tgaaaaaatt gataaaaata 7140
   gttggaacag aaaagagtat tttgaccact actttgcaag tgtaccttgt acctacagca 7200
   tgaccgttaa agtggatatc acacaaataa aggaaaaggg aatgaaacta tatcctgcaa 7260
   tgctttatta tattgcaatg attgtaaacc gccattcaga gtttaggacg gcaatcaatc 7320
   aagatggtga attggggata tatgatgaga tgataccaag ctatacaata tttcacaatg 7380
   atactgaaac attttccagc ctttggactg agtgtaagtc tgactttaaa tcatttttag 7440
   cagattatga aagtgatacg caacggtatg gaaacaatca tagaatggaa ggaaagccaa 7500
   atgctccgga aaacattttt aatgtatcta tgataccgtg gtcaaccttc gatggcttta 7560
   atctgaattt gcagaaagga tatgattatt tgattcctat ttttactatg gggaaatatt 7620
   ataaagaaga taacaaaatt atacttcctt tggcaattca agttcatcac gcagtatgtg 7680
   acggatttca catttgccgt tttgtaaacg aattgcagga attgataaat agttaacttc 7740
   aggtttgtct gtaactaaaa actagtattt aacctaggat caaaaaaatt tccaataatc 7800
   ccactctaag ccacaaacac gccctataaa atcccgcttt aatcccactt tgagacacat 7860
   gtaatattac tttacgccct agtatagtga taatttttta cattcaatgc cacgcaaaaa 7920
   aataaagggg cactataata aaagttcctt cggaactaac taaagtaaaa aattatcttt 7980
   acaacctccc caaaaaaaag aacaggtaca aagtacccta taatacaagc gtaaaaaaaa 8040
   tgagggtaaa aataaaaaaa taaaaaaata aaaaaataaa aaaataaaaa aataaaaaaa 8100
   taaaaaaata taaaaataaa aaaatataaa aataaaaaaa tataaaaata aaaaaataaa 8160
   aaaatataaa aataaaaaaa taaaaaaata taaaaatatt ttttatttaa agtttgaaaa 8220
   aaattttttt atattatata atctttgaag aaaagaatat aaaaaatgag cctttataaa 8280
   agcccatttt ttttcatata cgtaatatga cgttctaatg tttttattgg tacttctaac 8340
   attagagtaa tttctttatt tttaaagcct ttttctttaa gggcttttat tttttttctt 8400
   aatacattta attcctcttt ttttgttgct tttcctttag cttttaattg ctcttgataa 8460
   ttttttttac ctctaatatt ttctcttctc ttatattcct ttttagaaat tattattgtc 8520
   atatattttt gttcttcttc tgtaatttct aataactcta taagagtttc attcttatac 8580
   ttatattgct tatttttatc taaataacat ctttcagcac ttctagttgc tcttataact 8640
   tctctttcac ttaaatgttg tctaaacata ctattaagtt ctaaaacatc atttaatgcc 8700
   ttctcaatgt cttctgtaaa gctacaaaga taatatctat ataaaaataa tataagctct 8760
   ctgtgtcctt ttaaatcata ttctcttagt tcacaaagtt ttattatgtc ttgtattctt 8820
   ccataatata aacttctttc tctataaata taatttattt tgcttggtct accctttttc 8880
   ctttcatatg gttttaattc aggtaaaaat ccattttgta tttctcttaa gtcataaata 8940
   tattcgtact catctaatat attgactact gtttttgatt tagagtttat acttcctgga 9000
   actcttaata ttctcgttgc atctaaggct tgtctatctg ctccaaagta ttttaattga 9060
   ttatataaat attcttgaac cgctttccat aatggtaatg ctttactagg tactgcattt 9120
   attatccata ttaaatacat tcctcttcca ctatctatta catagtttgg tataggaata 9180
   ctttgattaa aataattctt ttctaagtcc attaatacct ggtctttagt tttgccagtt 9240
   ttataataat ccaagtctat aaacagtgta tttaactctt ttatattttc taatcgccta 9300
   cacggcttat aaaaggtatt tagagttata tagatatttt catcactcat atctaaatct 9360
   tttaattcag cgtatttata gtgccattgg ctatatcctt ttttatctat aacgctcctg 9420
   gttatccacc ctttacttct actatgaata ttatctatat agttcttttt attcagcttt 9480
   aatgcgtttc tcacttattc acctcccctt ctgtaaaact aagaaaatta tatcatattt 9540
   tcaataatta ttaactattc ttaaactctt aataaaaaat agagtaagtc cccaattgaa 9600
   acttaatcta ttttttatgt tttaatttat tatttttatt aaaatatttt aaactaaatt 9660
   aaatgattct ttttaatttt ttactatttc attccataat atattactat aattatttac      9720
   aaataatatt tcttcatttg taatatttag atgatttact aattttagtt tttatatatt      9780
   aaataattaa tgtataattt atataaaaaa tcaaaggagc ttataaatta tgattatttc      9840
   caaagatact aaagatttaa tttttttcaa ttttaacaat actttttgta atattatgtt      9900
   taaatttaat tgtatttttt tcatataata aagccgttga agtaaaccaa tccattttcc      9960
   ttatgatgtt attattaaat ttaagtttta taataatatc tttattatat ttattgtttt     10020
   taaaaaaact agtgaaattt ctagtgaaat ttccggcttt attaaactta tttttaggaa     10080
   ttttattttc attttcatct ttacaggatt tgattatatc tttaaatatg ttttatcaaa     10140
   tattatcttt ttctaaattt atatatattt ttattatatt tattattata tatattttat     10200
   ttttaagttt ctttctaaca gctattaaaa agaaacttaa aaataaaaac acgtactcta     10260
   aaccaataaa taaaactatt tttattattg ctgccttgat tggaatagtt tttagtaaaa     10320
   ttaatttcaa tattccacaa tattatatta taagctagca cgcctcgagt atattgataa     10380
   aaataataat agtgggtata attaagttgt taggaggtta gttagaacta aatgtaaaat     10440
   gttagcgttt tagagctaga aatagcaagt taaaataagg ctagtccgtt atcaacttga     10500
   aaaagtggca ccgagtcggt gctttttttg aagcttg                              10537
<210> 27
   <211> 27
   <212> DNA
   <213> Artificial Sequence
<220>
   <223> amorce
<400> 27
   ctttttaaaa aagttaaata aggaagg 27
<210> 28
   <211> 28
   <212> DNA
   <213> Artificial Sequence
<220>
   <223> amorce
<400> 28
   gtttaactta agttacagaa aagctagg 28
<210> 29
   <211> 12849
   <212> DNA
   <213> Artificial Sequence
<220>
   <223> plasmide
<400> 29
   ctagtagtgg atttaatccc aaatgagcca acagaaccag aaccagaaac agaatcagaa 60
   caagtaacat tggatttaga aatggaagaa gaaaaaagca atgacttcgt gtgaataatg 120
   cacgaaatcg ttgcttattt ttttttaaaa gcggtatact agatataacg aaacaacgaa 180
   ctgaatagaa acgaaaaaag agccatgaca catttataaa atgtttgacg acattttata 240
   aatgcatagc ccgataagat tgccaaacca acgcttatca gttagtcaga tgaactcttc 300
   cctcgtaaga agttatttaa ttaactttgt ttgaagacgg tatataaccg tactatcatt 360
   atatagggaa atcagagagt tttcaagtat ctaagctact gaatttaaga attgttaagc 420
   aatcaatcgg aaatcgtttg attgcttttt ttgtattcat ttatagaagg tggagtttgt 480
   atgaatcatg atgaatgtaa aacttatata aaaaatagtt tattggagat aagaaaatta 540
   gcaaatatct atacactaga aacgtttaag aaagagttag aaaagagaaa tatctactta 600
   gaaacaaaat cagataagta tttttcttcg gagggggaag attatatata taagttaata 660
   gaaaataaca aaataattta ttcgattagt ggaaaaaaat tgacttataa aggaaaaaaa 720
   tctttttcaa aacatgcaat attgaaacag ttgaatgaaa aagcaaacca agttaattaa 780
   acaacctatt ttataggatt tataggaaag gagaacagct gaatgaatat cccttttgtt 840
   gtagaaactg tgcttcatga cggcttgtta aagtacaaat ttaaaaatag taaaattcgc 900
   tcaatcacta ccaagccagg taaaagcaaa ggggctattt ttgcgtatcg ctcaaaatca 960
   agcatgattg gcggtcgtgg tgttgttctg acttccgagg aagcgattca agaaaatcaa 1020
   gatacattta cacattggac acccaacgtt tatcgttatg gaacgtatgc agacgaaaac 1080
   cgttcataca cgaaaggaca ttctgaaaac aatttaagac aaatcaatac cttctttatt 1140
   gattttgata ttcacacggc aaaagaaact atttcagcaa gcgatatttt aacaaccgct 1200
   attgatttag gttttatgcc tactatgatt atcaaatctg ataaaggtta tcaagcatat 1260
   tttgttttag aaacgccagt ctatgtgact tcaaaatcag aatttaaatc tgtcaaagca 1320
   gccaaaataa tttcgcaaaa tatccgagaa tattttggaa agtctttgcc agttgatcta 1380
   acgtgtaatc attttggtat tgctcgcata ccaagaacgg acaatgtaga attttttgat 1440
   cctaattacc gttattcttt caaagaatgg caagattggt ctttcaaaca aacagataat 1500
   aagggcttta ctcgttcaag tctaacggtt ttaagcggta cagaaggcaa aaaacaagta 1560
   gatgaaccct ggtttaatct cttattgcac gaaacgaaat tttcaggaga aaagggttta 1620
   atagggcgta ataacgtcat gtttaccctc tctttagcct actttagttc aggctattca 1680
   atcgaaacgt gcgaatataa tatgtttgag tttaataatc gattagatca acccttagaa 1740
   gaaaaagaag taatcaaaat tgttagaagt gcctattcag aaaactatca aggggctaat 1800
   agggaataca ttaccattct ttgcaaagct tgggtatcaa gtgatttaac cagtaaagat 1860
   ttatttgtcc gtcaagggtg gtttaaattc aagaaaaaaa gaagcgaacg tcaacgtgtt 1920
   catttgtcag aatggaaaga agatttaatg gcttatatta gcgaaaaaag cgatgtatac 1980
   aagccttatt tagtgacgac caaaaaagag attagagaag tgctaggcat tcctgaacgg 2040
   acattagata aattgctgaa ggtactgaag gcgaatcagg aaattttctt taagattaaa 2100
   ccaggaagaa atggtggcat tcaacttgct agtgttaaat cattgttgct atcgatcatt 2160
   aaagtaaaaa aagaagaaaa agaaagctat ataaaggcgc tgacaaattc ttttgactta 2220
   gagcatacat tcattcaaga gactttaaac aagctagcag aacgccctaa aacggacaca 2280
   caactcgatt tgtttagcta tgatacaggc tgaaaataaa acccgcacta tgccattaca 2340
   tttatatcta tgatacgtgt ttgttttttc tttgctgttt agcgaatgat tagcagaaat 2400
   atacagagta agattttaat taattattag ggggagaagg agagagtagc ccgaaaactt 2460
   ttagttggct tggactgaac gaagtgaggg aaaggctact aaaacgtcga ggggcagtga 2520
   gagcgaagcg aacacttgat tttttaattt tctatctttt ataggtcatt agagtatact 2580
   tatttgtcct ataaactatt tagcagcata atagatttat tgaataggtc atttaagttg 2640
   agcatattag aggaggaaaa tcttggagaa atatttgaag aacccgatta catggattgg 2700
   attagttctt gtggttacgt ggtttttaac taaaagtagt gaatttttga tttttggtgt 2760
   gtgtgtcttg ttgttagtat ttgctagtca aagtgattaa atagaattct agcgccattc 2820
   gccattcagg ctgcgcaact gttgggaagg gcgatcggtg cgggcctctt cgctattacg 2880
   ccagctggcg aaagggggat gaaataaaag gttattttgc attgacaaag ataattaaat 2940
   attttattat tagttcataa gttagtttaa tatactaaca aaaataaagc aagtaaaata 3000
   tacctaaaat ataaaaaaat taggatagga aaacgatagt tatgaagtgg cattcaagga 3060
   gggatggata aaaagtacag tattggtcta gacataggaa ctaactctgt tgggtgggct 3120
   gttataacag atgaatataa agttccatca aaaaaattta aagtattagg aaacactgat 3180
   agacattcaa taaaaaaaaa cttgataggt gctttattat tcgattcagg agagactgct 3240
   gaagctacac gtttaaaaag aacagctaga cgtagatata caagaagaaa aaataggata 3300
   tgttatcttc aagaaatttt tagtaatgaa atggcaaaag ttgatgattc attctttcac 3360
   agactagaag aaagtttctt agttgaagaa gataagaagc atgaaagaca ccctattttt 3420
   ggtaatatcg tagatgaagt agcatatcat gagaagtatc caactatcta tcatttaaga 3480
   aagaaattag ttgattctac agataaagct gatctgagat taatatattt agctttagct 3540
   catatgatta aatttagagg acatttttta atagaaggtg atttaaaccc agacaacagc 3600
   gatgtagata aattatttat ccaattagtt caaacttata atcaattatt cgaagagaat 3660
   ccaattaatg caagtggtgt agacgctaag gctatattat cagctagatt atcaaaatct 3720
   agaagattag aaaatctaat agctcaactt cctggagaaa agaaaaatgg actttttggg 3780
   aacctaatag ctctctcact cggactaaca ccaaatttta aaagcaattt tgatcttgct 3840
   gaagacgcaa agttacaact atcaaaggat acatacgatg atgatttaga taatttgtta 3900
   gctcaaatag gtgatcaata tgctgatttg tttcttgcag caaaaaactt aagtgatgca 3960
   attttactat cagatatact tagagtaaat acagaaataa caaaggctcc tttatcagca 4020
   agtatgatta aacgatatga tgagcatcat caagatttaa cattattaaa ggcacttgta 4080
   agacaacaat taccagaaaa atataaagaa attttctttg atcaatctaa aaatggatat 4140
   gctggatata tagacggtgg agcaagtcaa gaagagtttt ataaatttat aaagcctatt 4200
   ttagaaaaaa tggatggaac tgaagaatta cttgttaaac ttaacagaga agatttactt 4260
   agaaaacaaa gaacttttga taatggttca attcctcacc aaattcattt aggagaatta 4320
   catgctatac taagaagaca agaagatttt tatccatttc ttaaagataa tagagaaaaa 4380
   attgaaaaaa ttttaacttt tagaatacca tattatgtag gaccacttgc aaggggaaat 4440
   tcaagatttg catggatgac tagaaaatca gaagaaacta taaccccgtg gaattttgaa 4500
   gaagtagtag ataaaggagc tagtgctcaa tcatttatag aaagaatgac aaattttgat 4560
   aagaatcttc ctaacgaaaa ggttttgcca aagcatagcc ttctttatga gtattttaca 4620
   gtttataatg agcttactaa agtaaaatac gttacagaag gaatgagaaa accagcattt 4680
   ttgtctggtg aacaaaagaa agcaatagta gacctattat ttaaaacaaa taggaaggtt 4740
   accgtaaagc aacttaaaga agattacttc aaaaaaattg aatgctttga tagtgttgaa 4800
   atatcaggag ttgaagatag atttaatgct tcacttggta catatcacga tctcttaaaa 4860
   attataaaag ataaggattt tttagataat gaagaaaatg aagatattct tgaagatata 4920
   gtattaacat tgacactttt tgaagataga gaaatgatag aagaaagatt aaaaacatat 4980
   gcacatcttt ttgatgataa ggttatgaag caacttaaaa gaagaagata tacaggttgg 5040
   ggacgtttgt caagaaagct aattaatggt attagagata aacaatcagg aaagactatt 5100
   ctcgattttc ttaaatcaga tggatttgct aatagaaact ttatgcaatt aattcatgat 5160
   gattctctta ctttcaaaga ggatattcaa aaggctcaag tttctggaca aggcgatagc 5220
   ttacacgaac acattgctaa ccttgcaggg agccccgcta tcaaaaaagg aattttacaa 5280
   acagttaaag ttgtagatga acttgttaaa gttatgggaa gacacaaacc tgagaatata 5340
   gttatagaaa tggccagaga aaatcaaaca acacaaaaag gacaaaaaaa ttctagagag 5400
   agaatgaaga gaattgaaga aggaataaaa gagctaggat cacaaatatt aaaagaacat 5460
   ccagttgaaa atactcaatt gcaaaatgaa aagttatatt tgtattactt acaaaatgga 5520
   agagatatgt atgttgatca agaactcgat attaatagat taagtgacta tgatgttgat 5580
   catattgttc ctcaatcatt tttaaaagat gattcaatcg ataacaaagt attaactaga 5640
   tcagataaaa atagaggaaa gtcagataat gtaccatctg aagaagttgt taaaaaaatg 5700
   aagaactatt ggagacaact tttaaatgca aagctaatta cacaaagaaa atttgacaat 5760
   ttaacaaaag cagaaagagg aggattaagc gaattagaca aagctggatt tataaaaaga 5820
   caacttgttg agacaagaca aataactaag catgttgctc aaatacttga ttcaagaatg 5880
   aatacaaaat atgatgaaaa tgataaatta atcagagaag taaaagtaat aacattaaag 5940
   tcaaaattag tatcagattt cagaaaggat tttcaatttt acaaagttcg tgaaataaat 6000
   aactatcatc atgctcatga tgcatactta aatgctgttg taggaactgc tcttattaag 6060
   aaatatccta aactagaaag cgaatttgtt tatggagatt ataaagttta tgatgtgcgc 6120
   aaaatgatcg cgaaatccga acaagaaatc ggtaaggcta cagcaaaata tttcttttat 6180
   agtaatataa tgaatttttt taagacagaa ataactttgg ctaatggtga aatcagaaaa 6240
   agaccactta tcgaaacaaa tggagagaca ggagaaatag tatgggataa aggaagagat 6300
   tttgctactg ttagaaaagt actaagtatg ccacaagtaa atatcgtaaa gaaaactgaa 6360
   gttcaaactg gaggtttctc taaggaatca attttaccta agagaaattc agataagtta 6420
   attgcaagga aaaaagattg ggacccaaaa aaatacggtg gttttgatag tccaacagtt 6480
   gcctatagtg ttcttgtagt agcgaaagtt gagaaaggta agtcaaaaaa gttgaaaagc 6540
   gtaaaagaac ttcttggtat cacaattatg gaaagatctt catttgaaaa aaatccaatt 6600
   gactttttag aagctaaggg ttataaagaa gttaaaaagg atttaatcat aaaactacca 6660
   aagtatagtc tatttgaact cgaaaacgga agaaaacgaa tgctcgctag cgcaggagaa 6720
   cttcaaaaag gaaatgaact tgcgctgcca tcaaagtatg taaatttctt atatttagct 6780
   tctcattatg agaaattaaa aggatcacca gaggataatg aacaaaagca actatttgta 6840
   gaacaacaca aacattattt agatgaaata atagaacaaa tatctgaatt ttctaaaaga 6900
   gttatacttg ccgacgcaaa tctagataag gtgctttcag cgtataataa acacagagat 6960
   aaaccaataa gagaacaagc agaaaacatt atccatcttt ttacattaac taatcttggt 7020
   gcaccagctg catttaagta ctttgataca acaatagata gaaaaagata cacatctact 7080
   aaagaagtat tagacgcaac tttaatacat caatctatta cagggcttta tgaaacaaga 7140
   attgatttaa gtcaactagg cggagattaa gaattttttt agaaagaaca tgtgagcaaa 7200
   aggccagcaa aaggccagga accgtaaaaa ggccgcgttg ctggcgtttt tccataggct 7260
   ccgcccccct gacgagcatc acaaaaatcg acgctcaagt cagaggtggc gaaacccgac 7320
   aggactataa agataccagg cgtttccccc tggaagctcc ctcgtgcgct ctcctgttcc 7380
   gaccctgccg cttaccggat acctgtccgc ctttctccct tcgggaagcg tggcgctttc 7440
   tcatagctca cgctgtaggt atctcagttc ggtgtaggtc gttcgctcca agctgggctg 7500
   tgtgcacgaa ccccccgttc agcccgaccg ctgcgcctta tccggtaact atcgtcttga 7560
   gtccaacccg gtaagacacg acttatcgcc actggcagca gccactggta acaggattag 7620
   cagagcgagg tatgtaggcg gtgctacaga gttcttgaag tggtggccta actacggcta 7680
   cactagaagg acagtatttg gtatctgcgc tctgctgaag ccagttacct tcggaaaaag 7740
   agttggtagc tcttgatccg gcaaacaaac caccgctggt agcggtggtt tttttgtttg 7800
   caagcagcag attacgcgca gaaaaaaagg atctcaagaa gatcctttga tcttttctac 7860
   ggggtctgac gctcagtgga acgaaaactc acgttaaggg attttggtca tgagattatc 7920
   aaaaaggatc ttcacctaga tccttttaaa ttaaaaatga agttttaaat caatctaaag 7980
   tatatatgag taaacttggt ctgacagtta ccaatgctta atcagtgagg cacctatctc 8040
   agcgatctgt ctatttcgtt catccatagt tgcctgactc cccgtcgtgt agataactac 8100
   gatacgggag ggcttaccat ctggccccag tgctgcaatg ataccgcgag acccacgctc 8160
   accggctcca gatttatcag caataaacca gccagccgga agggccgagc gcagaagtgg 8220
   tcctgcaact ttatccgcct ccatccagtc tattaattgt tgccgggaag ctagagtaag 8280
   tagttcgcca gttaatagtt tgcgcaacgt tgttgccatt gctacaggca tcgtggtgtc 8340
   acgctcgtcg tttggtatgg cttcattcag ctccggttcc caacgatcaa ggcgagttac 8400
   atgatccccc atgttgtgca aaaaagcggt tagctccttc ggtcctccga tcgttgtcag 8460
   aagtaagttg gccgcagtgt tatcactcat ggttatggca gcactgcata attctcttac 8520
   tgtcatgcca tccgtaagat gcttttctgt gactggtgag tactcaacca agtcattctg 8580
   agaatagtgt atgcggcgac cgagttgctc ttgcccggcg tcaatacggg ataataccgc 8640
   gccacatagc agaactttaa aagtgctcat cattggaaaa cgttcttcgg ggcgaaaact 8700
   ctcaaggatc ttaccgctgt tgagatccag ttcgatgtaa cccactcgtg cacccaactg 8760
   atcttcagca tcttttactt tcaccagcgt ttctgggtga gcaaaaacag gaaggcaaaa 8820
   tgccgcaaaa aagggaataa gggcgacacg gaaatgttga atactcatac tcttcctttt 8880
   tcaatattat tgaagcattt atcagggtta ttgtctcatg agcggataca tatttgaatg 8940
   tatttagaaa aataaacaaa taggggttcc gcgcacattt ccccgaaaag tgccacctga 9000
   ctgccgggcc tcttgcggga tcaaaagaaa aacgaaatga tacaccaatc agtgcaaaaa 9060
   aagatataat gggagataag acggttcgtg ttcgtgctga cttgcaccat atcataaaaa 9120
   tcgaaacagc aaagaatggc ggaaacgtaa aagaagttat ggaaataaga cttagaagca 9180
   aacttaagag tgtgttgata gtgcagtatc ttaaaatttt gtataatagg aattgaagtt 9240
   aaattagatg ctaaaaattt gtaattaaga aggagtgatt acatgaacaa aaatataaaa 9300
   tattctcaaa actttttaac gagtgaaaaa gtactcaacc aaataataaa acaattgaat 9360
   ttaaaagaaa ccgataccgt ttacgaaatt ggaacaggta aagggcattt aacgacgaaa 9420
   ctggctaaaa taagtaaaca ggtaacgtct attgaattag acagtcatct attcaactta 9480
   tcgtcagaaa aattaaaact gaatactcgt gtcactttaa ttcaccaaga tattctacag 9540
   tttcaattcc ctaacaaaca gaggtataaa attgttggga gtattcctta ccatttaagc 9600
   acacaaatta ttaaaaaagt ggtttttgaa agccatgcgt ctgacatcta tctgattgtt 9660
   gaagaaggat tctacaagcg taccttggat attcaccgaa cactagggtt gctcttgcac 9720
   actcaagtct cgattcagca attgcttaag ctgccagcgg aatgctttca tcctaaacca 9780
   aaagtaaaca gtgtcttaat aaaacttacc cgccatacca cagatgttcc agataaatat 9840
   tggaagctat atacgtactt tgtttcaaaa tgggtcaatc gagaatatcg tcaactgttt 9900
   actaaaaatc agtttcatca agcaatgaaa cacgccaaag taaacaattt aagtaccgtt 9960
   acttatgagc aagtattgtc tatttttaat agttatctat tatttaacgg gaggaaataa 10020
   ttctatgagt ccctaggggt tccgcgcaca tttccccgaa aagtgccacc tgaacgaagc 10080
   atctgtgctt cattttgtag aacaaaaatg caacgcgaga gcgctaattt ttcaaacaaa 10140
   gaatctgagc tgcattttta cagaacagaa atgcaacgcg aaagcgctat tttaccaacg 10200
   aagaatctgt gcttcatttt tgtaaaacaa aaatgcaacg cgagagcgct aatttttcaa 10260
   acaaagaatc tgagctgcat ttttacagaa cagaaatgca acgcgagagc gctattttac 10320
   caacaaagaa tctatacttc ttttttgttc tacaaaaatg catcccgaga gcgctatttt 10380
   tctaacaaag catcttagat tacttttttt ctcctttgtg cgctctataa tgcagtctct 10440
   tgataacttt ttgcactgta ggtccgttaa ggttagaaga aggctacttt ggtgtctatt 10500
   ttctcttcca taaaaaaagc ctgactccac ttcccgcgtt tactgattac tagcgaagct 10560
   gcgggtgcat tttttcaaga taaaggcatc cccgattata ttctataccg atgtggattg 10620
   cgcatacttt gtgaacagaa agtgatagcg ttgatgattc ttcattggtc agaaaattat 10680
   gaacggtttc ttctattttg tctctatata ctacgtatag gaaatgttta cattttcgta 10740
   ttgttttcga ttcactctat gaatagttct tactacaatt tttttgtcta aagagtaata 10800
   ctagagataa acataaaaaa tgtagaggtc gagtttagat gcaagttcaa ggagcgaaag 10860
   gtggatgggt aggttatata gggatatagc acagagatat atagcaaaga gatacttttg 10920
   agcaatgttt gtggaagcgg tattcgcaat attttagtag ctcgttacag tccggtgcgt 10980
   ttttggtttt ttgaaagtgc gtcttcagag cgcttttggt tttcaaaagc gctctgaagt 11040
   tcctatactt tctagagaat aggaacttcg gaataggaac ttcaaagcgt ttccgaaaac 11100
   gagcgcttcc gaaaatgcaa cgcgagctgc gcacatacag ctcactgttc acgtcgcacc 11160
   tatatctgcg tgttgcctgt atatatatat acatgagaag aacggcatag tgcgtgttta 11220
   tgcttaaatg cgtacttata tgcgtctatt tatgtaggat gaaaggtagt ctagtacctc 11280
   ctgtgatatt atcccattcc atgcggggta tcgtatgctt ccttcagcac taccctttag 11340
   ctgttctata tgctgccact cctcaattgg attagtctca tccttcaatg ctatcatttc 11400
   ctttgatatt ggatcatact aagaaaccat tattatcatg acattaacct ataaaaatag 11460
   gcgtatcacg aggccctttc gtctcgcgcg tttcggtgat gacggtgaaa acctctgaca 11520
   catgcagctc ccggagacgg tcacagcttg tctgtaagcg gatgccggga gcagacaagc 11580
   ccgtcagggc gcgtcagcgg gtgttggcgg gtgtcggggc tggcttaact atgcggcatc 11640
   agagcagatt gtactgagag tgcaccatac cacagctttt caattcaatt catcattttt 11700
   tttttattct tttttttgat ttcggtttct ttgaaatttt tttgattcgg taatctccga 11760
   acagaaggaa gaacgaagga aggagcacag acttagattg gtatatatac gcatatgtag 11820
   tgttgaagaa acatgaaatt gcccagtatt cttaacccaa ctgcacagaa caaaaacctg 11880
   caggaaacga agataaatca tgtcgaaagc tacatataag gaacgtgctg ctactcatcc 11940
   tagtcctgtt gctgccaagc tatttaatat catgcacgaa aagcaaacaa acttgtgtgc 12000
   ttcattggat gttcgtacca ccaaggaatt actggagtta gttgaagcat taggtcccaa 12060
   aatttgttta ctaaaaacac atgtggatat cttgactgat ttttccatgg agggcacagt 12120
   taagccgcta aaggcattat ccgccaagta caatttttta ctcttcgaag acagaaaatt 12180
   tgctgacatt ggtaatacag tcaaattgca gtactctgcg ggtgtataca gaatagcaga 12240
   atgggcagac attacgaatg cacacggtgt ggtgggccca ggtattgtta gcggtttgaa 12300
   gcaggcggca gaagaagtaa caaaggaacc tagaggcctt ttgatgttag cagaattgtc 12360
   atgcaagggc tccctatcta ctggagaata tactaagggt actgttgaca ttgcgaagag 12420
   cgacaaagat tttgttatcg gctttattgc tcaaagagac atgggtggaa gagatgaagg 12480
   ttacgattgg ttgattatga cacccggtgt gggtttagat gacaagggag acgcattggg 12540
   tcaacagtat agaaccgtgg atgatgtggt ctctacagga tctgacatta ttattgttgg 12600
   aagaggacta tttgcaaagg gaagggatgc taaggtagag ggtgaacgtt acagaaaagc 12660
   aggctgggaa gcatatttga gaagatgcgg ccagcaaaac taaaaaactg tattataagt 12720
   aaatgcatgt atactaaact cacaaattag agcttcaatt taattatatc agttattacc 12780
   ctatgcggtg tgaaataccg cacagatgcg taaggagaaa ataccgccct aggcccaact 12840
   aactcaacg 12849
<210> 30
   <211> 9367
   <212> DNA
   <213> Artificial Sequence
<220>
   <223> plasmide
<400> 30
   aaaaataatg gccctaggga tatacaattt ttaaaaaata tagctaatct tgcataaact      60
   atataataaa tctaatttta gaatgtataa tttaaagggt tatggctgta tatagtcata     120
   acccttatat ttcattaatt aaagttaaat tttgtatatt caaaaagctc ttgatattta     180
   cggcagagcc accatataga gatgcctcag accctaatga tgaaatttct atatttattc     240
   ctttgttaaa agagctaact aacatatctt ttgtaatctg taatatctca gggatatcac     300
   taattatttg gctatttaaa tatattattt caggagcata agttgtaata gcattattta     360
   tagctattgt taagtaacta caaaactcat gaataacttt tttggcattc tggttatctt     420
   cataatatag ttgctttact atatcagaat ctattttagg aatattttct aaagaagaaa     480
   gctgttcaaa tacctttttt tctgaacaat actgttctaa acagccacga tttccacaag      540
   gacatagttt accattaggc atgatgatag tgtgaccaat ttctccactc ataccatttc     600
   tgccactata taatttatta tttattataa tgcctgaacc aaatccactg tgaatactga     660
   gactaagtag tgagttatgt acagttgaaa aagtattctc agctaaagct gttaagtttg     720
   cttcattttc tatgtgaata ggaaagtcat actttttgct aaggatgcta tataagtcaa     780
   tctcattaag attgtaatat ggagtaaaca atactttatt ttcacaagta atcccatgta     840
   tagccaaagt aagaccaatt accttataag gagtgtctat tttggatatg ttataactat     900
   ttataatttc atctatcaat tgtataacat tgtctttact tacttgtata tctgttaatt     960
   tcttagaatt tataatagtt ccatctaagt aagatagaga agaaaatata tagtcgtatc    1020
   caatgtccat acttaaagaa atccctgcac atttattaaa tactaataat atgggttttc    1080
   ttccgccact atgagtacta tttccaattc ctatttcatg aactagagat tcatcaataa    1140
   gttttttggt aatagcggat atagttgctt tattcaatcc tatagtagaa gctatacttg     1200
   ccctagaaat aggaccattt tttataattt gttcaagtac caatctttca ttcatttctc    1260
   gaatagtgta tttatcagta accaatttga tattcctcct taaaataata ttgtaatact    1320
   ttttacacaa aaataaaagg ttattttgca ttgacaaaga taattaaata ttttattatt    1380
   agttcataag ttagtttaat atactaacaa aaataaagca agtaaaatat acctaaaata    1440
   taaaaaaatt aggataggaa aacgatagtt atgaagtggc attcaaggag tatggaaaaa    1500
   ctcaagttta tgcttgtttt agagctagaa atagcaagtt aaaataaggc tagtccgtta    1560
   tcaacttgaa aaagtggcac cgagtcggtg cttttaaagt attgttaaaa ataactctgt    1620
   agaattataa attagttcta cagagttatt ttttaaaaaa attcaagctt gcatgcctgc    1680
   aggccatttt agctaaggat atctataatg aaaagattta gaggaggaat aatttatgaa    1740
   aaagattttt gtacttggag caggaacaat gggtgctggt atcgttcaag cattcgctca    1800
   aaaaggttgt gaagtaattg taagagacat aaaggaagaa tttgttgaca gaggaatagc    1860
   tggaatcact aaaggattag aaaagcaagt tgctaaagga aaaatgtctg aagaagataa    1920
   agaagctata ctttcaagaa tttcaggaac aactgatatg aaattagctg ctgactgtga    1980
   tttagtagtt gaagctgcaa tcgaaaacat gaaaattaag aaggaaatct tcgctgaatt    2040
   agatggaatt tgtaagccag aagcgatttt agcttcaaac acttcatctt tatcaattac    2100
   tgaagttgct tcagctacaa agagacctga taaagttatc ggaatgcatt tctttaatcc    2160
   agctccagta atgaagcttg ttgaaattat taaaggaata gctacttctc aagaaacttt    2220
   tgatgctgtt aaggaattat cagttgctat tggaaaagaa ccagtagaag ttgcagaagc    2280
   tccaggattc gttgtaaaca gaatattaat cccaatgatt aacgaagctt catttatcct    2340
   acaagaagga atagcttcag ttgaagatat tgatacagct atgaaatatg gtgctaacca    2400
   tccaatggga cctttagctt taggagatct tattggatta gacgtttgct tagctatcat    2460
   ggatgtttta ttcactgaaa caggtgataa caagtacaga gctagcagca tattaagaaa    2520
   atatgttaga gctggatggc ttggaagaaa atcaggaaaa ggattctatg attactctaa    2580
   ataatatcat gaatatatgg aaaaactcaa gtttatgctt caaactgctt cccctaattc    2640
   cccattttta tatatatttg tattaaatta actttaatgt tacaatgttc ttagtatatt    2700
   ttccttaata ttacattagt tctataaact ttattgttct taatatttaa ataaaatcca    2760
   tgaagggagg aaaaaactat cttttaaaag tttatagtaa ataaaaaaaa attattaatg    2820
   taaaaatata ctaagtatag aatatttata atagggggta ttaacttgtt ttcaaaaatc    2880
   aaaaaaatta atttttttaa aaaaacattt tcttttttaa ttgctgttgt aatgatgttg    2940
   tttacagtat taggaacaaa tacttataaa gctgaagctg caagtggtgg tgcctgggct    3000
   caatgtggag gtgaaaactt ccatggtgat aaatgttgtg tttccggtca cacttgtgtt    3060
   agtattaacc aatggtattc acaatgtcaa ccaggaggtg ctccaagcaa taatgcttca    3120
   aacaataata ataacaataa caataataac aacaacaata ataacaacaa caataatcac    3180
   aacaacaaca acaataacaa caataacaat aacaatggtg gtagtggtag tactaaaaac    3240
   ttcttcgata accaaattta tgctaaccca aagtttattg aagaagtcaa ttcttctatt    3300
   ccaagattaa gttatgattt acaacaaaag gctcaaaagg ttaagaatgt tccaactgcc    3360
   gtttggttag cttgggatgg agccactgga gaagttgctc aacatcttaa agctgctggt    3420
   tctaaaactg ttgtcttcat catgtacatg attccaactc gtgattgtaa cgctaatgcc    3480
   tctgctggtg gtgctggtaa cctcaacact tacaagggat acgttgataa tattgctaga    3540
   actattcgta gttatccaaa ttcaaaggtt gttatgattc ttgaaccaga tactcttggt    3600
   aaccttgtta ctgctaatag tgctaactgt caaaacgttc gtaacttaca taagaatgct    3660
   ttatcttatg gtgttaatgt tttcggtagc atgagtaatg ttagtgttta ccttgatgct    3720
   gctcatggtg cttggttagg tagctctact gataaggttg cttctgttgt taaggaaatc    3780
   ttaaataatg ctccaaatgg aaagattcgt ggtttaagta ctaacatttc taactaccaa 3840
   tcaatttctt ctgaatacca ataccaccaa aaacttgcct ctgctcttgc tgctgtcggt 3900
   gttccaaaca tgcacttcat tgttgatact ggtcgtaatg gtgttactat taattctgga 3960
   acatggtgta acttagtcgg tactggtctt ggtgaacgtc caagaggtaa tccaaatgct 4020
   ggtatgccat tattagatgc ttacatgtgg cttaagactc caggagaatc tgacggttca 4080
   tcctctggtt ctagagctga tccaaattgt tctagtaatg attctcttag aggtgctcca 4140
   gatgctggtc aatggttcca tgattacttc gctcaattag taagaaatgc tagaccatca 4200
   ttttaagcaa atttctaaat gattgaattt aacaaaaatt actaatctag aggatccccg 4260
   ggtaccgagc tcgaattcgt aatcatggaa atatataaat aactactaga ttacataaat 4320
   aagtttaaat taaagaatat tgtaaaattc aacaaaacat tttattactg ttaagtaagt 4380
   gatataatat attaaataat aactattttc ttaagattaa ttgtataatt taattgaata 4440
   ttggatatta aaaaaataaa tagttaaaaa atatgatata ggtggtacac atcagaaaat 4500
   gggcaatata aactgtgaca taatctttag aaaaaaactt tagtgtagaa tatattattt 4560
   aaagaagaat atatgaatga aataagttaa aactaaggtt gatatgatat tatgtttatc 4620
   aatgatttta ctaaaaagat aaaaatatat tttgaatatt cataatcaaa gggtagggtt 4680
   catcaatatg catattagat tatgagatga tgcaaactaa ataattttat actgtaaggg 4740
   agacggataa taaaatggat tataaatctg tagaatttta tgataattat ttaataataa 4800
   aagaattaag aaattttaaa ttaaaacata tatttgagtg cggacagatt tttaggtttg 4860
   aagaggtagc agaaaatgat tttattgtaa ttgcgtttgg aagattaatt gaagttaaag 4920
   aagatggaaa tgacgtaata atttataatt ctacaaagga agattttaaa aatatttggc 4980
   ttaagtattt tgatttagat agagattact cagttataaa agatgaactt tcaaaagatg 5040
   ttttacttaa acaaagtatt gaatttggat atggtgttag agtcttgaat caagatccat 5100
   ttgaaatgtt gcttagcttt attatttcag cgcgaaataa tataccatca ataaagaaga 5160
   ctgtaaataa aatatctaat aaatggggaa aagaaattat ttataaggat aaaacctact 5220
   atgcgtttcc taatataggt gaaataaaag atgctacact agaagaaata caggagacag 5280
   gagcatcttc catggacgcg tgacgtcgac tctagaaatt cttctaaata taagaatatt 5340
   ttaaagaaat atctttatat attagttatt aaaatttata agattataag aaacattata 5400
   acatatttta gaacttttta actattctaa aagattaatt tacatattaa catttaatta 5460
   tgggtaaaaa ctattttgaa aaatgattta tatggaatta tgtttcttaa atatacaatc 5520
   atgtttcatg aatacataat tattttaaat gtattgggag ggtaaattga ttgtgaaacg 5580
   cggcgatgtt tattttgctg atttatctcc tgttgttggc tcagagcaag gcggggtgcg 5640
   cccggtttta gtgatccaaa atgacatcgg aaatcgcttc agcccaactg ctattgttgc 5700
   agccataaca gcacaaatac agaaagcgaa attaccaacc cacgtcgaaa tcgatgcaaa 5760
   acgctacggt tttgaaagag attccgttat tttgctggag caaattcgga cgattgacaa 5820
   gcaaaggtta acggataaga ttactcatct ggatgatgaa atgatggata aggttgatga 5880
   agccttacaa atcagtttgg cactcattga tttttagaca tatttgcagg ttgctcaaat 5940
   agagcgaaag aacatgtgag caaaaggcca gcaaaaggcc aggaaccgta aaaaggccgc 6000
   gttgctggcg tttttccata ggctccgccc ccctgacgag catcacaaaa atcgacgctc 6060
   aagtcagagg tggcgaaacc cgacaggact ataaagatac caggcgtttc cccctggaag 6120
   ctccctcgtg cgctctcctg ttccgaccct gccgcttacc ggatacctgt ccgcctttct 6180
   cccttcggga agcgtggcgc tttctcatag ctcacgctgt aggtatctca gttcggtgta 6240
   ggtcgttcgc tccaagctgg gctgtgtgca cgaacccccc gttcagcccg accgctgcgc 6300
   cttatccggt aactatcgtc ttgagtccaa cccggtaaga cacgacttat cgccactggc 6360
   agcagccact ggtaacagga ttagcagagc gaggtatgta ggcggtgcta cagagttctt 6420
   gaagtggtgg cctaactacg gctacactag aagaacagta tttggtatct gcgctctgct 6480
   gaagccagtt accttcggaa aaagagttgg tagctcttga tccggcaaac aaaccaccgc 6540
   tggtagcggt ggtttttttg tttgcaagca gcagattacg cgcagaaaaa aaggatctca 6600
   agaagatcct ttgatctttt ctacggggtc tgacgctcag tggaacgaaa actcacgtta 6660
   agggattttg gtcatgagat tatcaaaaag gagtttaaac ttctaaaatc tgattaccaa 6720
   ttagaatgaa tatttcccaa atattaaata ataaaacaaa aaaattgaaa aaagtgtttc 6780
   caccattttt tcaatttttt tataattttt ttaatctgtt atttaaatag tttatagtta 6840
   aatttacatt ttcattagtc cattcaatat tctctccaag ataactacga actgctaaca 6900
   aaattctctc cctatgttct aatggagaag attcagccac tgcatttccc gcaatatctt 6960
   ttggtatgat tttacccgtg tccatagtta aaatcatacg gcataaagtt aatatagagt 7020
   tggtttcatc atcctgataa ttatctatta attcctctga cgaatccata atggctcttc 7080
   tcacatcaga aaatggaata tcaggtagta attcctctaa gtcataattt ccgtatattc 7140
   ttttattttt tcgttttgct tggtaaagca ttatggttaa atctgaattt aattccttct 7200
   gaggaatgta tccttgttca taaagctctt gtaaccattc tccataaata aattcttgtt 7260
   tgggaggatg attccacggt accatttctt gctgaataat aattgttaat tcaatatatc 7320
   gtaagttgct tttatctcct attttctttg aaataggtct aattttttgt ataagtattt 7380
   ctttactttg atctgtcaat ggttcagata cgacgactaa aaagtcaaga tcactatttg 7440
   gttttagtcc actctcaact cctgatccaa acatgtaagt accaataagg ttatttttta 7500
   aatgtttccg aagtattttt ttcactttat taatttgttc gtatgtattc aaatatatcc 7560
   tcctcactat tttgattagt acctatttta tatccatagt tgttaattaa ataaacttaa 7620
   tttagtttat ttatagattt cattggcttc taaatttttt atctagataa taattatttt 7680
   agttaatttt attctagatt atatatgata tgatctttca tttccataaa actaaagtaa 7740
   gtgtaaacct attcattggg ccggccgctt ataatccata acaatcatcc tttctgtgac 7800
   actgtcagac acttatcaca ttaagtatat actattatta aactattcta tatacttaat 7860
   ttattttaat agaaaaacat aatatcataa taacttcaaa attaaacttt atttatgatt 7920
   tcatacttga ctttgatttt agaaaggata tactttttag cagatttgga aacggctttg 7980
   gacgtagttt gcccatagat gaacaaacaa actacatcca aaaattatac ttttcccttc 8040
   attggtatcc gtatttttac atcttaatag cgtatgtatt acaacacacc taaacaacga 8100
   ccttacggtc tgctactgca tatcctagct tgattgttta gttgcctcaa ctatgcttaa 8160
   ccctaccccg aactcttttt ttattgtggg ttttcgtcgt gaagtcccac cgacacataa 8220
   tcataacata agatgtatta tgaaaatgcg agtgactatc cttttgtatc ggctcactac 8280
   accacagata tattttttag tgcatactgt gtcggcactc tcaatattaa attaataaat 8340
   aaattatttt ttctttttac tcttctttac atgagctttt ttaaagctcc ttgcataata 8400
   tttaatgcat gtacgttctt ttttctgttc ttcctctgta aaacatctca tttttatggc 8460
   acaccatcca tatcggttca tctttgaaca attaatacat tggactttcc ctttatgtaa 8520
   acatcttgac tcattgtatt tactgcaata ggttgctact gttttatcat gaatcatcaa 8580
   cgaatgtatt ttgcatattt cagtatcttt aataaactct ttgcaatttt tacaagtttt 8640
   catatacgcc cttcttttca taaattaatt tatgaattct atgtattcca aggagctttt 8700
   taaagctccc cctttcgtac tacttagcta caagcactat aaaagtcata atgtttactg 8760
   ctaaagtcaa taatgatatt gctaatacct tgttatttga taagatactg ctttcctctg 8820
   tcactttgct cacccccttt cattttcata aattaattat gaaaaaataa atatacttct 8880
   aatttttatc aaataaaaaa gcctttgcgt actgcttcca atacacaaag gctataaact 8940
   tctaaatctt acttattgca atttacattt atatctgtta agataatctc ataaattgaa 9000
   tatatatagt taatgtttct cttgtatgtc ggtacatttg aaatattgct atagatagag 9060
   ttctctaacg gcttgatgtg ttggtagcac atttaagttt tggcttatat actaagttgg 9120
   tagcttaata tataagagct gaggacttat ttttttatta aatttttcaa cttgtctata 9180
   ttttaacccg taattgaata cataacaagt attttttttt gtattcaatt aaacattcat 9240
   aaatgagtat aattaatcat actaaattct ataattttct tttctgtaaa tttctttcta 9300
   ttcagcactg ttatgccttt tgactatcac ttaataaaaa ataagaaatg aattgtcaat 9360
   tgttcaa 9367
<210> 31
   <211> 25
   <212> DNA
   <213> Artificial Sequence
<220>
   <223> amorce
<400> 31
   agataattat gaagttaatc cttag 25
<210> 32
   <211> 27
   <212> DNA
   <213> Artificial Sequence
<220>
   <223> amorce
<400> 32
   catttgcttt caggtcttct tttgctg 27